(19) Europäisches Patentamt
European Patent Office
Office européen des brevets

(11) **EP 4 360 660 A1**

(12) **EUROPEAN PATENT APPLICATION**

(43) Date of publication:
**01.05.2024 Bulletin 2024/18**

(21) Application number: **22203296.3**

(22) Date of filing: **24.10.2022**

(51) International Patent Classification (IPC):
***A61K 49/08*** *(2006.01)* ***A61K 49/10*** *(2006.01)*
***A61K 49/12*** *(2006.01)*

(52) Cooperative Patent Classification (CPC):
**A61K 49/124**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Bayer AG**
**51373 Leverkusen (DE)**

(72) Inventor: **The designation of the inventor has not yet been filed**

(74) Representative: **BIP Patents**
**c/o Bayer Intellectual Property GmbH**
**Alfred-Nobel-Straße 50**
**40789 Monheim am Rhein (DE)**

(54) **PROCESS FOR THE PREPARATION OF A GADOLINIUM CONTRAST AGENT**

(57) The present invention relates to a process for the preparation of a gadolinium chelate compound of formula (I) or its stereoisomers, tautomers, N-oxides, hydrates, solvates, or salts thereof, or mixtures of same.

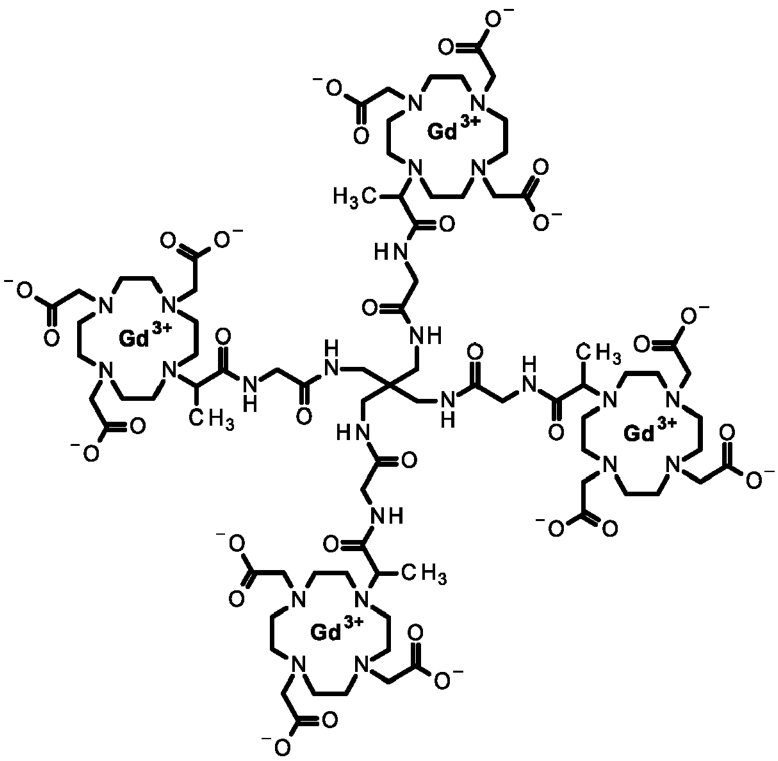

**(I)**

Further, the present invention relates to crystalline forms of the gadolinium chelate of formula (I), to a process for the preparation of said crystalline forms as well as to intermediate compounds in the synthesis of the gadolinium chelate compound of formula (I) and/or its crystalline forms as well as their stereoisomers, tautomers, N-oxides, hydrates, solvates, or salts thereof, or mixtures of same.



Processed by Luminess, 75001 PARIS (FR)

## Description

### FIELD OF THE INVENTION

The present invention relates to the items characterized in the patent claims, namely to a process for the preparation of a gadolinium chelate compound of formula (I) or its stereoisomers, tautomers, N-oxides, hydrates, solvates, or salts thereof, or mixtures of same.

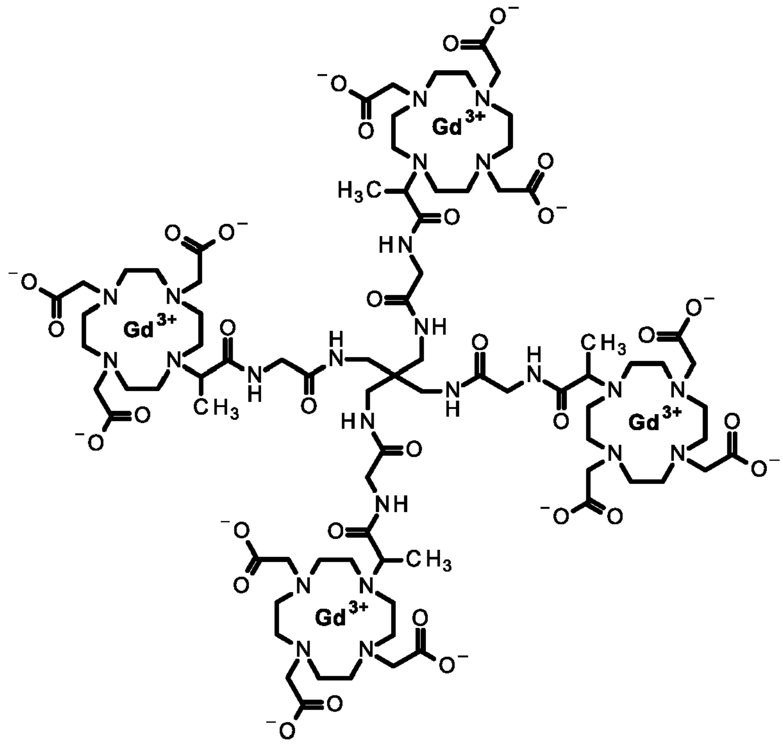

(I)

Further, the present invention relates to crystalline forms of the gadolinium chelate of formula (I), to a process for the preparation of said crystalline forms as well as to intermediate compounds in the synthesis of the gadolinium chelate compound of formula (I) and/or its crystalline forms as well as their stereoisomers, tautomers, N-oxides, hydrates, solvates, or salts thereof, or mixtures of same.

### BACKGROUND OF THE INVENTION

Gd-based contrast agents play a crucial role for diagnosis using magnet resonance imaging (MRI) techniques. One such Gd-based agent is the compound of formula (I), which is described in WO2016/193190 (BAYER AG). However, the synthetic process for the preparation of the compound of formula (I) described in the prior art, while leading to the desired product, encompasses several hurdles which are detrimental to its synthesis on a large scale.

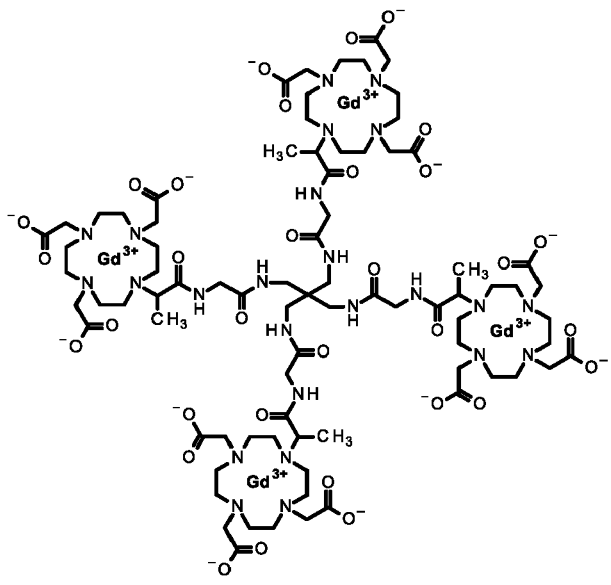

(I)

For example, the manufacture of the compound of formula (II), i.e. the 4-nitrophenol ester of carboxylic acid (IV) described in the prior art (WO 2001051095 A2) is hardly amendable for scale-up on metric ton scale. This is based on the fact that the route involves the formation of the pyridinium salt of carboxylic acid (IV) using highly toxic pyridine in water followed by isolation via lyophilization (WO 2001051095 A2, 21d, p. 44). Thereafter, reaction of the salt with an excess of bis (4-nitrophenol) carbonate in neat pyridine gives the corresponding activated ester of formula (II) after a long reaction time of 3 days which is isolated via filtration followed by washing of the highly toxic chemicals pyridine and dichloromethane.

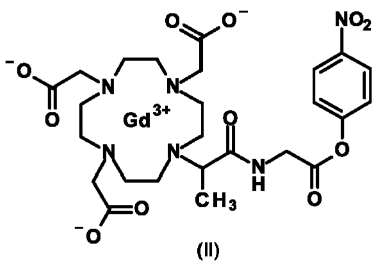

(II)

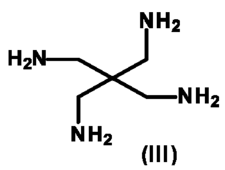

(III)

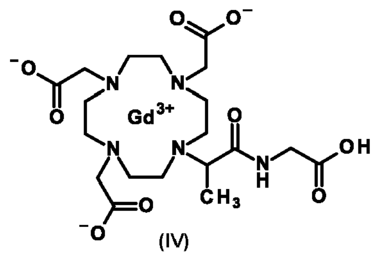

(IV)

Further, the fragment union of a tetraamine of formula (III) or a salt thereof and activated ester (II) has only been described in high dilution and using a large excess of the valuable intermediate of formula (II) to prepare the compound of formula (I). Upon distillation of large parts of the highly boiling solvent dimethyl sulfoxide, a process which requires low pressures and high temperatures, the crude material was precipitated by the addition of a large excess of ethyl acetate, thus complicating its manufacture on a large scale. Upon removal of low molecular impurities via diafiltration/ultrafiltration in an aqueous solution, the retentate was lyophilized and subjected to chromatography yielding an amorphous solid. Overall, the yield for the sequence was low. High dilution, an excess of reagents and cumbersome procedures, i.e. chromatography and lyophilization are the most likely factors that account for the high costs and little efficiency of the process.

Thus, there is an unmet need to provide a process for the preparation of a compound of general formula (I) which:

- is reliable and amendable on scale,
- is cost-efficient and provides a high overall yield,
- encompasses a reliable and scalable process for the manufacture of the compound of formula (II),
- enables the provision of the compound of general formula (I) as a crystalline material in high chemical purity, thus facilitating the compliance with the necessary regulatory requirements for clinical trials and market supply,

- tames and allows for the monitoring of the strong hygroscopic nature of the compound of general formula (I), thus facilitating transportation and handling for analytical purposes as well as for drug product manufacture, and at the same time allows to maintain the levels of residual ethanol in accordance with regulatory requirements.

Surprisingly, a process has now been found, and this constitutes the basis of the present invention, that allows for the preparation of a compound of general formula (I) and which overcomes the disadvantages described above for previously described processes. Further, the present invention also relates to novel crystalline forms of the gadolinium chelate of formula (I), to a process for the preparation of said crystalline forms as well as to intermediate compounds in the synthesis of the gadolinium chelate compound of formula (I) and/or its crystalline forms.

Further, it has surprisingly been found that in the context of the present invention it is possible to

- develop a one-pot protocol for the reaction of a compound of formula (IV) to a compound of formula (II) which avoids the use of the highly toxic and environmentally hazardous solvent pyridine including the isolation of the compound of formula (IV) as a crystalline material. This is a process that requires a fine balancing of the different variables involved in the reaction.
- provide a process to manufacture the compound of formula (II) in such a fashion that allows to reduce the equivalents in the subsequent reaction to the compound of formula (I) as well as to perform the reaction in a (much) higher concentration. Surprisingly, this protocol enables the isolation of the compound of formula (I) as the crude material as a well-behaved solid without the need for the distillation of the highly boiling solvent dimethyl sulfoxide.
- prepare the compound of formula (I) as a crude material in high purity, i.e. a purity of at least 50% (w/w) and preferably of at least 70% (w/w), together with very low amounts of residual organic solvents
- prepare the compound of formula (I) in crystalline form, thereby avoiding the cumbersome, expensive and inefficient use of preparative HPLC and enabling manufacture of the title compound in high chemical purity and in crystalline form.
- isolate the compound of formula (I) in different solid-state forms accessible by well-defined parameters and conditions, thereby offering a reliable and controllable process for its manufacturing and handling.

## SUMMARY OF THE INVENTION

The present invention relates to a process for the preparation of a gadolinium chelate compound of formula (I) as well as a crystalline form of said gadolinium chelate of formula (I), to a process for the preparation of said crystalline form as well as to intermediate compounds in the synthesis of said gadolinium chelate compound of formula (I) and/or its crystalline form.

## DEFINITIONS

The term "substituted" means that one or more hydrogen atoms on the designated atom or group are replaced with a selection from the indicated group, provided that the designated atom's normal valency under the existing circumstances is not exceeded. Combinations of substituents and/or variables are permissible.

The term "optionally substituted" means that the number of substituents can be equal to or different from zero.

When groups in the compounds according to the invention are substituted, it is possible for said groups to be mono-substituted or poly-substituted with substituent(s), unless otherwise specified. Within the scope of the present invention, the meanings of all groups which occur repeatedly are independent from one another. It is possible that groups in the compounds according to the invention are substituted with one, two or three identical or different substituents, particularly with one substituent.

Should a composite substituent be composed of more than one parts, e.g. ($C_1$-$C_3$-alkoxy)-($C_2$-$C_6$-alkyl)-, it is possible for the position of a given part to be at any suitable position of said composite substituent, *i.e.* the $C_1$-$C_3$-alkoxy part can be attached to any carbon atom of the $C_2$-$C_6$-alkyl part of said ($C_1$-$C_3$-alkoxy)-($C_2$-$C_6$-alkyl)- group. A hyphen at the beginning or at the end of such a composite substituent indicates the point of attachment of said composite substituent to the rest of the molecule.

The term "comprising" when used in the specification includes "consisting of".

If within the present text any item is referred to as "as mentioned herein", it means that it may be mentioned anywhere in the present text.

In the context of the present invention, the term "relative humidity" can be defined as the humidity calculated according to the following equation

$$RH = \frac{p_W}{p_{wL} = f(T)}$$

wherein pw represents the water partial pressure and pwL represents the water vapor pressure of the liquid phase.

The relative humidity as a function of the temperature and of the vapor pressure is shown in, for example: Thermische Trennverfahren - Grundlagen, Auslegung, Apparate by Klaus Sattler (K. Sattler, Thermische Trennverfahren - Grundlagen, Auslegung, Apparate, Wiley-VCH, Weinheim, Germany, 2nd Edition, 1995, p. 415).

In a "liquid-gaseous" system, the water molecules of the gas phase are in equilibrium with the water molecules in the liquid phase. The concentration of water molecules in the gas phase is expressed by the water partial pressure. At 100% water atmosphere, the water partial pressure is equal to the total pressure. In the case of a mixture of water and another gas component (e.g. nitrogen), the water partial pressure is equal to the total pressure multiplied by the mole fraction of water in the gas phase (Dalton's law). The water vapor pressure of the liquid phase is a temperature-dependent property of the substance.

In a "solid-gaseous" system, the water molecules of the gas phase are in equilibrium with the water molecules that are absorbed/adsorbed in/on the solid phase. The concentration of water molecules in the gas phase is expressed by the water partial pressure. At 100% water atmosphere, the water partial pressure is equal to the total pressure. In the case of a mixture of water and another gas component (e.g. nitrogen), the water partial pressure is equal to the total pressure multiplied by the mole fraction of the water in the gas phase (Dalton's law). The water vapor pressure of the solid phase is a temperature-dependent property of the substance. The relative humidity set in the "Dynamic Vapour Sorption" as well as in the production process refer to water in the "liquid-gaseous" system.

The terms as mentioned in the present text have the following meanings:
The term "halogen atom" means a fluorine, chlorine, bromine or iodine atom, particularly a fluorine, chlorine or bromine atom.

The term "$C_1$-$C_6$-alkyl" means a linear or branched, saturated, monovalent hydrocarbon group having 1, 2, 3, 4, 5 or 6 carbon atoms, e.g. a methyl, ethyl, propyl, isopropyl, butyl, sec-butyl, isobutyl, tert-butyl, pentyl, isopentyl, 2-methylbutyl, 1-methylbutyl, 1-ethylpropyl, 1,2-dimethylpropyl, neo-pentyl, 1,1-dimethylpropyl, hexyl, 1-methylpentyl, 2-methylpentyl, 3-methylpentyl, 4-methylpentyl, 1-ethylbutyl, 2-ethylbutyl, 1,1-dimethylbutyl, 2,2-dimethylbutyl, 3,3-dimethylbutyl, 2,3-dimethylbutyl, 1,2-dimethylbutyl or 1,3-dimethylbutyl group, or an isomer thereof. Particularly, said group has 1, 2, 3 or 4 carbon atoms ("$C_1$-$C_4$-alkyl"), *e.g.* a methyl, ethyl, propyl, isopropyl, butyl, sec-butyl isobutyl, or tert-butyl group, more particularly 1, 2 or 3 carbon atoms ("$C_1$-$C_3$-alkyl"), *e.g.* a methyl, ethyl, n-propyl or isopropyl group.

The term "$C_1$-$C_3$-haloalkyl" means a linear or branched, saturated, monovalent hydrocarbon group in which the term "$C_1$-$C_3$-alkyl" is as defined *supra,* and in which one or more of the hydrogen atoms are replaced, identically or differently, with a halogen atom. Particularly, said halogen atom is a fluorine atom. Said $C_1$-$C_3$-haloalkyl group is, for example, fluoromethyl, difluoromethyl, trifluoromethyl, 2-fluoroethyl, 2,2-difluoroethyl, 2,2,2-trifluoroethyl, pentafluoroethyl, 3,3,3-trifluoropropyl or 1,3-difluoropropan-2-yl.

The term "$C_2$-$C_6$-hydroxyalkyl" means a linear or branched, saturated, monovalent hydrocarbon group in which the term "$C_2$-$C_6$-alkyl" is defined *supra,* and in which 1, 2 or 3 hydrogen atoms are replaced with a hydroxy group, e.g. a 2-hydroxyethyl, 3-hydroxypropyl, 2-hydroxypropyl, 1-hydroxypropan-2-yl, 2,3-dihydroxypropyl, 1,3-dihydroxypropan-2-yl, 1,3-dihydroxy-2-(hydroxymethyl)propan-2-yl, 3-hydroxy-2-methyl-propyl, 2-hydroxy-2-methyl-propyl group.

The term "$C_1$-$C_3$-alkoxy" means a linear or branched, saturated, monovalent group of formula ($C_1$-$C_3$-alkyl)-O-, in which the term "$C_1$-$C_3$-alkyl" is as defined *supra, e.g.* a methoxy, ethoxy, n-propoxy or isopropoxy group.

The term "$C_3$-$C_6$-cycloalkyl" means a saturated, monovalent, mono- or bicyclic hydrocarbon ring which contains 3, 4, 5 or 6 carbon atoms ("$C_3$-$C_6$-cycloalkyl"). Said $C_3$-$C_6$-cycloalkyl group is for example, a monocyclic hydrocarbon ring, *e.g.* a cyclopropyl, cyclobutyl, cyclopentyl or cyclohexyl group.

The term "$C_1$-$C_6$", as used in the present text, *e.g.* in the context of the definition of "$C_1$-$C_6$-alkyl" means an alkyl group having a finite number of carbon atoms of 1 to 6, *i.e.* 1, 2, 3, 4, 5 or 6 carbon atoms.

Further, as used herein, the term "$C_3$-$C_6$", as used in the present text, *e.g.* in the context of the definition of "$C_3$-$C_6$-cycloalkyl", means a cycloalkyl group having a finite number of carbon atoms of 3 to 6, *i.e.* 3, 4, 5 or 6 carbon atoms.

When a range of values is given, said range encompasses each value and sub-range within said range.

For example:

"$C_1$-$C_6$" encompasses $C_1$, $C_2$, $C_3$, $C_4$, $C_5$, $C_6$, $C_1$-$C_6$, $C_1$-$C_5$, $C_1$-$C_4$, $C_1$-$C_3$, $C_1$-$C_2$, $C_2$-$C_6$, $C_2$-$C_5$, $C_2$-$C_4$, $C_2$-$C_3$, $C_3$-$C_6$, $C_3$-$C_5$, $C_3$-$C_4$, $C_4$-$C_6$, $C_4$-$C_6$, and $C_5$-$C_6$;
"$C_1$-$C_4$" encompasses $C_1$, $C_2$, $C_3$, $C_4$, $C_1$-$C_4$, $C_1$-$C_3$, $C_1$-$C_2$, $C_2$-$C_4$, $C_2$-$C_3$ and $C_3$-$C_4$;
"$C_1$-$C_3$" encompasses $C_1$, $C_2$, $C_3$, $C_1$-$C_3$, $C_1$-$C_2$ and $C_2$-$C_3$;
"$C_2$-$C_6$" encompasses $C_2$, $C_3$, $C_4$, $C_5$, $C_6$, $C_2$-$C_6$, $C_2$-$C_6$, $C_2$-$C_4$, $C_2$-$C_3$, $C_3$-$C_6$, $C_3$-$C_5$, $C_3$-$C_4$, $C_4$-$C_6$, $C_4$-$C_6$, and $C_5$-$C_6$;
"$C_3$-$C_6$" encompasses $C_3$, $C_4$, $C_5$, $C_6$, $C_3$-$C_6$, $C_3$-$C_5$, $C_3$-$C_4$, $C_4$-$C_6$, $C_4$-$C_6$, and $C_5$-$C_6$.

The compounds of this invention may contain one or more asymmetric centre, depending upon the location and nature of the various substituents desired. Asymmetric carbon atoms may be present in the (R) or (S) configuration, which can result in racemic mixtures in the case of a single asymmetric centre, and in diastereomeric mixtures in the case of multiple asymmetric centres. In certain instances, asymmetry may also be present due to restricted rotation about a given bond, for example, the central bond adjoining two substituted aromatic rings of the specified compounds.

In the context of the present invention, the compound of formula (I) can exist in different stereoisomeric, i.e., diastereomeric and/or enantiomeric forms, since it contains four asymmetric centers (marked * below):

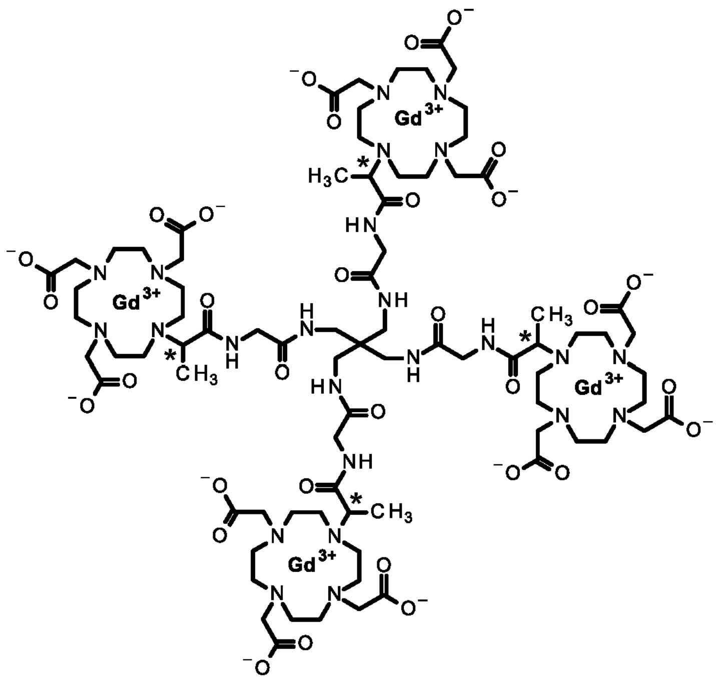

**(I)**

A skilled person will understand that the compound of formula (I) can exist in different configurations, i.e. (R, R, R, R), (S, S, S, S), (R, S, S, S), (S, R, R, R), (S, S, R, R), either in pure form or as mixtures of two or more different configurations. Preferably, the compound of formula (I) prepared according to the process of the present invention is obtained as a mixture of diastereomers. More preferably, the compound of formula (I) prepared according to the process of the present invention is obtained as a mixture of the (S, S, R, R), (S, R, R, R) + (R, S, S, S), and (R, R, R, R) + (S, S, S, S) diastereomers in a ratio of from 60:30:10 to a ratio of from 30:60:10, more preferably in a ratio of from 50:40:10 to a ratio of from 40:50:10. Also more preferably, the compound of formula (I) prepared according to the process of the present invention is obtained as a mixture of the (S, S, R, R), (S, R, R, R) + (R, S, S, S), and (R, R, R, R) + (S, S, S, S) diastereomers in a ratio of from 30:60:10 to a ratio of from 75:25:0, or in a ratio of from 40:55:5 to a ratio of from 65:35:0.

Preferred compounds are those which produce the more desirable biological activity. Separated, pure or partially purified isomers and stereoisomers or racemic or diastereomeric mixtures of the compounds of this invention are also included within the scope of the present invention. The purification and the separation of such materials can be accomplished by standard techniques known in the art.

The optical isomers can be obtained by resolution of the racemic mixtures according to conventional processes, for example, by the formation of diastereoisomeric salts using an optically active acid or base or formation of covalent diastereomers. Examples of appropriate acids are tartaric, diacetyltartaric, ditoluoyltartaric and camphorsulfonic acid. Mixtures of diastereoisomers can be separated into their individual diastereomers on the basis of their physical and/or chemical differences by methods known in the art, for example, by chromatography or fractional crystallisation. The optically active bases or acids are then liberated from the separated diastereomeric salts. A different process for separation of optical isomers involves the use of chiral chromatography (*e.g.*, chiral HPLC columns), with or without conventional derivatisation, optimally chosen to maximise the separation of the enantiomers. Suitable chiral HPLC columns are manufactured by Daicel, *e.g.*, Chiracel OD and Chiracel OJ among many others, all routinely selectable. Enzymatic separations, with or without derivatisation, are also useful. The optically active compounds of this invention can likewise be obtained by chiral syntheses utilizing optically active starting materials.

In order to limit different types of isomers from each other reference is made to IUPAC Rules Section E (Pure

Appl Chem 45, 11-30, 1976).

The present invention includes all possible stereoisomers of the compounds of the present invention as single stereoisomers, or as any mixture of said stereoisomers, *e.g.* R- or S-isomers, in any ratio. Isolation of a single stereoisomer, *e.g.* a single enantiomer or a single diastereomer, of a compound of the present invention may be achieved by any suitable state of the art method, such as chromatography, especially chiral chromatography, for example.

The present invention also relates to useful forms of the compounds as disclosed herein, such as metabolites, hydrates, solvates, salts, in particular pharmaceutically acceptable salts, and co-precipitates.

The compounds of the present invention can exist as a hydrate, or as a solvate, or as a mixture thereof, i.e., a mixed hydrate and/or a mixed solvate, particularly when the compounds of the present invention contain polar solvents, in particular water, methanol or ethanol for example as structural element of the crystal lattice of the compounds. The amount of polar solvents, in particular water, may exist in a stoichiometric or non-stoichiometric ratio. In the case of stoichiometric solvates, *e.g.*, a hydrate, hemi-, (semi-), mono-, sesqui-, di-, tri-, tetra-, penta- *etc.* solvates or hydrates, respectively, are possible. The present invention includes all such hydrates or solvates or mixtures thereof, i.e., mixed hydrates and/or mixed solvates.

Further, the compounds of the present invention can exist in the form of a salt. Said salt may be either an inorganic or organic addition salt, particularly any pharmaceutically acceptable inorganic or organic addition salt, customarily used in pharmacy.

The term "pharmaceutically acceptable salt" refers to a relatively non-toxic, inorganic or organic acid addition salt of a compound of the present invention. For example, see S. M. Berge, et al. "Pharmaceutical Salts," J. Pharm. Sci. 1977, 66, 1-19. The production of especially neutral salts is described in US 5,560,903.

Pharmaceutically acceptable salts of the compounds according to the invention include salts of mineral acids and carboxylic acids, for example, without being limited thereto, salts of hydrochloric acid, sulfuric acid, phosphoric acid, acetic acid, propionic acid, lactic acid, tartaric acid, malic acid, citric acid, fumaric acid, maleic acid, aspartic acid and glutamic acid.

Those skilled in the art will further recognize that acid addition salts of the claimed compounds may be prepared by reaction of the compounds with the appropriate inorganic or organic acid via any of a number of known methods.

The present invention includes all possible salts of the compounds of the present invention as single salts, or as any mixture of said salts, in any ratio.

In the present text, in particular in the Experimental Section, for the synthesis of intermediates and of examples of the present invention, when a compound is mentioned as a salt form with the corresponding base or acid, the exact stoichiometric composition of said salt form, as obtained by the respective preparation and/or purification process, is, in most cases, unknown.

This applies analogously to cases in which synthesis intermediates or example compounds or salts thereof have been obtained, by the preparation and/or purification processes described, as solvates, such as hydrates with (if defined) unknown stoichiometric composition.

## DESCRIPTION OF THE INVENTION

### Isolation of a compound of formula (I)

In accordance with a first aspect, the present invention relates to a process for the isolation of a compound of general formula (I),

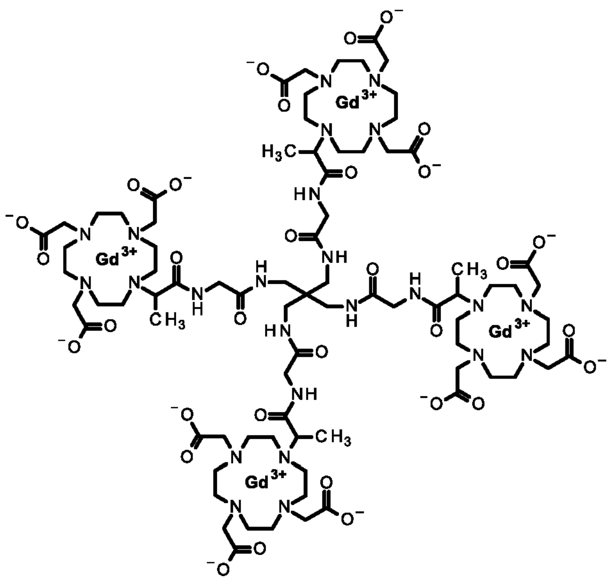

**(I)**

or its stereoisomers, tautomers, N-oxides, hydrates, solvates, or salts thereof, or mixtures of same, said process comprising

(i) providing a mixture comprising a compound of formula (I), water and at least a first organic solvent,
(ii) removing water from the mixture provided in (i),
(iii) adding a second organic solvent,
(iv) optionally adding a third organic solvent,
(v) isolating the compound of formula (I).

In a further embodiment of the first aspect, the present invention relates to a process for the preparation of a compound of general formula (I), *supra,* or its stereoisomers, tautomers, N-oxides, hydrates, solvates, or salts thereof, or mixtures of same, said process comprising

(i) providing a mixture comprising a compound of formula (I), water and at least a first organic solvent,
(ii) removing water from the mixture provided in (i),
(iii) adding a second organic solvent,
(iv) adding a third organic solvent,
(v) isolating the compound of formula (I).

In a further embodiment of the first aspect, the present invention relates to a process for the preparation of a compound of general formula (I), *supra,* or its stereoisomers, tautomers, N-oxides, hydrates, solvates, or salts thereof, or mixtures of same, said process comprising

(i) providing a mixture comprising a compound of formula (I), water and at least a first organic solvent,
(ii) removing water from the mixture provided in (i) until the water content of the mixture lies between 10% and 20% by weight (w/w),
(iii) adding a second organic solvent,
(iv) adding a third organic solvent,
(v) isolating the compound of formula (I).

In a further embodiment of the first aspect, the present invention relates to a process for the preparation of a compound of general formula (I), *supra,* or its stereoisomers, tautomers, N-oxides, hydrates, solvates, or salts thereof, or mixtures of same, said process comprising

(i) providing a mixture comprising a compound of formula (I), water and at least a first organic solvent,
(ii) removing water from the mixture provided in (i),
(iii) adding a second organic solvent, wherein said second organic solvent is a $C_1$-$C_4$ alcohol,

(iv) adding a third organic solvent,
(v) isolating the compound of formula (I).

In a further embodiment of the first aspect, the present invention relates to a process for the preparation of a compound of general formula (I), *supra,* or its stereoisomers, tautomers, N-oxides, hydrates, solvates, or salts thereof, or mixtures of same, said process comprising

(i) providing a mixture comprising a compound of formula (I), water and at least a first organic solvent,
(ii) removing water from the mixture provided in (i),
(iii) adding a second organic solvent, wherein said second organic solvent is a $C_1$-$C_4$ alcohol,
(iv) adding a third organic solvent, wherein said third organic solvent is acetone
(v) isolating the compound of formula (I).

In a further embodiment of the first aspect, the present invention relates to a process for the preparation of a compound of general formula (I), *supra,* or its stereoisomers, tautomers, N-oxides, hydrates, solvates, or salts thereof, or mixtures of same, said process comprising

(i) providing a mixture comprising a compound of formula (I), water and at least a first organic solvent, wherein said at least first organic solvent is DMSO,
(ii) removing water from the mixture provided in (i),
(iii) adding a second organic solvent, wherein said second organic solvent is a $C_1$-$C_5$ alcohol,
(iv) adding a third organic solvent, wherein said third organic solvent is acetone
(v) isolating the compound of formula (I).

In a further embodiment of the first aspect, the present invention relates to a process for the preparation of a compound of general formula (I), *supra,* or its stereoisomers, tautomers, N-oxides, hydrates, solvates, or salts thereof, or mixtures of same, said process comprising

(i) providing a mixture comprising a compound of formula (I), water and at least a first organic solvent,
(ii) removing water from the mixture provided in (i),
(iii) adding a second organic solvent, wherein said second organic solvent is an alcohol selected from the list consisting of ethanol, n-propanol and iso-propanol,
(iv) adding a third organic solvent, wherein said third organic solvent is acetone
(v) isolating the compound of formula (I).

In a further embodiment of the first aspect, the present invention relates to a process for the preparation of a compound of general formula (I), *supra,* or its stereoisomers, tautomers, N-oxides, hydrates, solvates, or salts thereof, or mixtures of same, said process comprising

(i) providing a mixture comprising a compound of formula (I), water and at least a first organic solvent, wherein said at least first organic solvent is DMSO,
(ii) removing water from the mixture provided in (i),
(iii) adding a second organic solvent, wherein said second organic solvent is an alcohol selected from the list consisting of ethanol, n-propanol and iso-propanol,
(iv) adding a third organic solvent, wherein said third organic solvent is acetone
(v) isolating the compound of formula (I).

In a further embodiment of the first aspect, the present invention relates to a process for the preparation of a compound of general formula (I), *supra,* or its stereoisomers, tautomers, N-oxides, hydrates, solvates, or salts thereof, or mixtures of same, said process comprising

(i) providing a mixture comprising a compound of formula (I), water and at least a first organic solvent,
(ii) removing water from the mixture provided in (i) until the water content of the mixture lies between 10% and 20% by weight (w/w),
(iii) adding a second organic solvent, wherein said second organic solvent is an alcohol selected from the list consisting of ethanol, n-propanol and iso-propanol,
(iv) adding a third organic solvent, wherein said third organic solvent is acetone
(v) isolating the compound of formula (I).

In a further embodiment of the first aspect, the present invention relates to a process for the preparation of a compound of general formula (I), *supra,* or its stereoisomers, tautomers, N-oxides, hydrates, solvates, or salts thereof, or mixtures of same, said process comprising

(i) providing a mixture comprising a compound of formula (I), water and at least a first organic solvent,
(ii) removing water from the mixture provided in (i) until the water content of the mixture lies between 10% and 20% by weight (w/w),
(iii) adding a second organic solvent, wherein said second organic solvent is ethanol,
(iv) adding a third organic solvent, wherein said third organic solvent is acetone,
(v) isolating the compound of formula (I).

In a further embodiment of the first aspect, the present invention relates to a process for the preparation of a compound of general formula (I), *supra,* or its stereoisomers, tautomers, N-oxides, hydrates, solvates, or salts thereof, or mixtures of same, said process comprising

(i) providing a mixture comprising a compound of formula (I), water and at least a first organic solvent, wherein said at least first organic solvent is DMSO,
(ii) removing water from the mixture provided in (i) until the water content of the mixture lies between 10% and 20% by weight (w/w),
(iii) adding a second organic solvent, wherein said second organic solvent is ethanol,
(iv) adding a third organic solvent, wherein said third organic solvent is acetone,
(v) isolating the compound of formula (I).

Step (i) of the process according to the present invention comprises providing a mixture comprising a compound of formula (I), water and at least a first organic solvent. Preferably, said at least first organic solvent is DMSO. More preferably, the mixture comprising a compound of formula (I), water and at least a first organic solvent is the mixture obtained in the reaction leading to the formation of the compound of formula (I).

In step (ii), water is removed from the mixture provided in step (i). Preferably, water is removed from the mixture until the water content of the mixture lies between 10% and 20% by weight (w/w).

In step (iii), a second organic solvent is added to the mixture. Preferably, said second organic solvent is an alcohol selected from the list consisting of ethanol, n-propanol and iso-propanol. More preferably, said second organic solvent is ethanol.

In step (iv), a third organic solvent is added to the mixture. Preferably, said third organic solvent is selected from the list consisting of acetone, methyl-ethyl-ketone and methyl-tert-butyl ether. More preferably, said third organic solvent is acetone. After the addition of the third organic solvent, a solid comprising the compound of formula (I) as a crude material is preferably obtained after step (iv). Additional and/or optional steps such as diafiltration, ultrafiltration, nanofiltration, extraction, treatment with activated charcoal, solvent evaporation, exchange resin treatment, ion exchange resin treatment and the like may be necessary and can be performed after the addition of the third organic solvent in step (iv) and prior to the isolation in step (v) or they may be comprised as part of the isolation process in step (v).

In step (v), the compound of formula (I) is isolated. Preferably, the compound of formula (I) is isolated as a solid. In the context of the present invention, the isolation of the compound of formula (I) may optionally comprise additional steps, such as diafiltration, ultrafiltration, nanofiltration, extraction, treatment with activated charcoal, solvent evaporation, exchange resin treatment, ion exchange resin treatment and the like. In a particularly preferred embodiment, the compound of formula (I) is isolated in step (v) by a process comprising diafiltration/ultrafiltration in water.

In a further embodiment of the first aspect, the present invention relates to a process for the preparation of a compound of general formula (I), *supra,* or its stereoisomers, tautomers, N-oxides, hydrates, solvates, or salts thereof, or mixtures of same, said process comprising

(i) providing a mixture comprising a compound of formula (I), water and at least a first organic solvent,
(ii) removing water from the mixture provided in (i) until the water content of the mixture lies between 10% and 20% by weight (w/w),
(iii) adding a second organic solvent, wherein said second organic solvent is an alcohol selected from the list consisting of ethanol, n-propanol and iso-propanol,
(iv) adding a third organic solvent, wherein said third organic solvent is acetone
(v) isolating the compound of formula (I) by a process comprising diafiltration, ultrafiltration or the use of an ion exchange resin.

In a further embodiment of the first aspect, the present invention relates to a process for the preparation of a

compound of general formula (I), *supra,* or its stereoisomers, tautomers, N-oxides, hydrates, solvates, or salts thereof, or mixtures of same, said process comprising

(i) providing a mixture comprising a compound of formula (I), water and at least a first organic solvent, wherein said at least first organic solvent is DMSO,
(ii) removing water from the mixture provided in (i) until the water content of the mixture lies between 10% and 20% by weight (w/w),
(iii) adding a second organic solvent, wherein said second organic solvent is ethanol,
(iv) adding a third organic solvent, wherein said third organic solvent is acetone,
(v) isolating the compound of formula (I) by a process comprising diafiltration, ultrafiltration or the use of an ion exchange resin.

**Crystalline Forms I and II of the compound of Formula (I)**

It has been surprisingly found that the compound of formula (I) exists in crystalline form, i.e. as a crystalline material, i.e. as a crystalline solid. Thus, the present invention relates to the compound of formula (I) in crystalline form, i.e. as a crystalline material, i.e. as a crystalline solid. It has been found that the compound of formula (I) can exist in at least two crystalline Forms I and II either in pure form or as a mixture thereof in any ratio.

The crystalline forms of the compound of formula (I) may be characterized by analytical methods well known in the field of the pharmaceutical industry for characterizing solids. Such methods comprise but are not limited to powder X-ray diffraction (PXRD/XRPD), Fourier transform Infrared (FTIR) spectroscopy, differential scanning calorimetry (DSC), thermogravimetric analysis (TGA) and dynamic vapour sorption (DVS). The crystalline forms of the compound of formula (I) may be characterized by one of the aforementioned methods or by combining two or more of them. In particular, the crystalline forms of the compound of formula (I) may be characterized by one of the following embodiments or by combining two or more of the following embodiments.

**Crystalline Form I of the compound of formula (I)**

The crystalline Form I of the compound of formula (I) is characterized by having a powder X-ray diffractogram comprising reflections at 2-Theta angles of (6.8 ± 0.2)°, (9.1 ± 0.2)° and (11.4 ± 0.2)°, when measured at room temperature with Cu-Kalpha$_1$ radiation having a wavelength of 0.15419 nm. Preferably, the crystalline Form I of the compound of formula (I) is characterized by having a powder X-ray diffractogram comprising reflections at 2-Theta angles of (6.8 ± 0.2)°, (9.1 ± 0.2)°, (10.1 ± 0.2)°, (11.4 ± 0.2)° and (12.0 ± 0.2)°, when measured at room temperature with Cu-Kalpha$_1$ radiation having a wavelength of 0.15419 nm. Preferably, the crystalline Form I of the compound of formula (I) is characterized by having a powder X-ray diffractogram comprising reflections at 2-Theta angles of (6.8 ± 0.2)°, (9.1 ± 0.2)°, (10.1 ± 0.2)°, (11.4 ± 0.2)°, (12.0 ± 0.2)°, (14.4 ± 0.2)° and (23.5 ± 0.2)°, when measured at room temperature with Cu-Kalpha$_1$ radiation having a wavelength of 0.15419 nm. Preferably, the crystalline Form I of the compound of formula (I) is characterized by having a powder X-ray diffractogram comprising reflections at 2-Theta angles of (6.8 ± 0.2)°, (9.1 ± 0.2)°, (10.1 ± 0.2)°, (11.4 ± 0.2)°, (12.0 ± 0.2)°, (13.6 ± 0.2)°, (14.4 ± 0.2)°, (17.2 ± 0.2)°, (23.5 ± 0.2)° and (29.0 ± 0.2)°, when measured at room temperature with Cu-Kalpha$_1$ radiation having a wavelength of 0.15419 nm.

Alternatively or additionally, the crystalline Form I of the compound of formula (I) is characterized by having a powder X-ray diffractogram essentially the same as displayed in Figure 3 of the present invention, when measured at room temperature with Cu-Kalpha$_1$ radiation having a wavelength of 0.15419 nm.

Alternatively or additionally, the crystalline Form I of the compound of formula (I) is characterized by having a Fourier transform Infrared spectrum comprising bands at wavenumbers of (1083 ± 2) $cm^{-1}$, (1383 ± 2) $cm^{-1}$, and (1594 ± 2) $cm^{-1}$, when measured at room temperature with a diamond ATR cell. Preferably, the crystalline Form I of the compound of formula (I) is characterized by having a Fourier transform Infrared spectrum comprising peaks at wavenumbers of (713 ± 2) $cm^{-1}$, (1083 ± 2) $cm^{-1}$, (1383 ± 2) $cm^{-1}$, (1557 ± 2) $cm^{-1}$ and (1594 ± 2) $cm^{-1}$, when measured at room temperature with a diamond ATR cell. Preferably, the crystalline Form I of the compound of formula (I) is characterized by having a Fourier transform Infrared spectrum comprising bands at wavenumbers of (494 ± 2) $cm^{-1}$, (564 ± 2) $cm^{-1}$, (713 ± 2) $cm^{-1}$, (1083 ± 2) $cm^{-1}$, (1383 ± 2) $cm^{-1}$, (1557 ± 2) $cm^{-1}$ and (1594 ± 2) $cm^{-1}$, when measured at room temperature with a diamond ATR cell. Preferably, the crystalline Form I of the compound of formula (I) is characterized by having a Fourier transform Infrared spectrum comprising bands at wavenumbers of (494 ± 2) $cm^{-1}$, (564 ± 2) $cm^{-1}$, (713 ± 2) $cm^{-1}$, (935 ± 2) $cm^{-1}$, (1083 ± 2) $cm^{-1}$, (1383 ± 2) $cm^{-1}$, (1557 ± 2) $cm^{-1}$, (1594 ± 2) $cm^{-1}$, (1661 ± 2) $cm^{-1}$ and (3313 ± 2) $cm^{-1}$, when measured at room temperature with a diamond ATR cell.

Alternatively, the crystalline Form I of the compound of formula (I) is characterized by having a Fourier transform Infrared spectrum essentially the same as displayed in Figure 4 of the present invention, when measured at room temperature with a diamond ATR cell.

Alternatively or additionally, the crystalline Form I of the compound of formula (I) is characterized by having a Raman spectrum comprising peaks at wavenumbers of (938 ± 2) $cm^{-1}$, (1460 ± 2) $cm^{-1}$ and (2890 ± 2) $cm^{-1}$, when measured at room temperature with a laser at a wavelength of 1064nm. Preferably, the crystalline Form I of the compound of formula (I) is characterized by having a Raman spectrum comprising bands at wavenumbers of (387 ± 2) $cm^{-1}$, (834 ± 2) $cm^{-1}$, (938 ± 2) $cm^{-1}$, (1460 ± 2) $cm^{-1}$ and (2890 ± 2) $cm^{-1}$, when measured at room temperature with a laser at a wavelength of 1064nm. Preferably, the crystalline Form I of the compound of formula (I) is characterized by having a Raman spectrum comprising peaks at wavenumbers of (387 ± 2) $cm^{-1}$, (834 ± 2) $cm^{-1}$, (938 ± 2) $cm^{-1}$, (1305 ± 2) $cm^{-1}$, (1460 ± 2) $cm^{-1}$, (2890 ± 2) $cm^{-1}$ and (2965 ± 2) $cm^{-1}$, when measured at room temperature with a laser at a wavelength of 1064nm. Preferably, the crystalline Form I of the compound of formula (I) is characterized by having a Raman spectrum comprising peaks at wavenumbers of (387 ± 2) $cm^{-1}$, (834 ± 2) $cm^{-1}$, (938 ± 2) $cm^{-1}$, (1241 ± 2) $cm^{-1}$, (1305 ± 2) $cm^{-1}$, (1393 ± 2) $cm^{-1}$, (1422 ± 2) $cm^{-1}$, (1460 ± 2) $cm^{-1}$, (2890 ± 2) $cm^{-1}$ and (2965 ± 2) $cm^{-1}$, when measured at room temperature with a laser at a wavelength of 1064nm.

Alternatively, the crystalline Form I of the compound of formula (I) is characterized by having a Raman spectrum essentially the same as displayed in Figure 5 of the present invention, when measured at room temperature with a laser at a wavelength of 1064nm.

**Crystalline Form II of the compound of formula (I)**

The crystalline Form II of the compound of formula (I) is characterized by having a powder X-ray diffractogram comprising reflections at 2-Theta angles of (10.2 ± 0.2)°, (10.8 ± 0.2)° and (11.3 ± 0.2)°, when measured at room temperature with Cu-$Kalpha_1$ radiation having a wavelength of 0.15419 nm. Preferably, the crystalline Form II of the compound of formula (I) is characterized by having a powder X-ray diffractogram comprising reflections at 2-Theta angles of (7.3 ± 0.2)°, (10.2 ± 0.2)°, (10.8 ± 0.2)°, (11.3 ± 0.2)° and (13.3 ± 0.2)°, when measured at room temperature with Cu-$Kalpha_1$ radiation having a wavelength of 0.15419 nm. Preferably, the crystalline Form II of the compound of formula (I) is characterized by having a powder X-ray diffractogram comprising reflections at 2-Theta angles of (7.1 ± 0.2)°, (7.3 ± 0.2)°, (10.2 ± 0.2)°, (10.8 ± 0.2)°, (11.3 ± 0.2)°, (13.3 ± 0.2)° and (15.1 ± 0.2)°, when measured at room temperature with Cu-$Kalpha_1$ radiation having a wavelength of 0.15419 nm. Preferably, the crystalline Form II of the compound of formula (I) is characterized by having a powder X-ray diffractogram comprising reflections at 2-Theta angles of (5.4 ± 0.2)°, (7.1 ± 0.2)°, (7.3 ± 0.2)°, (10.2 ± 0.2)°, (10.8 ± 0.2)°, (11.3 ± 0.2)°, (13.3 ± 0.2)°, (14.8 ± 0.2)°, (15.1 ± 0.2)° and (15.6 ± 0.2)°, when measured at room temperature with Cu-Kalpha1 radiation having a wavelength of 0.15419 nm.

Alternatively, the crystalline Form II of the compound of formula (I) is characterized by having a powder X-ray diffractogram essentially the same as displayed in Figure 8 of the present invention, when measured at room temperature with Cu-$Kalpha_1$ radiation having a wavelength of 0.15419 nm.

Alternatively or additionally, the crystalline Form II of the compound of formula (I) is characterized by having a Fourier transform Infrared spectrum comprising bands at wavenumbers of (1082 ± 2) $cm^{-1}$, (1380 ± 2) $cm^{-1}$ and (1596 ± 2) $cm^{-1}$, when measured at room temperature with a diamond ATR cell. Preferably, the crystalline Form II of the compound of formula (I) is characterized by having a Fourier transform Infrared spectrum comprising bands at wavenumbers of (716 ± 2) $cm^{-1}$, (1082 ± 2) $cm^{-1}$, (1380 ± 2) $cm^{-1}$, (1557 ± 2) $cm^{-1}$ and (1596 ± 2) $cm^{-1}$, when measured at room temperature with a diamond ATR cell. Preferably, the crystalline Form II of the compound of formula (I) is characterized by having a Fourier transform Infrared spectrum comprising bands at wavenumbers of (495 ± 2) $cm^{-1}$, (716 ± 2) $cm^{-1}$, (1082 ± 2) $cm^{-1}$, (1316 ± 2) $cm^{-1}$, (1380 ± 2) $cm^{-1}$, (1557 ± 2) $cm^{-1}$ and (1596 ± 2) $cm^{-1}$, when measured at room temperature with a diamond ATR cell. Preferably, the crystalline Form II of the compound of formula (I) is characterized by having a Fourier transform Infrared spectrum comprising bands at wavenumbers of (495 ± 2) $cm^{-1}$, (716 ± 2) $cm^{-1}$, (933 ± 2) $cm^{-1}$, (1082 ± 2) $cm^{-1}$, (1316 ± 2) $cm^{-1}$, (1380 ± 2) $cm^{-1}$, (1557 ± 2) $cm^{-1}$, (1596 ± 2) $cm^{-1}$, (1660 ± 2) $cm^{-1}$ and (3291 ± 2) $cm^{-1}$, when measured at room temperature with a diamond ATR cell.

Alternatively, the crystalline Form II of the compound of formula (I) is characterized by having a Fourier transform Infrared spectrum essentially the same as displayed in Figure 9 of the present invention, when measured at room temperature with a diamond ATR cell.

Alternatively or additionally, the crystalline Form II of the compound of formula (I) is characterized by having a Raman spectrum comprising bands at wavenumbers of (935 ± 2) $cm^{-1}$, (1472 ± 2) $cm^{-1}$ and (2888 ± 2) $cm^{-1}$, when measured at room temperature with a laser at a wavelength of 1064nm. Preferably, the crystalline Form II of the compound of formula (I) is characterized by having a Raman spectrum comprising bands at wavenumbers of (390 ± 2) $cm^{-1}$, (833 ± 2) $cm^{-1}$, (935 ± 2) $cm^{-1}$, (1472 ± 2) $cm^{-1}$ and (2888 ± 2) $cm^{-1}$, when measured at room temperature with a laser at a wavelength of 1064nm. Preferably, the crystalline Form II of the compound of formula (I) is characterized by having a Raman spectrum comprising bands at wavenumbers of (390 ± 2) $cm^{-1}$, (833 ± 2) $cm^{-1}$, (935 ± 2) $cm^{-1}$, (1277 ± 2) $cm^{-1}$, (1472 ± 2) $cm^{-1}$, (2888 ± 2) $cm^{-1}$ and (2951 ± 2) $cm^{-1}$, when measured at room temperature with a laser at a wavelength of 1064nm. Preferably, the crystalline Form II of the compound of formula (I) is characterized by having a Raman spectrum comprising bands at wavenumbers of (390 ± 2) $cm^{-1}$, (833 ± 2) $cm^{-1}$, (935 ± 2) $cm^{-1}$, (1244 ± 2)

$cm^{-1}$, (1277 ± 2) $cm^{-1}$, (1390 ± 2) $cm^{-1}$, (1429 ± 2) $cm^{-1}$, (1472 ± 2) $cm^{-1}$, (2888 ± 2) $cm^{-1}$ and (2951 ± 2) $cm^{-1}$, when measured at room temperature with a laser at a wavelength of 1064nm.

Alternatively, the crystalline Form II of the compound of formula (I) is characterized by having a Raman spectrum essentially the same as displayed in Figure 10 of the present invention, when measured at room temperature with a laser at a wavelength of 1064nm.

**Isolation of the compound of formula (I) in crystalline Form I**

In step (v), the compound of formula (I) is isolated. In the context of the present invention, step (v) can be understood as being part of a broader process also comprising steps (i), (ii), (iii) and (iv) as well as a standalone process comprising the individual sub-steps (v-1) to (v-3) described herein below leading to the isolation of the compound of formula (I), preferably leading to the selective isolation of the compound of formula (I) in crystalline Form I or in crystalline Form II.

In a preferred embodiment, the isolation of the compound of formula (I) in step (v) comprises the following steps:

(v-1) providing an aqueous mixture comprising the compound of formula (I),
(v-2) adding an organic solvent,
(v-3) drying the solid obtained in (v-2).

In a further preferred embodiment, the isolation of the compound of formula (I) in step (v) comprises the following steps:

(v-1) providing an aqueous mixture comprising the compound of formula (I),
(v-2) adding an organic solvent selected from the group consisting of ethanol, n-propanol and iso-propanol or a mixture thereof,
(v-3) drying the solid obtained in (v-2).

In a further preferred embodiment, the isolation of the compound of formula (I) in step (v) comprises the following steps:

(v-1) providing an aqueous mixture comprising the compound of formula (I),
(v-2) adding ethanol,
(v-3) drying the solid obtained in (v-2).

In step (v-1), an aqueous mixture comprising the compound of formula (I) is provided. Preferably, the only solvent in the aqueous mixture comprising the compound of formula (I) is water. Preferably, the compound of formula (I) is completely dissolved in the aqueous mixture. Thus preferably, in step (v-1) the aqueous mixture comprising the compound of formula (I) is an aqueous solution of the compound of formula (I) in water. In order to achieve complete dissolution of the compound of formula (I) and/or to obtain an aqueous mixture or aqueous solution suitable to isolate the compound of formula (I) in crystalline Form I or in crystalline Form II, processes such as filtration, diafiltration, ultrafiltration, nanofiltration, treatment with an ion exchange resin and the like may be necessary. Preferably, the content of the compound of formula (I) in the aqueous mixture provided in step (v-1) is in the range of from 30% to 70% by weight (w/w), more preferably of from 40% to 60% by weight (w/w), even more preferably of from 45% to 55% by weight (w/w).

In step (v-2), an organic solvent selected from the group consisting of ethanol, n-propanol and iso-propanol or a mixture thereof is added to the mixture provided in step (v-1). Preferably, the solvent is ethanol or iso-propanol or a mixture thereof. More preferably, the solvent is ethanol. Preferably, the organic solvent is added until the water content of the mixture is in the range of from 5% to 25% by weight (w/w), more preferably of from 7% to 20% by weight (w/w).

In a further preferred embodiment, the isolation of the compound of formula (I) in step (v) comprises the following steps:

(v-1) providing a mixture comprising the compound of formula (I) in water,
(v-2) adding ethanol,
(v-3) drying the solid obtained in (v-2).

In a further preferred embodiment, the isolation of the compound of formula (I) in step (v) comprises the following steps:

(v-1) providing a solution of the compound of formula (I) in water,

(v-2) adding ethanol,
(v-3) drying the solid obtained in (v-2).

In step (v-2), the organic solvent is preferably added at a temperature of from 45 °C to 75 °C, more preferably of from 50 °C to 70 °C, more preferably of from 55 °C to 65 °C. Especially preferred is the addition of ethanol, which is carried out at a temperature as described above. After the addition of the organic solvent, the water content of the mixture is preferably in the range of from 5% to 25% by weight (w/w), more preferably of from 7% to 20% by weight (w/w).

Upon addition of the organic solvent in step (v-2), a solid is obtained, which is dried in step (v-3).

In one embodiment, the compound of formula (I) is isolated in step (v) as a crystalline material, i.e. as a solid in a crystalline form (Form I) characterized by having a powder X-ray diffractogram comprising reflections at 2-Theta angles of (6.8 ± 0.2)°, (9.1 ± 0.2)°, (10.1 ± 0.2)°, (11.4 ± 0.2)° and (12.0 ± 0.2)°, when measured at room temperature with Cu-Kalpha1 radiation having a wavelength of 0.15419 nm.

In one embodiment, the solid is dried at a relative humidity of from 18% to 70%, preferably of from 30% to 65%, upon which the compound of formula (I) is obtained in crystalline Form I. Thus, step (v-3) comprises drying the resulting solid from step (v-2) at a relative humidity of from 18% to 70%, preferably of from 30% to 65% to yield the compound of formula (I) in crystalline Form I. Additionally or alternatively, step (v-3) comprises drying the resulting solid from step (v-2) at a relative humidity of from 18% to 70%, preferably of from 30% to 65% to yield the compound of formula (I) in crystalline Form I comprising reflections at 2-Theta angles of (6.8 ± 0.2)°, (9.1 ± 0.2)°, (10.1 ± 0.2)°, (11.4 ± 0.2)° and (12.0 ± 0.2)°, when measured at room temperature with Cu-Kalpha1 radiation having a wavelength of 0.15419 nm. Preferably, the drying process is carried out at the relative humidity described above for a time of from 1 h to 48 h.

In a preferred embodiment, step (v) comprises the isolation of the compound of formula (I) in crystalline Form I by:

(v-1) providing an aqueous mixture comprising the compound of formula (I),
(v-2) adding ethanol,
(v-3) drying the solid obtained in (v-2) at a relative humidity of from 18% to 70%, preferably of from 30% to 65%.

In a further preferred embodiment, step (v) comprises the isolation of the compound of formula (I) in crystalline Form I by:

(v-1) providing an aqueous solution of the compound of formula (I),
(v-2) adding ethanol,
(v-3) drying the solid obtained in (v-2) at a relative humidity of from 18% to 70%, preferably of from 30% to 65%.

In a further preferred embodiment, step (v) comprises the isolation of the compound of formula (I) in crystalline Form I by:

(v-1) providing an aqueous mixture comprising the compound of formula (I) in the range of from 30% to 70% by weight (w/w), more preferably of from 40% to 60% by weight (w/w), even more preferably of from 45% to 55% by weight (w/w),
(v-2) adding ethanol,
(v-3) drying the solid obtained in (v-2) at a relative humidity of from 18% to 70%, preferably of from 30% to 65%.

In a further preferred embodiment, step (v) comprises the isolation of the compound of formula (I) in crystalline Form I by:

(v-1) providing an aqueous mixture comprising the compound of formula (I) in the range of from 30% to 70% by weight (w/w), more preferably of from 40% to 60% by weight (w/w), even more preferably of from 45% to 55% by weight (w/w),
(v-2) adding ethanol until the water content of the mixture is in the range of from 5% to 25% by weight (w/w), more preferably of from 7% to 20% by weight (w/w),
(v-3) drying the solid obtained in (v-2) at a relative humidity of from 18% to 70%, preferably of from 30% to 65%.

In a further preferred embodiment, step (v) comprises the isolation of the compound of formula (I) in crystalline Form I by:

(v-1) providing an aqueous mixture comprising the compound of formula (I) in the range of from 30% to 70% by weight (w/w), more preferably of from 40% to 60% by weight (w/w), most preferably of from 45% to 55% by weight (w/w),

(v-2) adding ethanol until the water content of the mixture is in the range of from 5 to 25% by weight (w/w), more preferably of from 7% to 20% by weight (w/w),
(v-3) drying the solid obtained in (v-2) at a relative humidity of from 18% to 70%, preferably of from 30% to 65% for a time of from 1 h to 48 h.

In a further preferred embodiment, step (v) comprises the isolation of the compound of formula (I) in crystalline Form I by:

(v-1) providing an aqueous solution of the compound of formula (I) in the range of from 30% to 70% by weight (w/w), more preferably of from 40% to 60% by weight (w/w), most preferably of from 45% to 55% by weight (w/w),
(v-2) adding ethanol until the water content of the mixture is in the range of from 5 to 25% by weight (w/w), more preferably of from 7% to 20% by weight (w/w),
(v-3) drying the solid obtained in (v-2) at a relative humidity of from 18% to 70%, preferably of from 30% to 65% for a time of from 1 h to 48 h.

**Isolation of the compound of formula (I) in crystalline Form II**

In a further embodiment, the compound of formula (I) is isolated in step (v) as a crystalline material, i.e. as a solid in a crystalline form (Form II) characterized by having a powder X-ray diffractogram comprising reflections at 2-Theta angles of (7.3 $\pm$ 0.2)°, (10.2 $\pm$ 0.2)°, (10.8 $\pm$ 0.2)°, (11.3 $\pm$ 0.2)° and (13.3 $\pm$ 0.2)°, when measured at room temperature with Cu-Kalpha1 radiation having a wavelength of 0.15419 nm.

In a further embodiment, the solid is first dried at a relative humidity of from 0% to 25%, preferably of from 2% to 18%, more preferably of from 5% to 16% and then further dried by adjusting the relative humidity until a final value of from 18% to 70%, preferably of from 25% to 70%, more preferably of from 30% to 70% is reached, upon which the compound of formula (I) is obtained in crystalline Form II.

Thus, step (v-3) comprises first drying the resulting solid from step (v-2) at a relative humidity of from 0% to 25%, preferably of from 2% to 18%, more preferably of from 5% to 16% followed by a second drying process by adjusting the relative humidity until a final value of from 18% to 70%, preferably of from 25% to 70%, more preferably of from 30% to 70% is reached, upon which the compound of formula (I) is obtained in crystalline Form II.

Additionally or alternatively, step (v-3) comprises first drying the resulting solid from step (v-2) at a relative humidity of from 0% to 25%, preferably of from 2% to 18%, more preferably of from 5% to 16% followed by a second drying process by adjusting the relative humidity until a final value of from 18% to 70%, preferably of from 25% to 70%, more preferably of from 30% to 70% is reached, upon which the compound of formula (I) is obtained in crystalline Form II comprising reflections at 2-Theta angles of 7.3 $\pm$ 0.2)°, (10.2 $\pm$ 0.2)°, (10.8 $\pm$ 0.2)°, (11.3 $\pm$ 0.2)° and (13.3 $\pm$ 0.2)°, when measured at room temperature with Cu-Kalpha1 radiation having a wavelength of 0.15419 nm.

In some cases, the formation of crystalline material was observed during the second drying process at low relative humidities which is not allocatable to Form I and Form II. An X-Ray Powder Diffractogram of this material is shown in Figure 33. This crystalline form may or may not be formed during the course of the first and/or second drying process and, if formed, may do so in different amounts and concomitantly with other species, such as crystalline Form II of the compound of formula (I) depending on the specific conditions in each case. Notwithstanding, adjusting the relative humidity of the second drying process until a final value of from 18% to 70%, preferably of from 25% to 70%, more preferably of from 30% to 70% is reached ensures the reliable conversion into and preparation of crystalline Form II of the compound of formula (I).

Preferably, the first drying process is carried out at the relative humidity described above for a time of from 0.5 h to 24 h, and the second drying process is carried out at the relative humidity described above for a time of from 0.5 h to 48 h.

In a preferred embodiment, step (v) comprises the isolation of the compound of formula (I) in crystalline Form II by:

(v-1) providing an aqueous mixture comprising the compound of formula (I),
(v-2) adding ethanol,
(v-3) drying the solid obtained in (v-2) first at a relative humidity of from 0% to 25%, preferably of from 2% to 18%, more preferably of from 5% to 16% followed by a second drying process by adjusting the relative humidity until a final value of from 18% to 70%, preferably of from 25% to 70%, more preferably of from 30% to 70% is reached.

In a preferred embodiment, step (v) comprises the isolation of the compound of formula (I) in crystalline Form II by:

(v-1) providing an aqueous solution of the compound of formula (I),
(v-2) adding ethanol,

(v-3) drying the solid obtained in (v-2) first at a relative humidity of from 0% to 25%, preferably of from 2% to 18%, more preferably of from 5% to 16% followed by a second drying process by adjusting the relative humidity until a final value of from 18% to 70%, preferably of from 25% to 70%, more preferably of from 30% to 70% is reached.

In a further preferred embodiment, step (v) comprises the isolation of the compound of formula (I) in crystalline Form II by:

(v-1) providing an aqueous mixture comprising the compound of formula (I) in the range of from 30% to 70% by weight (w/w), more preferably of from 40% to 60% by weight (w/w), even more preferably of from 45% to 55% by weight (w/w),
(v-2) adding ethanol,
(v-3) drying the solid obtained in (v-2) first at a relative humidity of from 0% to 25%, preferably of from 2% to 18%, more preferably of from 5% to 16% followed by a second drying process by adjusting the relative humidity until a final value of from 18% to 70%, preferably of from 25% to 70%, more preferably of from 30% to 70% is reached.

In a further preferred embodiment, step (v) comprises the isolation of the compound of formula (I) in crystalline Form II by:

(v-1) providing an aqueous mixture comprising the compound of formula (I) in the range of from 30% to 70% by weight (w/w), more preferably of from 40% to 60% by weight (w/w), even more preferably of from 45% to 55% by weight (w/w),
(v-2) adding ethanol until the water content of the mixture is in the range of from 5% to 25% by weight (w/w), more preferably of from 7% to 20% by weight (w/w),
(v-3) drying the solid obtained in (v-2) first at a relative humidity of from 0% to 25%, preferably of from 2% to 18%, more preferably of from 5% to 16% followed by a second drying process by adjusting the relative humidity until a final value of from 18% to 70%, preferably of from 25% to 70%, more preferably of from 30% to 70% is reached.

In a further preferred embodiment, step (v) comprises the isolation of the compound of formula (I) in crystalline Form II by:

(v-1) providing an aqueous solution of the compound of formula (I) in the range of from 30% to 70% by weight (w/w), more preferably of from 40% to 60% by weight (w/w), even more preferably of from 45% to 55% by weight (w/w),
(v-2) adding ethanol until the water content of the mixture is in the range of from 5% to 25% by weight (w/w), more preferably of from 7% to 20% by weight (w/w),
(v-3) drying the solid obtained in (v-2) first at a relative humidity of from 0% to 25%, preferably of from 2% to 18%, more preferably of from 5% to 16% followed by a second drying process by adjusting the relative humidity until a final value of from 18% to 70%, preferably of from 25% to 70%, more preferably of from 30% to 70% is reached.

In a further preferred embodiment, step (v) comprises the isolation of the compound of formula (I) in crystalline Form II by:

(v-1) providing an aqueous mixture comprising the compound of formula (I) in the range of from 30% to 70% by weight (w/w), more preferably of from 40% to 60% by weight (w/w), even more preferably of from 45% to 55% by weight (w/w),
(v-2) adding ethanol until the water content of the mixture is in the range of from 5% to 25% by weight (w/w), more preferably of from 7% to 20% by weight (w/w),
(v-3) drying the solid obtained in (v-2) first at a relative humidity of from 0% to 25%, preferably of from 2% to 18%, more preferably of from 5% to 16% for a time of from 0.5 h to 24 h, followed by a second drying process by adjusting the relative humidity until a final value of from 18% to 70%, preferably of from 25% to 70%, more preferably of from 30% to 70% is reached for a time of from 0.5 h to 48 h.

**Process for the preparation of crystalline Form I of the compound of formula (I)**

The present invention provides a process for the selective preparation of the compound of formula (I) in crystalline Form I. Specifics to the process according to steps (i) to (iv), *supra,* apply to the process described herewith, as do specifics to the process according to steps (v-1) to (v-3), *supra,* for the isolation of crystalline Form I of the compound of formula (I).
In a preferred embodiment of the first aspect, the present invention relates to a process for the preparation of

a compound of general formula (I), *supra,* or its stereoisomers, tautomers, N-oxides, hydrates, solvates, or salts thereof, or mixtures of same, said process comprising

(i) providing a mixture comprising a compound of formula (I), water and at least a first organic solvent,
(ii) removing water from the mixture provided in (i),
(iii) adding a second organic solvent,
(iv) adding a third organic solvent,
(v) isolating the compound of formula (I) in crystalline Form I by a process comprising:

(v-1) providing an aqueous mixture comprising the compound of formula (I),
(v-2) adding ethanol,
(v-3) drying the solid obtained in (v-2) at a relative humidity of from 18% to 70%, preferably of from 30% to 65%.

In a preferred embodiment of the first aspect, the present invention relates to a process for the preparation of a compound of general formula (I), *supra,* or its stereoisomers, tautomers, N-oxides, hydrates, solvates, or salts thereof, or mixtures of same, said process comprising

(i) providing a mixture comprising a compound of formula (I), water and at least a first organic solvent,
(ii) removing water from the mixture provided in (i),
(iii) adding a second organic solvent,
(iv) adding a third organic solvent,
(v) isolating the compound of formula (I) in crystalline Form I by a process comprising:

(v-1) providing an aqueous solution of the compound of formula (I),
(v-2) adding ethanol,
(v-3) drying the solid obtained in (v-2) at a relative humidity of from 18% to 70%, preferably of from 30% to 65%.

In a further preferred embodiment of the first aspect, the present invention relates to a process for the preparation of a compound of general formula (I), *supra,* or its stereoisomers, tautomers, N-oxides, hydrates, solvates, or salts thereof, or mixtures of same, said process comprising

(i) providing a mixture comprising a compound of formula (I), water and at least a first organic solvent,
(ii) removing water from the mixture provided in (i),
(iii) adding a second organic solvent,
(iv) adding a third organic solvent,
(v) isolating the compound of formula (I) in crystalline Form I by a process comprising:

(v-1) providing an aqueous mixture comprising the compound of formula (I),
(v-2) adding ethanol,
(v-3) drying the solid obtained in (v-2) at a relative humidity of from 18% to 70%, preferably of from 30% to 65%.

In a further embodiment of the first aspect, the present invention relates to a process for the preparation of a compound of general formula (I), *supra,* or its stereoisomers, tautomers, N-oxides, hydrates, solvates, or salts thereof, or mixtures of same, said process comprising

(i) providing a mixture comprising a compound of formula (I), water and at least a first organic solvent,
(ii) removing water from the mixture provided in (i) until the water content of the mixture lies between 10% and 20% by weight (w/w),
(iii) adding a second organic solvent, wherein said second organic solvent is an alcohol selected from the list consisting of ethanol, n-propanol and iso-propanol,
(iv) adding a third organic solvent, wherein said third organic solvent is acetone,
(v) isolating the compound of formula (I) in crystalline Form I by a process comprising:

(v-1) providing an aqueous mixture comprising the compound of formula (I),
(v-2) adding ethanol,
(v-3) drying the solid obtained in (v-2) at a relative humidity of from 18% to 70%, preferably of from 30% to 65%.

In a further embodiment of the first aspect, the present invention relates to a process for the preparation of a

compound of general formula (I), *supra,* or its stereoisomers, tautomers, N-oxides, hydrates, solvates, or salts thereof, or mixtures of same, said process comprising

(i) providing a mixture comprising a compound of formula (I), water and at least a first organic solvent,
(ii) removing water from the mixture provided in (i) until the water content of the mixture lies between 10% and 20% by weight (w/w),
(iii) adding a second organic solvent, wherein said second organic solvent is ethanol,
(iv) adding a third organic solvent, wherein said third organic solvent is acetone,
(v) isolating the compound of formula (I) in crystalline Form I by a process comprising:

(v-1) providing an aqueous mixture comprising the compound of formula (I),
(v-2) adding ethanol,
(v-3) drying the solid obtained in (v-2) at a relative humidity of from 18% to 70%, preferably of from 30% to 65%.

In a further embodiment of the first aspect, the present invention relates to a process for the preparation of a compound of general formula (I), *supra,* or its stereoisomers, tautomers, N-oxides, hydrates, solvates, or salts thereof, or mixtures of same, said process comprising

(i) providing a mixture comprising a compound of formula (I), water and at least a first organic solvent, wherein said at least first organic solvent is DMSO,
(ii) removing water from the mixture provided in (i) until the water content of the mixture lies between 10% and 20% by weight (w/w),
(iii) adding a second organic solvent, wherein said second organic solvent is ethanol,
(iv) adding a third organic solvent, wherein said third organic solvent is acetone,
(v) isolating the compound of formula (I) in crystalline Form I by a process comprising:

(v-1) providing an aqueous mixture comprising the compound of formula (I),
(v-2) adding ethanol,
(v-3) drying the solid obtained in (v-2) at a relative humidity of from 18% to 70%, preferably of from 30% to 65%.

In a further embodiment of the first aspect, the present invention relates to a process for the preparation of a compound of general formula (I), *supra,* or its stereoisomers, tautomers, N-oxides, hydrates, solvates, or salts thereof, or mixtures of same, said process comprising

(i) providing a mixture comprising a compound of formula (I), water and at least a first organic solvent, wherein said at least first organic solvent is DMSO,
(ii) removing water from the mixture provided in (i) until the water content of the mixture lies between 10% and 20% by weight (w/w),
(iii) adding a second organic solvent, wherein said second organic solvent is ethanol,
(iv) adding a third organic solvent, wherein said third organic solvent is acetone,
(v) isolating the compound of formula (I) in crystalline Form I by a process comprising:

(v-1) providing an aqueous mixture comprising the compound of formula (I),
(v-2) adding ethanol,
(v-3) drying the solid obtained in (v-2) at a relative humidity of from 18% to 70%, preferably of from 30% to 65%.

In a further embodiment of the first aspect, the present invention relates to a process for the preparation of a compound of general formula (I), *supra,* or its stereoisomers, tautomers, N-oxides, hydrates, solvates, or salts thereof, or mixtures of same, said process comprising

(i) providing a mixture comprising a compound of formula (I), water and at least a first organic solvent, wherein said at least first organic solvent is DMSO,
(ii) removing water from the mixture provided in (i) until the water content of the mixture lies between 10% and 20% by weight (w/w),
(iii) adding a second organic solvent, wherein said second organic solvent is ethanol,
(iv) adding a third organic solvent, wherein said third organic solvent is acetone,
(v) isolating the compound of formula (I) in crystalline Form I by a process comprising:

(v-1) providing an aqueous solution of the compound of formula (I),
(v-2) adding ethanol,
(v-3) drying the solid obtained in (v-2) at a relative humidity of from 18% to 70%, preferably of from 30% to 65%.

### Process for the preparation of crystalline Form II of the compound of formula (I)

The present invention provides a process for the selective preparation of the compound of formula (I) in crystalline Form II. Specifics to the process according to steps (i) to (iv), *supra,* apply to the process described herewith, as do specifics to the process according to steps (v-1) to (v-3), *supra,* for the isolation of crystalline Form II of the compound of formula (I).

Thus, in a further preferred embodiment of the first aspect, the present invention relates to a process for the preparation of a compound of general formula (I), *supra,* or its stereoisomers, tautomers, N-oxides, hydrates, solvates, or salts thereof, or mixtures of same, said process comprising

(i) providing a mixture comprising a compound of formula (I), water and at least a first organic solvent,
(ii) removing water from the mixture provided in (i),
(iii) adding a second organic solvent,
(iv) adding a third organic solvent,
(v) isolating the compound of formula (I) in crystalline Form II by a process comprising:

(v-1) providing an aqueous mixture comprising the compound of formula (I),
(v-2) adding ethanol,
(v-3) drying the solid obtained in (v-2) first at a relative humidity of from 0% to 25%, preferably of from 2% to 18%, more preferably of from 5% to 16% followed by a second drying by adjusting the relative humidity until a final value of from 18% to 70%, preferably of from 25% to 70%, more preferably of from 30% to 70% is reached.

Thus, in a further preferred embodiment of the first aspect, the present invention relates to a process for the preparation of a compound of general formula (I), *supra,* or its stereoisomers, tautomers, N-oxides, hydrates, solvates, or salts thereof, or mixtures of same, said process comprising

(i) providing a mixture comprising a compound of formula (I), water and at least a first organic solvent,
(ii) removing water from the mixture provided in (i),
(iii) adding a second organic solvent,
(iv) adding a third organic solvent,
(v) isolating the compound of formula (I) in crystalline Form II by a process comprising:

(v-1) providing an aqueous solution of the compound of formula (I),
(v-2) adding ethanol,
(v-3) drying the solid obtained in (v-2) first at a relative humidity of from 0% to 25%, preferably of from 2% to 18%, more preferably of from 5% to 16% followed by a second drying by adjusting the relative humidity until a final value of from 18% to 70%, preferably of from 25% to 70%, more preferably of from 30% to 70% is reached.

Thus, in a further preferred embodiment of the first aspect, the present invention relates to a process for the preparation of a compound of general formula (I), *supra,* or its stereoisomers, tautomers, N-oxides, hydrates, solvates, or salts thereof, or mixtures of same, said process comprising

(i) providing a mixture comprising a compound of formula (I), water and at least a first organic solvent,
(ii) removing water from the mixture provided in (i),
(iii) adding a second organic solvent,
(iv) adding a third organic solvent,
(v) isolating the compound of formula (I) in crystalline Form II by a process comprising:

(v-1) providing an aqueous mixture comprising the compound of formula (I),
(v-2) adding ethanol,
(v-3) drying the solid obtained in (v-2) first at a relative humidity of from 0% to 25%, preferably of from 2% to 18%, more preferably of from 5% to 16% followed by a second drying process by adjusting the relative humidity until a final value of from 18% to 70%, preferably of from 25% to 70%, more preferably of from 30% to 70% is reached.

In a further embodiment of the first aspect, the present invention relates to a process for the preparation of a compound of general formula (I), *supra,* or its stereoisomers, tautomers, N-oxides, hydrates, solvates, or salts thereof, or mixtures of same, said process comprising

(i) providing a mixture comprising a compound of formula (I), water and at least a first organic solvent,
(ii) removing water from the mixture provided in (i) until the water content of the mixture lies between 10% and 20% by weight (w/w),
(iii) adding a second organic solvent, wherein said second organic solvent is an alcohol selected from the list consisting of ethanol, n-propanol and iso-propanol,
(iv) adding a third organic solvent, wherein said third organic solvent is acetone
(v) isolating the compound of formula (I) in crystalline Form II by a process comprising:

(v-1) providing an aqueous mixture comprising the compound of formula (I),
(v-2) adding ethanol,
(v-3) drying the solid obtained in (v-2) first at a relative humidity of from 0% to 25%, preferably of from 2% to 18%, more preferably of from 5% to 16% followed by a second drying process by adjusting the relative humidity until a final value of from 18% to 70%, preferably of from 25% to 70%, more preferably of from 30% to 70% is reached.

In a further embodiment of the first aspect, the present invention relates to a process for the preparation of a compound of general formula (I), *supra,* or its stereoisomers, tautomers, N-oxides, hydrates, solvates, or salts thereof, or mixtures of same, said process comprising

(i) providing a mixture comprising a compound of formula (I), water and at least a first organic solvent,
(ii) removing water from the mixture provided in (i) until the water content of the mixture lies between 10% and 20% by weight (w/w),
(iii) adding a second organic solvent, wherein said second organic solvent is ethanol,
(iv) adding a third organic solvent, wherein said third organic solvent is acetone,
(v) isolating the compound of formula (I) in crystalline Form II by a process comprising:

(v-1) providing an aqueous mixture comprising the compound of formula (I),
(v-2) adding ethanol,
(v-3) drying the solid obtained in (v-2) first at a relative humidity of from 0% to 25%, preferably of from 2% to 18%, more preferably of from 5% to 16% followed by a second drying process by adjusting the relative humidity until a final value of from 18% to 70%, preferably of from 25% to 70%, more preferably of from 30% to 70% is reached.

In a further embodiment of the first aspect, the present invention relates to a process for the preparation of a compound of general formula (I), *supra,* or its stereoisomers, tautomers, N-oxides, hydrates, solvates, or salts thereof, or mixtures of same, said process comprising

(i) providing a mixture comprising a compound of formula (I), water and at least a first organic solvent, wherein said at least first organic solvent is DMSO,
(ii) removing water from the mixture provided in (i) until the water content of the mixture lies between 10% and 20% by weight (w/w),
(iii) adding a second organic solvent, wherein said second organic solvent is ethanol,
(iv) adding a third organic solvent, wherein said third organic solvent is acetone,
(v) isolating the compound of formula (I) in crystalline Form II by a process comprising:

(v-1) providing an aqueous mixture comprising the compound of formula (I),
(v-2) adding ethanol,
(v-3) drying the solid obtained in (v-2) first at a relative humidity of from 0% to 25%, preferably of from 2% to 18%, more preferably of from 5% to 16% followed by a second drying process by adjusting the relative humidity until a final value of from 18% to 70%, preferably of from 25% to 70%, more preferably of from 30% to 70% is reached.

In a further embodiment of the first aspect, the present invention relates to a process for the preparation of a compound of general formula (I), *supra,* or its stereoisomers, tautomers, N-oxides, hydrates, solvates, or salts thereof,

or mixtures of same, said process comprising

(i) providing a mixture comprising a compound of formula (I), water and at least a first organic solvent, wherein said at least first organic solvent is DMSO,
(ii) removing water from the mixture provided in (i) until the water content of the mixture lies between 10% and 20% by weight (w/w),
(iii) adding a second organic solvent, wherein said second organic solvent is ethanol,
(iv) adding a third organic solvent, wherein said third organic solvent is acetone,
(v) isolating the compound of formula (I) in crystalline Form II by a process comprising:

(v-1) providing an aqueous solution of the compound of formula (I),
(v-2) adding ethanol,
(v-3) drying the solid obtained in (v-2) first at a relative humidity of from 0% to 25%, preferably of from 2% to 18%, more preferably of from 5% to 16% followed by a second drying process by adjusting the relative humidity until a final value of from 18% to 70%, preferably of from 25% to 70%, more preferably of from 30% to 70% is reached.

In a further embodiment of the first aspect, the present invention relates to a process for the preparation of a compound of general formula (I), *supra,* or its stereoisomers, tautomers, N-oxides, hydrates, solvates, or salts thereof, or mixtures of same, said process comprising

(i) providing a mixture comprising a compound of formula (I), water and at least a first organic solvent,
(ii) removing water from the mixture provided in (i) until the water content of the mixture lies between 10% and 20% by weight (w/w),
(iii) adding a second organic solvent, wherein said second organic solvent is an alcohol selected from the list consisting of ethanol, n-propanol and iso-propanol,
(iv) adding a third organic solvent, wherein said third organic solvent is acetone
(v) isolating the compound of formula (I) in crystalline Form II by a process comprising:

(v-1) providing an aqueous mixture comprising the compound of formula (I),
(v-2) adding ethanol,
(v-3) drying the solid obtained in (v-2) first at a relative humidity of from 0% to 25%, preferably of from 2% to 18%, more preferably of from 5% to 16% for a time of from 0.5 h to 24 h, followed by a second drying process by adjusting the relative humidity until a final value of from 18% to 70%, preferably of from 25% to 70%, more preferably of from 30% to 70% is reached for a time of from 0.5 h to 48 h.

In a further embodiment of the first aspect, the present invention relates to a process for the preparation of a compound of general formula (I), *supra,* or its stereoisomers, tautomers, N-oxides, hydrates, solvates, or salts thereof, or mixtures of same, said process comprising

(i) providing a mixture comprising a compound of formula (I), water and at least a first organic solvent,
(ii) removing water from the mixture provided in (i) until the water content of the mixture lies between 10% and 20% by weight (w/w),
(iii) adding a second organic solvent, wherein said second organic solvent is ethanol,
(iv) adding a third organic solvent, wherein said third organic solvent is acetone,
(v) isolating the compound of formula (I) in crystalline Form II by a process comprising:

(v-1) providing an aqueous mixture comprising the compound of formula (I),
(v-2) adding ethanol,
(v-3) drying the solid obtained in (v-2) first at a relative humidity of from 0% to 25%, preferably of from 2% to 18%, more preferably of from 5% to 16% for a time of from 0.5 h to 24 h, followed by a second drying process by adjusting the relative humidity until a final value of from 18% to 70%, preferably of from 25% to 70%, more preferably of from 30% to 70% is reached for a time of from 0.5 h to 48 h.

In a further embodiment of the first aspect, the present invention relates to a process for the preparation of a compound of general formula (I), *supra,* or its stereoisomers, tautomers, N-oxides, hydrates, solvates, or salts thereof, or mixtures of same, said process comprising

(i) providing a mixture comprising a compound of formula (I), water and at least a first organic solvent, wherein said at least first organic solvent is DMSO,
(ii) removing water from the mixture provided in (i) until the water content of the mixture lies between 10 and 20% by weight (w/w),
(iii) adding a second organic solvent, wherein said second organic solvent is ethanol,
(iv) adding a third organic solvent, wherein said third organic solvent is acetone,
(v) isolating the compound of formula (I) in crystalline Form II by a process comprising:

(v-1) providing an aqueous mixture comprising the compound of formula (I),
(v-2) adding ethanol,
(v-3) drying the solid obtained in (v-2) first at a relative humidity of from 0% to 25%, preferably of from 2% to 18%, more preferably of from 5% to 16% for a time of from 0.5 h to 24 h, followed by a second drying process by adjusting the relative humidity until a final value of from 18% to 70%, preferably of from 25% to 70%, more preferably of from 30% to 70% is reached for a time of from 0.5 h to 48 h.

**Crystallization of Form I of the compound of Formula (I)**

The present invention also provides a process for the preparation of the crystalline Form I of the compound of formula I, i.e. characterized by having a powder X-ray diffractogram comprising reflections at 2-Theta angles of (6.8 ± 0.2)°, (9.1 ± 0.2)°, (10.1 ± 0.2)°, (11.4 ± 0.2)° and (12.0 ± 0.2)°, when measured at room temperature with Cu-Kalpha1 radiation having a wavelength of 0.15419 nm.
Thus, in one embodiment, the present invention provides for a process for the preparation of the crystalline Form I of a compound of formula (I)

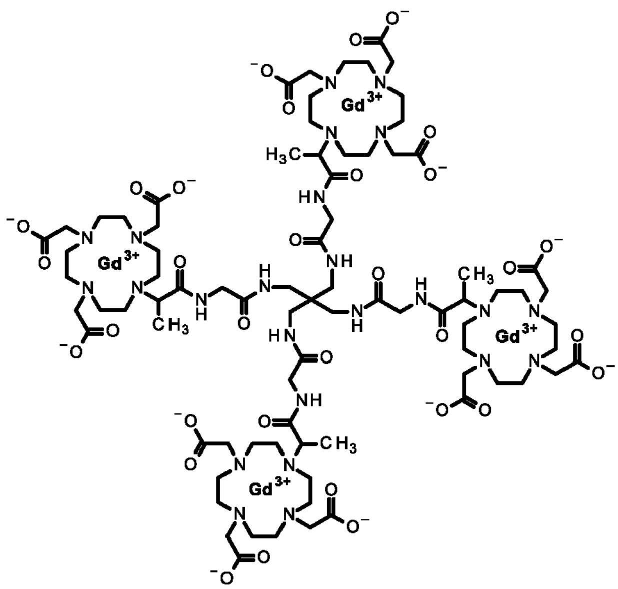

**(I)**

said process comprising:

(i) providing an aqueous mixture comprising the compound of formula (I),
(ii) adding an organic solvent,
(iii) drying the solid obtained in (ii) at a relative humidity of from 18% to 70%, preferably of from 30% to 65%.

In a further embodiment, the present invention provides for a process for the preparation of the crystalline Form I of a compound of formula (I), *supra,* said process comprising:

(i) providing an aqueous mixture comprising the compound of formula (I),
(ii) adding an organic solvent selected from the group consisting of ethanol, n-propanol and iso-propanol or a mixture thereof,
(iii) drying the solid obtained in (ii) at a relative humidity of from 18% to 70%, preferably of from 30% to 65%.

In a further embodiment, the present invention provides for a process for the preparation of the crystalline Form I of a compound of formula (I), *supra,* said process comprising:

(i) providing an aqueous mixture comprising the compound of formula (I),
(ii) adding ethanol,
(iii) drying the solid obtained in (ii) at a relative humidity of from 18% to 70%, preferably of from 30% to 65%.

In step (i), an aqueous mixture comprising the compound of formula (I) is provided. Preferably, the only solvent in the aqueous mixture comprising the compound of formula (I) is water. Preferably, the compound of formula (I) is completely dissolved in the aqueous mixture. Thus preferably, in step (i) the aqueous mixture comprising the compound of formula (I) is an aqueous solution of the compound of formula (I) in water. In order to achieve complete dissolution of the compound of formula (I) and/or to obtain an aqueous mixture or aqueous solution suitable to isolate the compound of formula (I) in crystalline Form I or in crystalline Form II, processes such as filtration, diafiltration, ultrafiltration, nanofiltration, treatment with an ion exchange resin and the like may be necessary. Preferably, the content of the compound of formula (I) in the aqueous mixture provided in step (i) is in the range of from 30% to 70% by weight (w/w), more preferably of from 40% to 60% by weight (w/w), even more preferably of from 45% to 55% by weight (w/w).

In step (ii), an organic solvent is added to the mixture provided in step (i). Preferably, the organic solvent is selected from the group consisting of ethanol, n-propanol and iso-propanol or a mixture thereof. Preferably, the solvent is ethanol or iso-propanol or a mixture thereof. More preferably, the solvent is ethanol. Preferably, the organic solvent is added until the water content of the mixture is in the range of from 5% to 25% by weight (w/w), more preferably of from 7% to 20% by weight (w/w).

In step (ii), the organic solvent is preferably added at a temperature of from 45 °C to 75 °C, more preferably of from 50 °C to 70 °C, more preferably of from 55 °C to 65 °C. Especially preferred is the addition of ethanol, which is carried out at a temperature as described above. After the addition of the organic solvent, the water content of the mixture is preferably in the range of from 5% to 25% by weight (w/w), more preferably of from 7% to 20% by weight (w/w).

Upon addition of the organic solvent in step (ii), a solid is obtained, which is dried in step (iii) at a relative humidity of from 18% to 70%, preferably of from 30% to 65%, upon which the compound of formula (I) is obtained in crystalline Form I. Thus, step (iii) comprises drying the resulting solid from step (ii) at a relative humidity of from 18% to 70%, preferably of from 30% to 65% to yield the compound of formula (I) in crystalline Form I. Additionally or alternatively, step (iii) comprises drying the resulting solid from step (ii) at a relative humidity of from 18% to 70%, preferably of from 30% to 65% to yield the compound of formula (I) in crystalline Form I comprising reflections at 2-Theta angles of (6.8 ± 0.2)°, (9.1 ± 0.2)°, (10.1 ± 0.2)°, (11.4 ± 0.2)° and (12.0 ± 0.2)°, when measured at room temperature with Cu-Kalpha1 radiation having a wavelength of 0.15419 nm. Preferably, the drying process is carried out at the relative humidity described above for a time of from 1 h to 48 h.

In a preferred embodiment, the present invention provides for a process for the preparation of the crystalline Form I of a compound of formula (I), *supra,* said process comprising:

(i) providing an aqueous mixture comprising the compound of formula (I),
(ii) adding ethanol,
(iii) drying the solid obtained in (ii) at a relative humidity of from 18% to 70%, preferably of from 30% to 65%.

In a preferred embodiment, the present invention provides for a process for the preparation of the crystalline Form I of a compound of formula (I), *supra,* said process comprising:

(i) providing an aqueous solution of the compound of formula (I),
(ii) adding ethanol,
(iii) drying the solid obtained in (ii) at a relative humidity of from 18% to 70%, preferably of from 30% to 65%.

In a further preferred embodiment, the present invention provides for a process for the preparation of the crystalline Form I of a compound of formula (I), *supra,* said process comprising:

(i) providing an aqueous mixture comprising the compound of formula (I) in the range of from 30% to 70% by weight (w/w), more preferably of from 40% to 60% by weight (w/w), even more preferably of from 45% to 55% by weight (w/w),
(ii) adding ethanol,
(iii) drying the solid obtained in (ii) at a relative humidity of from 18% to 70%, preferably of from 30% to 65%.

In a further preferred embodiment, the present invention provides for a process for the preparation of the crystalline Form I of a compound of formula (I), *supra,* said process comprising:

(i) providing an aqueous mixture comprising the compound of formula (I) in the range of from 30% to 70% by weight (w/w), more preferably of from 40% to 60% by weight (w/w), even more preferably of from 45% to 55% by weight (w/w),
(ii) adding ethanol until the water content of the mixture is in the range of from 5% to 25% by weight (w/w), more preferably of from 7% to 20% by weight (w/w),
(iii) drying the solid obtained in (ii) at a relative humidity of from 18% to 70%, preferably of from 30% to 65%.

In a further preferred embodiment, the present invention provides for a process for the preparation of the crystalline Form I of a compound of formula (I), *supra,* said process comprising:

(i) providing an aqueous solution of the compound of formula (I) in the range of from 30% to 70% by weight (w/w), more preferably of from 40% to 60% by weight (w/w), even more preferably of from 45% to 55% by weight (w/w),
(ii) adding ethanol until the water content of the mixture is in the range of from 5% to 25% by weight (w/w), more preferably of from 7% to 20% by weight (w/w),
(iii) drying the solid obtained in (ii) at a relative humidity of from 18% to 70%, preferably of from 30% to 65%.

In a further preferred embodiment, the present invention provides for a process for the preparation of the crystalline Form I of a compound of formula (I), *supra,* said process comprising:

(i) providing an aqueous mixture comprising the compound of formula (I) in the range of from 30% to 70% by weight (w/w), more preferably of from 40% to 60% by weight (w/w), even more preferably of from 45% to 55% by weight (w/w),
(ii) adding ethanol until the water content of the mixture is in the range of from 5% to 25% by weight (w/w), more preferably of from 7% to 20% by weight (w/w),
(iii) drying the solid obtained in (ii) at a relative humidity of from 18% to 70%, preferably of from 30% to 65% for a time of from 1 to 48 h.

**Crystallization of Form II of the compound of Formula (I)**

The present invention also provides a process for the preparation of the crystalline Form II of the compound of formula (I), i.e. characterized by having a powder X-ray diffractogram comprising reflections at 2-Theta angles of (7.3 $\pm$ 0.2)°, (10.2 $\pm$ 0.2)°, (10.8 $\pm$ 0.2)°, (11.3 $\pm$ 0.2)° and (13.3 $\pm$ 0.2)°, when measured at room temperature with Cu-Kalpha1 radiation having a wavelength of 0.15419 nm.
Thus, in one embodiment, the present invention provides for a process for the preparation of the crystalline Form II of a compound of formula (I)

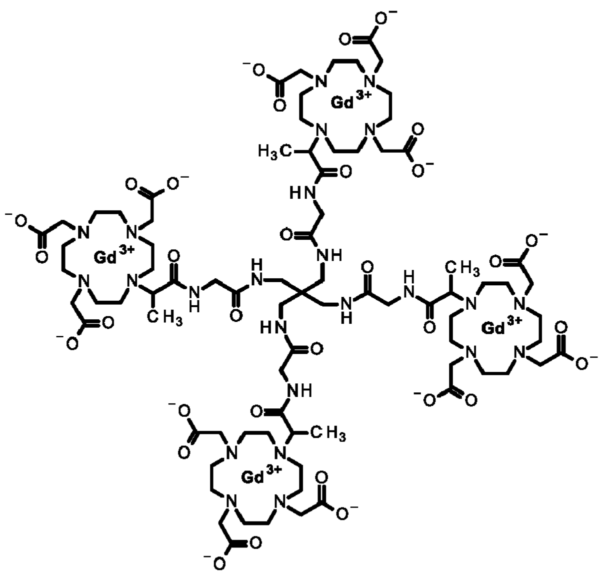

**(I)**

said process comprising:

(i) providing an aqueous mixture comprising the compound of formula (I),

(ii) adding an organic solvent,
(iii) drying the solid obtained in (ii) first at a relative humidity of from 0% to 25%, preferably of from 2% to 18%, more preferably of from 5% to 16% followed by a second drying process by adjusting the relative humidity until a final value of from 18% to 70%, preferably of from 25% to 70%, more preferably of from 30% to 70% is reached.

In a further embodiment, the present invention provides for a process for the preparation of the crystalline Form II of a compound of formula (I), *supra,* said process comprising:

(i) providing an aqueous mixture comprising the compound of formula (I),
(ii) adding an organic solvent selected from the group consisting of ethanol, n-propanol and iso-propanol or a mixture thereof,
(iii) drying the solid obtained in (ii) first at a relative humidity of from 0% to 25%, preferably of from 2% to 18%, more preferably of from 5% to 16% followed by a second drying process by adjusting the relative humidity until a final value of from 18% to 70%, preferably of from 25% to 70%, more preferably of from 30% to 70% is reached.

In a further embodiment, the present invention provides for a process for the preparation of the crystalline Form II of a compound of formula (I), *supra,* said process comprising:

(i) providing an aqueous mixture comprising the compound of formula (I),
(ii) adding ethanol,
(iii) drying the solid obtained in (ii) first at a relative humidity of from 0% to 25%, preferably of from 2% to 18%, more preferably of from 5% to 16% followed by a second drying process by adjusting the relative humidity until a final value of from 18% to 70%, preferably of from 25% to 70%, more preferably of from 30% to 70% is reached.

In a further embodiment, the present invention provides for a process for the preparation of the crystalline Form II of a compound of formula (I), *supra,* said process comprising:

(i) providing an aqueous solution of the compound of formula (I),
(ii) adding ethanol,
(iii) drying the solid obtained in (ii) first at a relative humidity of from 0% to 25%, preferably of from 2% to 18%, more preferably of from 5% to 16% followed by a second drying process by adjusting the relative humidity until a final value of from 18% to 70%, preferably of from 25% to 70%, more preferably of from 30% to 70% is reached.

In step (i), an aqueous mixture comprising the compound of formula (I) is provided. Preferably, the only solvent in the aqueous mixture comprising the compound of formula (I) is water. Preferably, the compound of formula (I) is completely dissolved in the aqueous mixture. Thus preferably, in step (i) the aqueous mixture comprising the compound of formula (I) is an aqueous solution of the compound of formula (I) in water. In order to achieve complete dissolution of the compound of formula (I) and/or to obtain an aqueous mixture or aqueous solution suitable to isolate the compound of formula (I) in crystalline Form I or in crystalline Form II, processes such as filtration, diafiltration, ultrafiltration, nanofiltration, treatment with an ion exchange resin and the like may be necessary. Preferably, the content of the compound of formula (I) in the aqueous mixture provided in step (i) is in the range of from 30% to 70% by weight (w/w), more preferably of from 40% to 60% by weight (w/w), even more preferably of from 45% to 55% by weight (w/w).

In step (ii), an organic solvent is added to the mixture provided in step (i). Preferably, the organic solvent is selected from the group consisting of ethanol, n-propanol and iso-propanol or a mixture thereof. Preferably, the solvent is ethanol or iso-propanol or a mixture thereof. More preferably, the solvent is ethanol. Preferably, the organic solvent is added until the water content of the mixture is in the range of from 5% to 25% by weight (w/w), more preferably of from 7% to 20% by weight (w/w).

In step (ii), the organic solvent is preferably added at a temperature of from 45 °C to 75 °C, more preferably of from 50 °C to 70°C, more preferably of from 55 °C to 65°C. Especially preferred is the addition of ethanol, which is carried out at a temperature as described above. After the addition of the organic solvent, the water content of the mixture is preferably in the range of from 5% to 25% by weight (w/w), more preferably of from 7% to 20% by weight (w/w).

Upon addition of the organic solvent in step (ii), a solid is obtained, which is dried in step (iii) first at a relative humidity of from 0% to 25%, preferably of from 2% to 18%, more preferably of from 5% to 16% and then further dried by adjusting the relative humidity until a final value of from 18% to 70%, preferably of from 25% to 70%, more preferably of from 30% to 70% is reached, upon which the compound of formula (I) is obtained in crystalline Form II.

Thus, step (iii) comprises first drying the resulting solid from step (ii) at a relative humidity of from 0% to 25%, preferably of from 2% to 18%, more preferably of from 5% to 16% followed by a second drying process by adjusting the relative humidity until a final value of from 18% to 70%, preferably of from 25% to 70%, more preferably of from 30% to

70% is reached to yield the compound of formula (I) in crystalline Form II.

Additionally or alternatively, step (iii) comprises first drying the resulting solid from step (ii) at a relative humidity of from 0% to 25%, preferably of from 2% to 18%, more preferably of from 5% to 16% followed by a second drying process by adjusting the relative humidity until a final value of from 18% to 70%, preferably of from 25% to 70%, more preferably of from 30% to 70% is reached to yield the compound of formula (I) in crystalline Form II comprising reflections at 2-Theta angles of (7.3 ± 0.2)°, (10.2 ± 0.2)°, (10.8 ± 0.2)°, (11.3 ± 0.2)° and (13.3 ± 0.2)°, when measured at room temperature with Cu-Kalpha1 radiation having a wavelength of 0.15419 nm.

Preferably, the first drying process is carried out at the relative humidity described above for a time of from 0.5 h to 24 h, and the second drying process is carried out at the relative humidity described above for a time of from 0.5 h to 48 h.

In a preferred embodiment, the present invention provides for a process for the preparation of the crystalline Form II of a compound of formula (I), *supra,* said process comprising:

(i) providing an aqueous mixture comprising the compound of formula (I),
(ii) adding ethanol,
(iii) drying the solid obtained in (ii) first at a relative humidity of from 0% to 25%, preferably of from 2% to 18%, more preferably of from 5% to 16% followed by a second drying process by adjusting the relative humidity until a final value of from 18% to 70%, preferably of from 25% to 70%, more preferably of from 30% to 70% is reached.

In a further preferred embodiment, the present invention provides for a process for the preparation of the crystalline Form II of a compound of formula (I), *supra,* said process comprising:

(i) providing an aqueous mixture comprising the compound of formula (I) in the range of from 30% to 70% by weight (w/w), more preferably of from 40% to 60% by weight (w/w), even more preferably of from 45% to 55% by weight (w/w),
(ii) adding ethanol,
(iii) drying the solid obtained in (ii) first at a relative humidity of from 0% to 25%, preferably of from 2% to 18%, more preferably of from 5% to 16% followed by a second drying process by adjusting the relative humidity until a final value of from 18% to 70%, preferably of from 25% to 70%, more preferably of from 30% to 70% is reached.

In a further preferred embodiment, the present invention provides for a process for the preparation of the crystalline Form II of a compound of formula (I), *supra,* said process comprising:

(i) providing an aqueous mixture comprising the compound of formula (I) in the range of from 30% to 70% by weight (w/w), more preferably of from 40% to 60% by weight (w/w), even more preferably of from 45% to 55% by weight (w/w),
(ii) adding ethanol until the water content of the mixture is in the range of from 5% to 25% by weight (w/w), more preferably of from 7% to 20% by weight (w/w),
(iii) drying the solid obtained in (ii) first at a relative humidity of from 0% to 25%, preferably of from 2% to 18%, more preferably of from 5% to 16% followed by a second drying process by adjusting the relative humidity until a final value of from 18% to 70%, preferably of from 25% to 70%, more preferably of from 30% to 70% is reached.

In a further preferred embodiment, the present invention provides for a process for the preparation of the crystalline Form II of a compound of formula (I), *supra,* said process comprising:

(i) providing an aqueous solution of the compound of formula (I) in the range of from 30% to 70% by weight (w/w), more preferably of from 40% to 60% by weight (w/w), even more preferably of from 45% to 55% by weight (w/w),
(ii) adding ethanol until the water content of the mixture is in the range of from 5% to 25% by weight (w/w), more preferably of from 7% to 20% by weight (w/w),
(iii) drying the solid obtained in (ii) first at a relative humidity of from 0% to 25%, preferably of from 2% to 18%, more preferably of from 5% to 16% followed by a second drying process by adjusting the relative humidity until a final value of from 18% to 70%, preferably of from 25% to 70%, more preferably of from 30% to 70% is reached.

In a further preferred embodiment, the present invention provides for a process for the preparation of the crystalline Form II of a compound of formula (I), *supra,* said process comprising:

(i) providing an aqueous mixture comprising the compound of formula (I) in the range of from 30% to 70% by weight (w/w), more preferably of from 40% to 60% by weight (w/w), most preferably of from 45% to 55% by weight (w/w),
(ii) adding ethanol until the water content of the mixture is in the range of from 5% to 25% by weight (w/w), more preferably of from 7% to 20% by weight (w/w),

(iii) drying the solid obtained in (ii) first at a relative humidity of from 0% to 25%, preferably of from 2% to 18%, more preferably of from 5% to 16% for a time of from 0.5 h to 24 h, followed by a second drying process by adjusting the relative humidity until a final value of from 18% to 70%, preferably of from 25% to 70%, more preferably of from 30% to 70% is reached for a time of from 0.5 h to 48 h.

**Preparation of a compound of formula (I) via a compound of formula (II)**

In a further embodiment, the present invention relates to a process for the preparation of a compound of formula (I) comprising

(i-1) providing a compound of formula (II) or a stereoisomer, tautomer, N-oxide, hydrate, solvate, or salt thereof, or mixtures of same,

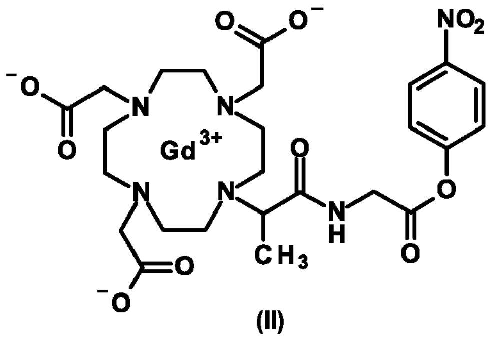

(II)

(i-2) and reacting the compound of formula (II) with a compound of formula (III) or a salt thereof

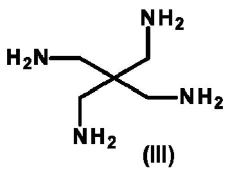

(III)

.

As described in the first aspect, step (i) of the process for the preparation of a compound of general formula (I), *supra,* or its stereoisomers, tautomers, N-oxides, hydrates, solvates, or salts thereof, or mixtures of same comprises the provision of a mixture comprising a compound of formula (I), water and at least a first organic solvent. In accordance with a further aspect, said provision in step (i) comprises

(i-1) providing a compound of formula (II) or a stereoisomer, tautomer, N-oxide, hydrate, solvate, or salt thereof, or mixtures of same,

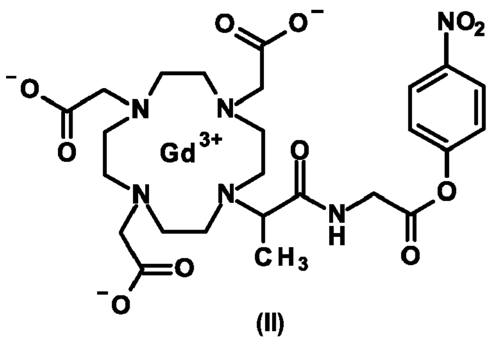

(II)

(i-2) and reacting the compound of formula (II) with a compound of formula (III) or a salt thereof

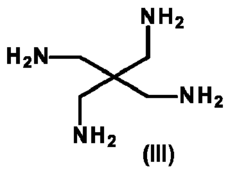

(III)

.

Thus, a further embodiment of the present invention relates to a process for the preparation of a compound of general formula (I), *supra,* or its stereoisomers, tautomers, N-oxides, hydrates, solvates, or salts thereof, or mixtures of same, said process comprising

(i) providing a mixture comprising a compound of formula (I), water and at least a first organic solvent by a process comprising

(i-1) providing a compound of formula (II) or a stereoisomer, tautomer, N-oxide, hydrate, solvate, or salt thereof, or mixtures of same,

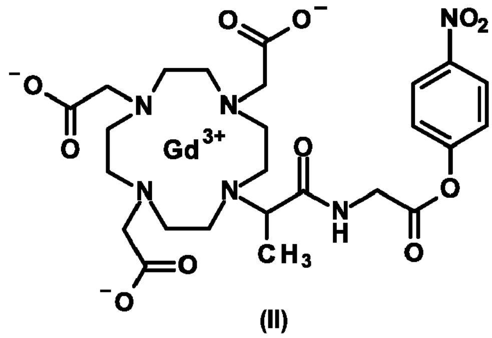

(II)

(i-2) and reacting the compound of formula (II) with a compound of formula (III) or a salt thereof

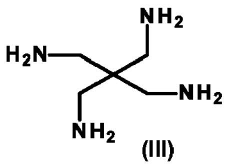

(III)

,

(ii) removing water from the mixture provided in (i),
(iii) adding a second organic solvent,
(iv) adding a third organic solvent,
(v) isolating the compound of formula (I).

In step (i-1), a compound of formula (II) or a stereoisomer, tautomer, N-oxide, hydrate, solvate, or salt thereof, or mixtures of same is provided. Preferably, the compound of formula (II) is provided as a solid in step (i-1). More preferably, in step (i-1), the compound of formula (II) is provided as a crystalline solid, i.e. in crystalline form. More preferably, the compound of formula (II) is provided as a crystalline solid in crystalline Form I. More preferably, the compound of formula (II) is provided as a crystalline solid in crystalline Form I as described herein below. More preferably, the compound of formula (II) is provided as a crystalline solid in crystalline Form I characterized by having a powder X-ray diffractogram comprising reflections at 2-Theta angles of ($8.0 \pm 0.2$)°, ($9.0 \pm 0.2$)°, and ($12.8 \pm 0.2$)°, when measured at room temperature with Cu-Kalpha1 radiation having a wavelength of 0.15419 nm.

Thus, a further embodiment of the present invention relates to a process for the preparation of a compound of general formula (I), *supra,* or its stereoisomers, tautomers, N-oxides, hydrates, solvates, or salts thereof, or mixtures of same, said process comprising

(i) providing a mixture comprising a compound of formula (I), water and at least a first organic solvent by a process comprising

(i-1) providing a compound of formula (II) or a stereoisomer, tautomer, N-oxide, hydrate, solvate, or salt thereof, or mixtures of same as a solid, preferably as a crystalline solid,

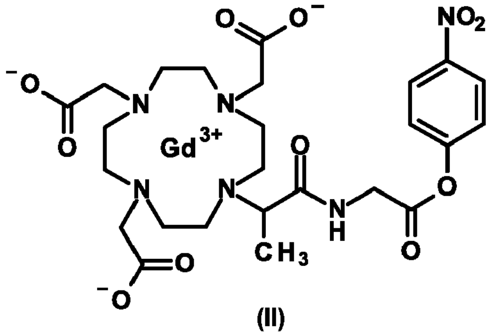

(II)

(i-2) and reacting the compound of formula (II) with a compound of formula (III) or a salt thereof

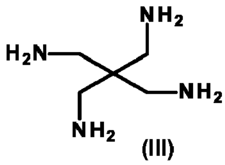

(III) ,

(ii) removing water from the mixture provided in (i),
(iii) adding a second organic solvent,
(iv) adding a third organic solvent,
(v) isolating the compound of formula (I).

Thus, a further embodiment of the present invention relates to a process for the preparation of a compound of general formula (I), *supra,* or its stereoisomers, tautomers, N-oxides, hydrates, solvates, or salts thereof, or mixtures of same, said process comprising

(i) providing a mixture comprising a compound of formula (I), water and at least a first organic solvent by a process comprising

(i-1) providing a compound of formula (II) or a stereoisomer, tautomer, N-oxide, hydrate, solvate, or salt thereof, or mixtures of same as a solid, preferably as a crystalline solid in crystalline Form I,

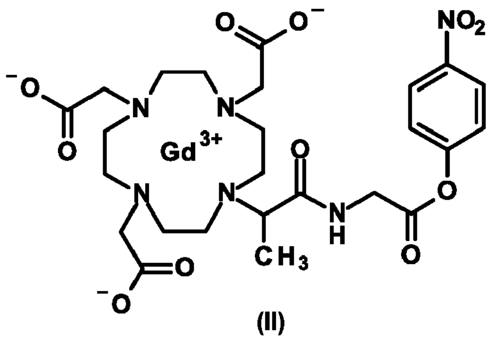

(II)

(i-2) and reacting the compound of formula (II) with a compound of formula (III) or a salt thereof

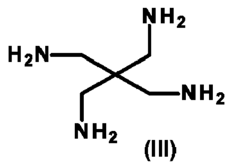

(III)

,

(ii) removing water from the mixture provided in (i),
(iii) adding a second organic solvent,
(iv) adding a third organic solvent,
(v) isolating the compound of formula (I).

In the context of the present invention, the provision of a mixture comprising a compound of formula (I), *supra,* water and at least a first organic solvent in step (i) by providing a compound of formula (II) and reacting said compound of formula (II) with a compound of formula (III) can be applied to any of the embodiments of the present invention described above or below which include said step (i).

In the context of the present invention, step (i) according to the process of the present invention comprises providing a mixture comprising a compound of formula (I), water and at least a first organic solvent by providing a compound of formula (II) and reacting said compound of formula (II) with a compound of formula (III).

The reaction of the compound of formula (II) with the compound of formula (III) is preferably carried out in water or in an organic solvent. If the reaction is carried out in an organic solvent, said organic solvent is selected from dimethyl sulfoxide, methylimidazole, dimethylacetamide, sulfolane and N-methylpyrrolidone. More preferably, the organic solvent for the reaction between the compound of formula (II) and the compound of formula (III) is the same as the at least first organic solvent used in step (i) of the process of the present invention. Most preferably, the organic solvent for the reaction between the compound of formula (II) and the compound of formula (III) is dimethyl sulfoxide (DMSO).

The reaction of the compound of formula (II) with the compound of formula (III) is preferably carried out in the presence of a base. Such base is preferably an organic base, more preferably a tertiary amine base. More preferably, the base is a tertiary amine base selected from triethylamine, tributylamine and N,N-diisopropylethylamine. Most preferably, the base is N,N-diisopropylethylamine. Such base is preferably used in an excess amount. Preferably, the excess of base lies in the range of from 4.2 to 8.0 equivalents with respect to the amount of the compound of formula (III).

The reaction of the compound of formula (II) with the compound of formula (III) is preferably carried out at a temperature of from 20 °C to 60 °C. In order to ensure a smooth conversion, the temperature lies preferably in the range of from 40 °C to 60 °C, more preferably of from 45 °C to 55°C.

The reaction of the compound of formula (II) with the compound of formula (III) is preferably carried out using an excess of chemical equivalents of the compound of formula (II). Preferably, the excess of compound of formula (II) lies in the range of from 4.0 to 6.0 equivalents, more preferably of from 4.0 to 4.6 equivalents.

After the reaction of the compound of formula (II) with the compound of formula (III), water is added to the reaction mixture prior to the isolation of the compound of formula (I). Preferably, water is added to the mixture obtained in step (i-2) from the reaction of the compound of formula (II) with the compound of formula (III) and said mixture is then treated with a base, preferably an inorganic base such as for example sodium hydroxide, neutralized with a mineral acid such as for example hydrochloric acid, and optionally extracted with an organic solvent. A preferred organic solvent for the optional extraction is methyl-tert-butyl ether (MTBE). Thus preferably, prior to step (ii), water is added to the mixture obtained in step (i-2) from the reaction of the compound of formula (II) with the compound of formula (III) and said mixture is then treated with a base, preferably an inorganic base such as for example sodium hydroxide, neutralized with a mineral acid such as for example hydrochloric acid, and optionally extracted with an organic solvent. If an extraction step is included, it is the aqueous phase that is subjected to step (ii) in the isolation of the compound of formula (I).

With regard to steps (ii), (iii), (iv) and (v), it is understood that these can be carried out according to the process of the present invention for the preparation of the compound of formula (I) - including steps (v-1), (v-2) and (v-3) - as described throughout this document.

**Crystalline Forms I, II and III of the compound of formula (II)**

In step (i-1), *supra,* a compound of formula (II) or a stereoisomer, tautomer, N-oxide, hydrate, solvate, or salt thereof, or mixtures of same is provided. Preferably, the compound of formula (II) is provided as a solid in step (i-1). More preferably, in step (i-1), the compound of formula (II) is provided as a crystalline solid, i.e. in crystalline form.

The crystalline Form I of the compound of formula (II) is characterized by having a powder X-ray diffractogram comprising reflections at 2-Theta angles of (8.0 ± 0.2)°, (9.0 ± 0.2)°, and (12.8 ± 0.2)°, when measured at room temperature with Cu-Kalpha1 radiation having a wavelength of 0.15419 nm. Preferably, the crystalline Form I of the

compound of formula (II) is characterized by having a powder X-ray diffractogram comprising reflections at 2-Theta angles of (8.0 ± 0.2)°, (9.0 ± 0.2)°, (11.7 ± 0.2)°, (12.8 ± 0.2)° and (14.9 ± 0.2)°, when measured at room temperature with Cu-Kalpha1 radiation having a wavelength of 0.15419 nm. Preferably, the crystalline Form I of the compound of formula (II) is characterized by having a powder X-ray diffractogram comprising reflections at 2-Theta angles of (8.0 ± 0.2)°, (9.0 ± 0.2)°, (10.4 ± 0.2)°, (11.7 ± 0.2)°, (12.8 ± 0.2)°, (14.9 ± 0.2)° and (17.4 ± 0.2)°, when measured at room temperature with Cu-Kalpha1 radiation having a wavelength of 0.15419 nm. Preferably, the crystalline Form I of the compound of formula (II) is characterized by having a powder X-ray diffractogram comprising reflections at 2-Theta angles of (8.0 ± 0.2)°, (9.0 ± 0.2)°, (10.4 ± 0.2)°, (11.7 ± 0.2)°, (12.8 ± 0.2)°, (14.9 ± 0.2)°, (17.4 ± 0.2)°, (22.7 ± 0.2)°, (23.0 ± 0.2)° and (28.3 ± 0.2)°, when measured at room temperature with Cu-Kalpha1 radiation having a wavelength of 0.15419 nm.

Alternatively, the crystalline Form I of the compound of formula (II) is characterized by having a powder X-ray diffractogram essentially the same as displayed in Figure 18 of the present invention, when measured at room temperature with Cu-Kalpha1 radiation having a wavelength of 0.15419 nm.

Alternatively or additionally, the crystalline Form I of the compound of formula (II) is characterized by having a Fourier transform Infrared spectrum comprising bands at wavenumbers of (1085 ± 2) $cm^{-1}$, (1343 ± 2) $cm^{-1}$ and (1598 ± 2) $cm^{-1}$, when measured at room temperature with a diamond ATR cell. Preferably, the crystalline Form I of the compound of formula (II) is characterized by having a Fourier transform Infrared spectrum comprising bands at wavenumbers of (715 ± 2) $cm^{-1}$, (1085 ± 2) $cm^{-1}$, (1343 ± 2) $cm^{-1}$, (1522 ± 2) $cm^{-1}$ and (1598 ± 2) $cm^{-1}$, when measured at room temperature with a diamond ATR cell. Preferably, the crystalline Form I of the compound of formula (II) is characterized by having a Fourier transform Infrared spectrum comprising bands at wavenumbers of (715 ± 2) $cm^{-1}$, (1085 ± 2) $cm^{-1}$, (1343 ± 2) $cm^{-1}$, (1390 ± 2) $cm^{-1}$, (1522 ± 2) $cm^{-1}$, (1598 ± 2) $cm^{-1}$ and (1688 ± 2) $cm^{-1}$, when measured at room temperature with a diamond ATR cell. Preferably, the crystalline Form I of the compound of formula (II) is characterized by having a Fourier transform Infrared spectrum comprising bands at wavenumbers of (715 ± 2) $cm^{-1}$, (1085 ± 2) $cm^{-1}$, (1154 ± 2) $cm^{-1}$, (1343 ± 2) $cm^{-1}$, (1390 ± 2) $cm^{-1}$, (1522 ± 2) $cm^{-1}$, (1557 ± 2) $cm^{-1}$, (1598 ± 2) $cm^{-1}$, (1688 ± 2) $cm^{-1}$ and (1779 ± 2) $cm^{-1}$, when measured at room temperature with a diamond ATR cell.

Alternatively, the crystalline Form I of the compound of formula (II) is characterized by having a Fourier transform Infrared spectrum essentially the same as displayed in Figure 19 of the present invention, when measured at room temperature with a diamond ATR cell.

Alternatively or additionally, the crystalline Form I of the compound of formula (II) is characterized by having a Raman spectrum comprising bands at wavenumbers of (1111 ± 2) $cm^{-1}$, (1346 ± 2) $cm^{-1}$ and (1594 ± 2) $cm^{-1}$, when measured at room temperature with a laser at a wavelength of 1064nm. Preferably, the crystalline Form I of the compound of formula (II) is characterized by having a Raman spectrum comprising bands at wavenumbers of (1111 ± 2) $cm^{-1}$, (1213 ± 2) $cm^{-1}$, (1346 ± 2) $cm^{-1}$, (1594 ± 2) $cm^{-1}$ and (2884 ± 2) $cm^{-1}$, when measured at room temperature with a laser at a wavelength of 1064nm. Preferably, the crystalline Form I of the compound of formula (II) is characterized by having a Raman spectrum comprising bands at wavenumbers of (374 ± 2) $cm^{-1}$, (865 ± 2) $cm^{-1}$, (1111 ± 2) $cm^{-1}$, (1213 ± 2) $cm^{-1}$, (1346 ± 2) $cm^{-1}$, (1594 ± 2) $cm^{-1}$ and (2884 ± 2) $cm^{-1}$, when measured at room temperature with a laser at a wavelength of 1064nm. Preferably, the crystalline Form I of the compound of formula (II) is characterized by having a Raman spectrum comprising bands at wavenumbers of (374 ± 2) $cm^{-1}$, (865 ± 2) $cm^{-1}$, (1111 ± 2) $cm^{-1}$, (1213 ± 2) $cm^{-1}$, (1346 ± 2) $cm^{-1}$, (1396 ± 2) $cm^{-1}$, (1463 ± 2) $cm^{-1}$, (1594 ± 2) $cm^{-1}$, (2884 ± 2) $cm^{-1}$ and (2987 ± 2) $cm^{-1}$, when measured at room temperature with a laser at a wavelength of 1064nm. Alternatively, the crystalline Form I of the compound of formula (II) is characterized by having a Raman spectrum essentially the same as displayed in Figure 20 of the present invention, when measured at room temperature with a laser at a wavelength of 1064nm.

Alternatively or additionally, the crystalline Form I of the compound of formula (II) is characterized by having a differential scanning calorimetric curve as displayed in Figure 21 of the present invention.

Alternatively or additionally, the crystalline Form I of the compound of formula (II) is characterized by having a TGA curve as displayed in Figure 22 of the present invention.

The crystalline Form II of the compound of formula (II) is characterized by having a powder X-ray diffractogram comprising reflections at 2-Theta angles of (6.6 ± 0.2)°, (10.3 ± 0.2)° and (11.1 ± 0.2)°, when measured at room temperature with Cu-Kalpha1 radiation having a wavelength of 0.15419 nm. Preferably, the crystalline Form II of the compound of formula (II) is characterized by having a powder X-ray diffractogram comprising reflections at 2-Theta angles of (6.6 ± 0.2)°, (10.3 ± 0.2)°, (11.1 ± 0.2)°, (11.5 ± 0.2)° and (11.7 ± 0.2)°, when measured at room temperature with Cu-Kalpha1 radiation having a wavelength of 0.15419 nm. Preferably, the crystalline Form II of the compound of formula (II) is characterized by having a powder X-ray diffractogram comprising reflections at 2-Theta angles of (6.0 ± 0.2)°, (6.6 ± 0.2)°, (10.3 ± 0.2)°, (11.1 ± 0.2)°, (11.5 ± 0.2)°, (11.7 ± 0.2)° and (12.2 ± 0.2)°, when measured at room temperature with Cu-Kalpha1 radiation having a wavelength of 0.15419 nm. Preferably, the crystalline Form II of the compound of formula (II) is characterized by having a powder X-ray diffractogram comprising reflections at 2-Theta angles of (6.0 ± 0.2)°, (6.6 ± 0.2)°, (8.3 ± 0.2)°, (10.3 ± 0.2)°, (11.1 ± 0.2)°, (11.5 ± 0.2)°, (11.7 ± 0.2)°, (12.2 ± 0.2)°, (12.6 ± 0.2)° and (13.0 ± 0.2)°, when measured at room temperature with Cu-Kalpha1 radiation having a wavelength

of 0.15419 nm.

Alternatively, the crystalline Form II of the compound of formula (II) is characterized by having a powder X-ray diffractogram essentially the same as displayed in Figure 23 of the present invention, when measured at room temperature with Cu-Kalpha1 radiation having a wavelength of 0.15419 nm.

Alternatively or additionally, the crystalline Form II of the compound of formula (II) is characterized by having a Fourier transform Infrared spectrum comprising bands at wavenumbers of (1085 ± 2) $cm^{-1}$, (1346 ± 2) $cm^{-1}$ and (1612 ± 2) $cm^{-1}$, when measured at room temperature with a diamond ATR cell. Preferably, the crystalline Form II of the compound of formula (II) is characterized by having a Fourier transform Infrared spectrum comprising bands at wavenumbers of (713 ± 2) $cm^{-1}$, (1085 ± 2) $cm^{-1}$, (1321 ± 2) $cm^{-1}$, (1346 ± 2) $cm^{-1}$ and (1612 ± 2) $cm^{-1}$, when measured at room temperature with a diamond ATR cell. Preferably, the crystalline Form II of the compound of formula (II) is characterized by having a Fourier transform Infrared spectrum comprising peaks at wavenumbers of (713 ± 2) $cm^{-1}$, (1085 ± 2) $cm^{-1}$, (1153 ± 2) $cm^{-1}$, (1321 ± 2) $cm^{-1}$, (1346 ± 2) $cm^{-1}$, (1612 ± 2) $cm^{-1}$ and (1774 ± 2) $cm^{-1}$, when measured at room temperature with a diamond ATR cell. Preferably, the crystalline Form II of the compound of formula (II) is characterized by having a Fourier transform Infrared spectrum comprising bands at wavenumbers of (713 ± 2) $cm^{-1}$, (1085 ± 2) $cm^{-1}$, (1153 ± 2) $cm^{-1}$, (1213 ± 2) $cm^{-1}$, (1321 ± 2) $cm^{-1}$, (1346 ± 2) $cm^{-1}$, (1521 ± 2) $cm^{-1}$, (1612 ± 2) $cm^{-1}$, (1683 ± 2) $cm^{-1}$ and (1774 ± 2) $cm^{-1}$, when measured at room temperature with a diamond ATR cell.

Alternatively, the crystalline Form II of the compound of formula (II) is characterized by having a Fourier transform Infrared spectrum essentially the same as displayed in Figure 24 of the present invention, when measured at room temperature with a diamond ATR cell.

Alternatively or additionally, the crystalline Form II of the compound of formula (II) is characterized by having a Raman spectrum comprising peaks at wavenumbers of (1335 ± 2) $cm^{-1}$, (1348 ± 2) $cm^{-1}$ and (1592 ± 2) $cm^{-1}$, when measured at room temperature with a laser at a wavelength of 1064nm. Preferably, the crystalline Form II of the compound of formula (II) is characterized by having a Raman spectrum comprising peaks at wavenumbers of (1111 ± 2) $cm^{-1}$, (1335 ± 2) $cm^{-1}$, (1348 ± 2) $cm^{-1}$, (1592 ± 2) $cm^{-1}$ and (2935 ± 2) $cm^{-1}$, when measured at room temperature with a laser at a wavelength of 1064nm. Preferably, the crystalline Form II of the compound of formula (II) is characterized by having a Raman spectrum comprising bands at wavenumbers of (864 ± 2) $cm^{-1}$, (1461 ± 2) $cm^{-1}$, (1111 ± 2) $cm^{-1}$, (1335 ± 2) $cm^{-1}$, (1348 ± 2) $cm^{-1}$, (1592 ± 2) $cm^{-1}$ and (2935 ± 2) $cm^{-1}$, when measured at room temperature with a laser at a wavelength of 1064nm. Preferably, the crystalline Form II of the compound of formula (II) is characterized by having a Raman spectrum comprising bands at wavenumbers of (864 ± 2) $cm^{-1}$, (1461 ± 2) $cm^{-1}$, (1111 ± 2) $cm^{-1}$, (1215 ± 2) $cm^{-1}$, (1295 ± 2) $cm^{-1}$, (1335 ± 2) $cm^{-1}$, (1348 ± 2) $cm^{-1}$, (1592 ± 2) $cm^{-1}$, (2885 ± 2) $cm^{-1}$ and (2935 ± 2) $cm^{-1}$, when measured at room temperature with a laser at a wavelength of 1064nm. Alternatively, the crystalline Form II of the compound of formula (II) is characterized by having a Raman spectrum essentially the same as displayed in Figure 25 of the present invention, when measured at room temperature with a laser at a wavelength of 1064nm.

Alternatively or additionally, the crystalline Form II of the compound of formula (II) is characterized by having a differential scanning calorimetric curve as displayed in Figure 26 of the present invention.

Alternatively or additionally, the crystalline Form II of the compound of formula (II) is characterized by having a TGA curve as displayed in Figure 27 of the present invention.

The crystalline Form III of the compound of formula (II) is characterized by having a powder X-ray diffractogram comprising reflections at 2-Theta angles of (5.8 ± 0.2)°, (10.2 ± 0.2)° and (11.1 ± 0.2)°, when measured at room temperature with Cu-Kalpha1 radiation having a wavelength of 0.15419 nm. Preferably, the crystalline Form III of the compound of formula (II) is characterized by having a powder X-ray diffractogram comprising reflections at 2-Theta angles of (5.8 ± 0.2)°, (9.6 ± 0.2)°, (10.2 ± 0.2)°, (11.1 ± 0.2)° and (11.3 ± 0.2)°, when measured at room temperature with Cu-Kalpha1 radiation having a wavelength of 0.15419 nm. Preferably, the crystalline Form III of the compound of formula (II) is characterized by having a powder X-ray diffractogram comprising reflections at 2-Theta angles of (5.6 ± 0.2)°, (5.8 ± 0.2)°, (9.6 ± 0.2)°, (10.2 ± 0.2)°, (11.1 ± 0.2)°, (11.3 ± 0.2)° and (12.1 ± 0.2)°, when measured at room temperature with Cu-Kalpha1 radiation having a wavelength of 0.15419 nm. Preferably, the crystalline Form III of the compound of formula (II) is characterized by having a powder X-ray diffractogram comprising reflections at 2-Theta angles of (5.6 ± 0.2)°, (5.8 ± 0.2)°, (7.7 ± 0.2)°, (9.6 ± 0.2)°, (10.2 ± 0.2)°, (11.1 ± 0.2)°, (11.3 ± 0.2)°, (12.1 ± 0.2)°, (13.0 ± 0.2)° and (14.3 ± 0.2)°, when measured at room temperature with Cu-Kalpha1 radiation having a wavelength of 0.15419 nm.

Alternatively, the crystalline Form III of the compound of formula (II) is characterized by having a powder X-ray diffractogram essentially the same as displayed in Figure 28 of the present invention, when measured at room temperature with Cu-Kalpha1 radiation having a wavelength of 0.15419 nm.

Alternatively or additionally, the crystalline Form III of the compound of formula (II) is characterized by having a Fourier transform Infrared spectrum comprising bands at wavenumbers of (1083 ± 2) $cm^{-1}$, (1329 ± 2) $cm^{-1}$ and (1607 ± 2) $cm^{-1}$, when measured at room temperature with a diamond ATR cell. Preferably, the crystalline Form III of the compound of formula (II) is characterized by having a Fourier transform Infrared spectrum comprising bands at wavenumbers of (1083 ± 2) $cm^{-1}$, (1291 ± 2) $cm^{-1}$, (1329 ± 2) $cm^{-1}$, (1521 ± 2) $cm^{-1}$ and (1607 ± 2) $cm^{-1}$, when measured

at room temperature with a diamond ATR cell. Preferably, the crystalline Form III of the compound of formula (II) is characterized by having a Fourier transform Infrared spectrum comprising bands at wavenumbers of (1083 ± 2) $cm^{-1}$, (1131 ± 2) $cm^{-1}$, (1291 ± 2) $cm^{-1}$, (1329 ± 2) $cm^{-1}$, (1347 ± 2) $cm^{-1}$, (1521 ± 2) $cm^{-1}$ and (1607 ± 2) $cm^{-1}$, when measured at room temperature with a diamond ATR cell. Preferably, the crystalline Form III of the compound of formula (II) is characterized by having a Fourier transform Infrared spectrum comprising bands at wavenumbers of (709 ± 2) $cm^{-1}$, (1083 ± 2) $cm^{-1}$, (1131 ± 2) $cm^{-1}$, (1291 ± 2) $cm^{-1}$, (1329 ± 2) $cm^{-1}$, (1347 ± 2) $cm^{-1}$, (1521 ± 2) $cm^{-1}$, (1607 ± 2) $cm^{-1}$, (1688 ± 2) $cm^{-1}$ and (1777 ± 2) $cm^{-1}$, when measured at room temperature with a diamond ATR cell.

Alternatively, the crystalline Form III of the compound of formula (II) is characterized by having a Fourier transform Infrared spectrum essentially the same as displayed in Figure 29 of the present invention, when measured at room temperature with a diamond ATR cell.

Alternatively or additionally, the crystalline Form III of the compound of formula (II) is characterized by having a Raman spectrum comprising bands at wavenumbers of (1113 ± 2) $cm^{-1}$, (1332 ± 2) $cm^{-1}$ and (1350 ± 2) $cm^{-1}$, when measured at room temperature with a laser at a wavelength of 1064nm. Preferably, the crystalline Form III of the compound of formula (II) is characterized by having a Raman spectrum comprising bands at wavenumbers of (1113 ± 2) $cm^{-1}$, (1295 ± 2) $cm^{-1}$, (1332 ± 2) $cm^{-1}$, (1350 ± 2) $cm^{-1}$ and (1592 ± 2) $cm^{-1}$, when measured at room temperature with a laser at a wavelength of 1064nm. Preferably, the crystalline Form III of the compound of formula (II) is characterized by having a Raman spectrum comprising bands at wavenumbers of (864 ± 2) $cm^{-1}$, (1113 ± 2) $cm^{-1}$, (1295 ± 2) $cm^{-1}$, (1332 ± 2) $cm^{-1}$, (1350 ± 2) $cm^{-1}$, (1592 ± 2) $cm^{-1}$ and (2932 ± 2) $cm^{-1}$, when measured at room temperature with a laser at a wavelength of 1064nm. Preferably, the crystalline Form III of the compound of formula (II) is characterized by having a Raman spectrum comprising bands at wavenumbers of (864 ± 2) $cm^{-1}$, (1113 ± 2) $cm^{-1}$, (1215 ± 2) $cm^{-1}$, (1295 ± 2) $cm^{-1}$, (1332 ± 2) $cm^{-1}$, (1350 ± 2) $cm^{-1}$, (1463 ± 2) $cm^{-1}$, (1592 ± 2) $cm^{-1}$, (2883 ± 2) $cm^{-1}$ and (2932 ± 2) $cm^{-1}$, when measured at room temperature with a laser at a wavelength of 1064nm.

Alternatively, the crystalline Form III of the compound of formula (II) is characterized by having a Raman spectrum essentially the same as displayed in Figure 30 of the present invention, when measured at room temperature with a laser at a wavelength of 1064nm.

Alternatively or additionally, the crystalline Form III of the compound of formula (II) is characterized by having a differential scanning calorimetric curve as displayed in Figure 31 of the present invention.

Alternatively or additionally, the crystalline Form III of the compound of formula (II) is characterized by having a TGA curve as displayed in Figure 32 of the present invention.

**Process for the preparation of a compound of formula (II) in crystalline form**

In a further aspect, the present invention relates to a process for the preparation of a compound of formula (II) in crystalline form, said process comprising

(a) providing a compound of formula (IV)

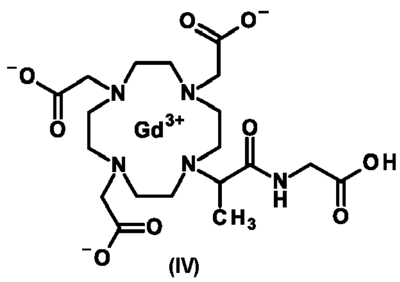

(IV)

(b) reacting the compound of formula (IV) with para-nitrophenol in the presence of a coupling agent
(c) and isolating the compound of formula (II) in crystalline form.

In a preferred embodiment, the compound of formula (II) is isolated in a crystalline form as described herein above. In a preferred embodiment, the compound of formula (II) is isolated in crystalline Form I, as described and characterized above.

In a preferred embodiment, the compound of formula (II) is isolated in a crystalline form characterized by having a powder X-ray diffractogram comprising reflections at 2-Theta angles of (8.0 ± 0.2)°, (9.0 ± 0.2)°, (11.7 ± 0.2)°, (12.8 ± 0.2)° and (14.9 ± 0.2)°, when measured at room temperature with Cu-Kalpha1 radiation having a wavelength of 0.15419 nm.

In a preferred embodiment, the compound of formula (II) is isolated in a crystalline form characterized by having

a powder X-ray diffractogram essentially the same as displayed in Figure 18 of the present invention, when measured at room temperature with Cu-Kalpha1 radiation having a wavelength of 0.15419 nm.

In a preferred embodiment, the compound of formula (II) is isolated in a crystalline form characterized by having a Fourier transform Infrared spectrum comprising bands at wavenumbers of (715 $\pm$ 2) $cm^{-1}$, (1085 $\pm$ 2) $cm^{-1}$, (1343 $\pm$ 2) $cm^{-1}$, (1522 $\pm$ 2) $cm^{-1}$ and (1598 $\pm$ 2) $cm^{-1}$, when measured at room temperature with a diamond ATR cell.

In a preferred embodiment, the compound of formula (II) is isolated in a crystalline form characterized by having a Fourier transform Infrared spectrum essentially the same as displayed in Figure 19 of the present invention.

In a preferred embodiment, the compound of formula (II) is isolated in a crystalline form characterized by having a Raman spectrum comprising bands at wavenumbers of (1111 $\pm$ 2) $cm^{-1}$, (1213 $\pm$ 2) $cm^{-1}$, (1346 $\pm$ 2) $cm^{-1}$, (1594 $\pm$ 2) $cm^{-1}$ and (2884 $\pm$ 2) $cm^{-1}$.

In a preferred embodiment, the compound of formula (II) is isolated in a crystalline form characterized by having a Raman spectrum essentially the same as displayed in Figure 20 of the present invention.

In a preferred embodiment, the compound of formula (II) is isolated in a crystalline form characterized by having a differential scanning calorimetric curve as displayed in Figure 21 of the present invention.

In a preferred embodiment, the compound of formula (II) is isolated in a crystalline form characterized by having a TGA curve as displayed in Figure 222 of the present invention.

In a preferred embodiment, the reaction of the compound of formula (IV) with para-nitrophenol in the presence of a coupling agent in step (b) is preferably carried out in an organic solvent or in a mixture of organic solvents. Preferably, the organic solvent is acetonitrile, formamide, tetrahydrofurane, 2-methyltetrahydrofurane or a mixture thereof. More preferably, the reaction of the compound of formula (IV) with para-nitrophenol in the presence of a coupling agent in step (b) is carried out in a mixture of acetonitrile and formamide or in a mixture of tetrahydrofurane and formamide. More preferably, the solvent mixture for the reaction of the compound of formula (IV) with para-nitrophenol comprises acetonitrile and formamide in a ratio of from 2:1 to 5:1 by weight (w/w), preferably of from 2.3:1 to 4.0:1 by weight (w/w)

In a preferred embodiment, the reaction of the compound of formula (IV) with para-nitrophenol in step (d) is carried out in the presence of a coupling agent, preferably a coupling agent selected from DIC (N,N-diisopropylcarbodiimide), DCC (N,N-dicyclohexylcarbodiimide), EDCI (1-ethyl-3-(3-dimethylaminopropyl)carbodiimide) and CDI (carbonyldiimidazol). Preferably, the reaction is carried out using DIC (N,N-diisopropylcarbodiimide) as the coupling agent. Preferably, prior to the addition of the coupling agent, a mixture of the compound of formula (IV) and para-nitrophenol in the suitable or preferred organic solvent or solvent mixture is stirred for an amount of time of from 0.5 h to 5 h, more preferably of from 1 h to 3 h. Preferably, prior to the addition of the coupling agent, a mixture of the compound of formula (IV) and para-nitrophenol in the suitable or preferred organic solvent or solvent mixture is stirred at a temperature of from -5 °C to 5 °C, more preferably of from -3 °C to 3 °C. Even more preferably, prior to the addition of the coupling agent, a mixture of the compound of formula (IV) and para-nitrophenol in the suitable or preferred organic solvent or solvent mixture is stirred at a temperature of from -5 °C to 5 °C for 0.5 h to 5 h. Even more preferably, prior to the addition of the coupling agent, a mixture of the compound of formula (IV) and para-nitrophenol is stirred in the suitable or preferred organic solvent or solvent mixture at a temperature of from -3 °C to 3 °C for 1 h to 3 h.

In a preferred embodiment, the reaction of the compound of formula (IV) with para-nitrophenol in the presence of a coupling agent in step (d) is preferably carried out at a temperature of from 15 °C to 40 °C, more preferably of from 20 °C to 26 °C.

In a preferred embodiment, the reaction of the compound of formula (IV) with para-nitrophenol in the presence of a coupling agent in step (d) is preferably carried out for 6 h to 72 h, more preferably of from 10 h to 43 h, most preferably from 12 h to 19 h.

In a preferred embodiment, the reaction of the compound of formula (IV) with para-nitrophenol in the presence of a coupling agent in step (d) is preferably carried out at a temperature of from 15 °C to 40 °C, more preferably of from 20 °C to 26 °C and for 6 h to 72 h, more preferably of from 10 h to 43 h, most preferably from 12 h to 19 h.

In a preferred embodiment, the isolation of the compound of formula (IV) in step (e) comprises the addition of a mixture of water and an organic solvent selected from tetrahydrofurane and acetonitrile or a mixture thereof to the reaction mixture. Preferably, the organic solvent is acetonitrile.

Preferably, the amount of water in the mixture of water and organic solvent added is selected so that the final water content of the complete reaction mixture lies in the range of from 0% to 6% by weight (w/w), more preferably of from 2% to 5% by weight (w/w).

In a preferred embodiment, the present invention relates to a process for the preparation of a compound of formula (II) in crystalline form, said process comprising

(a) providing a compound of formula (IV)

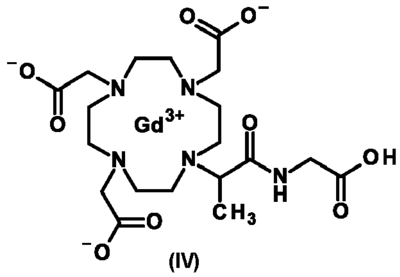

(IV)

(b) reacting the compound of formula (IV) with para-nitrophenol in the presence of a coupling agent, wherein the compound of formula (IV) and para-nitrophenol are stirred before the coupling agent is added to the reaction mixture,
(c) and isolating the compound of formula (II) in crystalline form.

In a preferred embodiment, the present invention relates to a process for the preparation of a compound of formula (II) in crystalline form, said process comprising

(a) providing a compound of formula (IV)

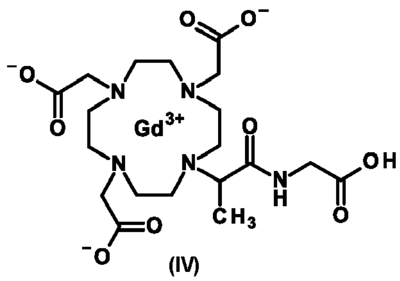

(IV)

(b) reacting the compound of formula (IV) with para-nitrophenol in the presence of the coupling agent N,N-diisopropylcarbodiimide, wherein the compound of formula (IV) and para-nitrophenol are stirred before the coupling agent is added to the reaction mixture,
(c) and isolating the compound of formula (II) in crystalline form.

In a preferred embodiment, the present invention relates to a process for the preparation of a compound of formula (II) in crystalline form, said process comprising

(a) providing a compound of formula (IV)

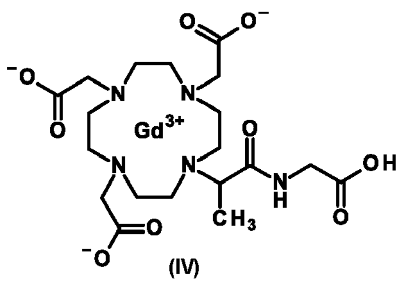

(IV)

(b) reacting the compound of formula (IV) with para-nitrophenol in the presence of a coupling agent, wherein the compound of formula (IV) and para-nitrophenol are stirred for 0.5 h to 5 h, preferably for 1 h to 3 h before the coupling agent is added to the reaction mixture,
(c) and isolating the compound of formula (II) in crystalline form.

In a preferred embodiment, the present invention relates to a process for the preparation of a compound of formula (II) in crystalline form, said process comprising

(a) providing a compound of formula (IV)

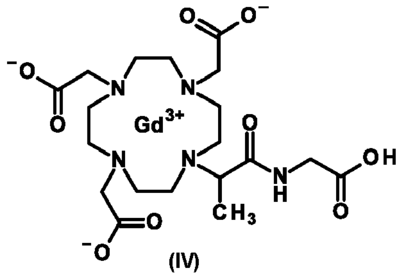

(IV)

(b) reacting the compound of formula (IV) with para-nitrophenol in the presence of the coupling agent N,N-diisopropylcarbodiimide, wherein the compound of formula (IV) and para-nitrophenol are stirred for 0.5 h to 5 h, preferably for 1 h to 3 h before the coupling agent is added to the reaction mixture,
(c) and isolating the compound of formula (II) in crystalline form.

In a preferred embodiment, the present invention relates to a process for the preparation of a compound of formula (II) in crystalline form, said process comprising

(a) providing a compound of formula (IV)

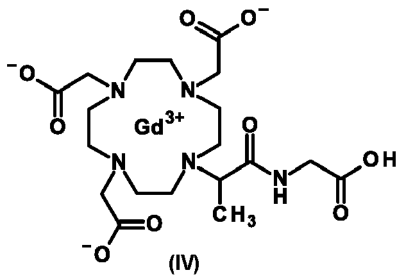

(IV)

(b) reacting the compound of formula (IV) with para-nitrophenol in the presence of a coupling agent, wherein the compound of formula (IV) and para-nitrophenol are stirred for 0.5 h to 5 h, preferably for 1 h to 3 h at a temperature of from -3 °C to 3 °C before the coupling agent is added to the reaction mixture,
(c) and isolating the compound of formula (II) in crystalline form.

In a preferred embodiment, the present invention relates to a process for the preparation of a compound of formula (II) in crystalline form, said process comprising

(a) providing a compound of formula (IV)

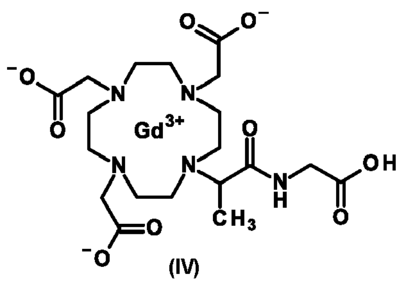

(IV)

(b) reacting the compound of formula (IV) with para-nitrophenol in the presence of a coupling agent, wherein the compound of formula (IV) and para-nitrophenol are stirred for 0.5 h to 5 h, preferably for 1 h to 3 h at a temperature of from -3 °C to 3 °C before the coupling agent is added to the reaction mixture,
(c) and isolating the compound of formula (II) in a crystalline form characterized by having a powder X-ray diffractogram comprising reflections at 2-Theta angles of (8.0 ± 0.2)°, (9.0 ± 0.2)°, and (12.8 ± 0.2)°, when measured at room temperature with Cu-Kalpha1 radiation having a wavelength of 0.15419 nm.

In a preferred embodiment, the present invention relates to a process for the preparation of a compound of

formula (II) in crystalline form, said process comprising

(a) providing a compound of formula (IV)

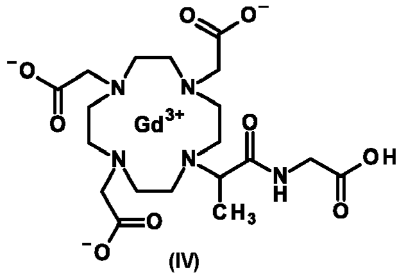

(IV)

(b) reacting the compound of formula (IV) with para-nitrophenol in the presence of the coupling agent N,N-diisopropylcarbodiimide, wherein the compound of formula (IV) and para-nitrophenol are stirred for 0.5 h to 5 h, preferably for 1 h to 3 h at a temperature of from -3 °C to 3 °C before the coupling agent is added to the reaction mixture,
(c) and isolating the compound of formula (II) in a crystalline form characterized by having a powder X-ray diffractogram comprising reflections at 2-Theta angles of (8.0 ± 0.2)°, (9.0 ± 0.2)°, and (12.8 ± 0.2)°, when measured at room temperature with Cu-Kalpha1 radiation having a wavelength of 0.15419 nm.

In a preferred embodiment, the present invention relates to a process for the preparation of a compound of formula (II) in crystalline form, said process comprising

(a) providing a compound of formula (IV)

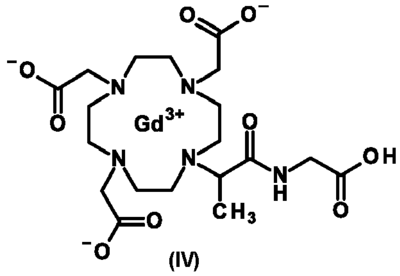

(IV)

(b) reacting the compound of formula (IV) with para-nitrophenol in the presence of a coupling agent, wherein the compound of formula (IV) and para-nitrophenol are stirred for 0.5 h to 5 h, preferably for 1 h to 3 h at a temperature of from -3 °C to 3 °C before the coupling agent is added to the reaction mixture,
(c) and isolating the compound of formula (II) in a crystalline form characterized by having a powder X-ray diffractogram comprising reflections at 2-Theta angles of (8.0 ± 0.2)°, (9.0 ± 0.2)°, and (12.8 ± 0.2)°, when measured at room temperature with Cu-Kalpha1 radiation having a wavelength of 0.15419 nm, wherein the isolation comprises the use of a mixture of water and an organic solvent, preferably wherein the organic solvent is acetonitrile, so that the final water content of the complete reaction mixture lies in the range of from 0% to 6% by weight (w/w), more preferably of from 2% to 5% by weight (w/w).

It is to be understood that the present invention relates also to any combination of the embodiments described above.
More particularly still, the present invention covers the intermediate compounds which are disclosed in the example section of this text, *infra.*

**GENERAL SYNTHESIS**

The compounds of formula (III) and (IV) according to the process of the present invention can be prepared as described in the literature.
The compound of formula (III) can be prepared from pentaerythrityl tetrabromide according to W. Hayes, et al., Tetrahedron 59 (2003), 7983 - 7996.
The compound of formula (IV) can be prepared starting from 10-Tetraazacyclododecane as described in DE

19652386, Schering AG.

## DESCRIPTION OF THE FIGURES

Figure 1 Relative humidity as a function of the temperature and the vapor pressure
Figure 2 X-Ray Powder Diffraction Spectrum of the compound of formula (I) - Amorphous Material
Figure 3 X-Ray Powder Diffraction Spectrum of the compound of formula (I) - Form I
Figure 4 IR Spectrum of the compound of formula (I) - Form I
Figure 5 Raman Spectrum of the compound of formula (I) - Form I
Figure 6 DSC curve of the compound of formula (I) - Form I
Figure 7 TGA curve of the compound of formula (I) - Form I
Figure 8 X-Ray Powder Diffraction Spectrum of the compound of formula (I) - Form II
Figure 9 IR Spectrum of the compound of formula (I) - Form II
Figure 10 Raman Spectrum of the compound of formula (I) - Form II
Figure 11 DSC curve of the compound of formula (I) - Form II
Figure 12 TGA curve of the compound of formula (I) - Form II
Figure 13 DVS Isotherm Plot for the conversion of the crystalline forms of the compound of formula (I) (Form I to Form II) 50-0-90-50 at 20°C
Figure 14 DVS Isotherm Plot for the conversion of the crystalline forms of the compound of formula (I) (Form I to Form II) 50-0-50 at 20°C
Figure 15 DVS Isotherm Plot for the conversion of the crystalline forms of the compound of formula (I) (Form I to Form II) 50-0-50 at 40°C
Figure 16. Asymmetric part of the unit cell for diastereomer 1 of the compound of formula (I)
Figure 17. Asymmetric part of the unit cell for diastereomer 3 of the compound of formula (I)
Figure 18. X-Ray Powder Diffraction Spectrum of the compound of formula (II) - Form I
Figure 19. IR Spectrum of the compound of formula (II) - Form I
Figure 20. Raman Spectrum of the compound of formula (II) - Form I
Figure 21. DSC curve of the compound of formula (II) - Form I
Figure 22. TGA curve of the compound of formula (II) - Form I
Figure 23. X-Ray Powder Diffraction Spectrum of the compound of formula (II) - Form II
Figure 24. IR Spectrum of the compound of formula (II) - Form II
Figure 25. Raman Spectrum of the compound of formula (II) - Form II
Figure 26. DSC curve of the compound of formula (II) - Form II
Figure 27. TGA curve of the compound of formula (II) - Form II
Figure 28. X-Ray Powder Diffraction Spectrum of the compound of formula (II) - Form III
Figure 29. IR Spectrum of the compound of formula (II) - Form III
Figure 30. Raman Spectrum of the compound of formula (II) - Form III
Figure 31. DSC curve of the compound of formula (II) - Form III
Figure 32. TGA curve of the compound of formula (II) - Form III
Figure 33. X-Ray Powder Diffraction Spectrum of a transient crystalline form of the compound of formula (I) during the second drying process in the preparation of crystalline Form II of the compound of formula (I).
Figure 34a. Sample solution 1 for assay of the compound of the formula (II) via hydrolysis to 4-Nitrophenol (HPLC-UV method b).
Figure 34b. Sample solution 2 (hydrolyzed) for assay of the compound of the formula (II) via hydrolysis to 4-Nitrophenol (HPLC-UV method b).
Figure 35a. SST solution for assay of the compound of the formula (II) via derivatization to the benzylamide derivative of the compound of the formula (IV) (HPLC-UV method c).
Figure 35b. SST solution of the compound of the formula (IV) *in* the compound of the formula (II) (HPLC-UV method c-1).
Figure 36a. SST solution 1 of the compound of the formula (I) crude (HPLC-UV method d-1).
Figure 36b. SST solution 2 of the compound of the formula (I) crude (HPLC-UV method d-1).
Figure 36c. SST solution of the compound of the formula (I) (HPLC-UV method d-1).
Figure 37. HPLC chromatogram of the isolated material, example 2, compound of the formula (I) crude (HPLC-UV method d-1).
Figure 38. HPLC chromatogram of the isolated material, example 4, compound of the formula (I) (HPLC-UV method d-1).

Figure 39. HPLC chromatogram of the isolated material, example 4.1, compound of the formula (I) (HPLC-UV method d-1).

## EXPERIMENTAL SECTION

### Abbreviations

| | |
|---|---|
| ACN | acetonitrile |
| AUC | area under the curve |
| bw | body weight |
| $CDCl_3$ | chloroform-d |
| CPMG | Carr-Purcell-Meiboom-Gill (MRI sequence) |
| $C_{Gd}$ | concentration of the compound normalized to the Gadolinium |
| $Cl_{tot}$ | total clearance |
| d | day(s) |
| $D_2O$ | deuterium oxide |
| DAD | diode array detector |
| DCM | dichloromethane |
| DMSO | dimethylsulfoxide |
| DMSO-$d_6$ | deuterated dimethylsulfoxide |
| DSC | Differential scanning calorimetry |
| DVS | Dynamic vapor sorption |
| ECCM | extracellular contrast media |
| EI | electron ionisation |
| ELSD | evaporative light scattering detector |
| ESI | electrospray ionisation |
| FBS | fetal bovine serum |
| h | hour |
| HCOOH | formic acid |
| HPLC | high performance liquid chromatography |
| HU | Hounsfield units |
| IR | inversion recovery |
| kDa | kilo Dalton |
| LC-MS | liquid chromatography-mass spectroscopy |
| ICP-MS | inductively coupled plasma mass spectrometry |
| MRI | magnetic resonance imaging |
| MRT | mean residence time |
| MS | mass spectrometry |
| m | multiplet |
| min | minute(s) |

(continued)

| | |
|---|---|
| NMR | nuclear magnetic resonance spectroscopy: chemical shifts (δ) are given in ppm. |
| q | quartet |
| $r_i$ | (where i=1, 2) relaxivities in L $mmol^{-1}$ $s^{-1}$ |
| $R_t$ | retention time |
| s | singlet |
| RC | reference compound |
| $R_i$ | (where i=1, 2) relaxation rates ($1/T_{1,2}$) |
| $R_{i(0)}$ | relaxation rate of the respective solvent |
| $T_{1,2}$ | relaxation time |
| T | Tesla |
| t | triplet |
| t½ α | plasma half-life, compartment V1 |
| t½ β | plasma half-life, compartment V2 |
| t½ γ | plasma half-life, compartment V3 |
| TFA | trifluoroacetic acid |
| TGA | Thermogravimetric analysis |
| THF | tetrahydrofuran |
| TI | inversion time |
| UPLC | ultra performance liquid chromatography |
| V1 + V2 | volume, compartments V1+V2 |
| $V_c$ (V1) | volume, central compartment V1 |
| $V_{d,ss}$ | volume of distribution at steady state |
| XRPD | X-ray powder diffraction |

**Materials and Instrumentation**

Unless stated otherwise, the chemicals used for the synthetic work were of reagent grade quality and were used as obtained.

All reagents, for which the synthesis is not described in the experimental section, are either commercially available, or are known compounds or may be formed from known compounds by known methods by a person skilled in the art.

**Instrumental set-up for XRPD measurements (Compound of formula (I) amorphous)**

X-ray powder diffraction (XRPD) data were recorded on a Panalytical X-Pert PRO diffractometer using Cu-$K_\alpha$-radiation, a position sensitive detector, at generator settings of 40 kV and 40 mA. The samples were collected in transition mode, being prepared as a thin layer between two foils. The scanning range was between 2° and 38° 2 theta with a 0.013° step at 25 sec/step.

**Instrumental set-up for XRPD measurements (Compound of formula (I) crystalline Forms I and II including transient crystalline form)**

X-ray powder diffraction (XRPD) data were recorded on a STOE STADI P diffractometer using monochromatized $CuK_{\alpha 1}$-radiation, a position sensitive detector, at generator settings of 40 kV and 40 mA. The samples were collected in transition mode, being prepared as a thin layer between two foils. The scanning range was between 2 ° and 38 ° 2

theta with a 0.2° step at 15 sec/step.

**Instrumental set-up for XRPD measurements (Compound of formula (II) crystalline Forms I, II and III)**

X-ray powder diffraction (XRPD) data were recorded on a STOE STADI P diffractometer using monochromatized $CuK_{\alpha 1}$-radiation, a position sensitive detector, at generator settings of 40 kV and 40 mA. The samples were collected in transition mode, being prepared as a thin layer between two foils. The scanning range was between 2 ° and 40 ° 2 theta with a 0.5° step at 15 sec/step.

**Instrumental set-up for SCD measurements (Compound of formula (I) diastereomer 1)**

The crystallographic data of diastereomer 1 of the compound of formula (I) as well as a figure depicting the thermal ellipsoids and numbering of the structure, are shown in Table 4 and Figure 16. Colorless crystals were obtained by dissolving 100 mg compound of formula (I) diastereomer 1 (see example 5) in 150 mg water at 60 °C, slowly adding 700 mg ethanol, and finally slowly diffusing ethanol vapour into the solution at 60 °C.

Single crystal X-ray diffraction data were collected on a Rigaku Oxford Diffraction XtaLAB Synergy-S diffractometer equipped with a dualflex source (Cu at Zero), HyPix-6000HE detector and an Oxford Cryosystems Cobra cooling device. The data were collected using $CuK\alpha$ radiation. The structure was solved and refined using the Shelx suite of programs and OLEX2 was used as an interface to view the structure with and produce figures. Unless otherwise stated, hydrogen atoms attached to carbon were placed geometrically and allowed to refine with a riding isotropic displacement parameter. Hydrogen atoms attached to a heteroatom were located in a difference Fourier synthesis map and were allowed to refine freely with an isotropic displacement parameter. The asymmetric unit was found to contain ¼ of compound of formula (I) diastereomer 1 where a fully occupied $Gd^{3+}$ ion is coordinated to the ligand. It also contains one fully occupied water molecule, 1 fully occupied EtOH molecules and two partially occupied EtOH molecules refined to 75% and 80% occupancy each (see Figure x). The molecule of compound of formula (I) contains four Gd-ligand units with the central carbon atom (C21) sitting on special position (rotoinversion). The crystal structure belongs to the symmorphic spacegroup I-4 and the rotoinversion symmetry element -4 implies that the investigated sample of compound of formula (I) (Example 5), is in fact the R,S,R,S (or S,R,S,R) diastereoisomer and thus confirms the structure of the compound of formula (I) diastereomer 1.

**Instrumental set-up for SCD measurements (Compound of formula (I) diastereomer 3)**

The crystallographic data of diastereomer 3 of the compound of formula (I) as well as a figure depicting the thermal ellipsoids and numbering of the structure, are shown in Table 5 and Figure 17. Colorless crystals were obtained by dissolving 50 mg compound of formula (I) diastereomer 3 (see example 7) in 36 mg water at 60 °C, slowly adding 100 mg ethanol, and finally slowly diffusing iso-propanol vapour into the solution at 60 °C.

Single crystal X-ray diffraction data were collected on a Rigaku Oxford Diffraction XtaLAB Synergy-S diffractometer equipped with a dualflex source (Cu at Zero), HyPix-6000HE detector and an Oxford Cryosystems Cobra cooling device. The data were collected using $CuK\alpha$. The structures were solved and refined using the Shelx suite of programs and OLEX2 was used as an interface to view the structure with and produce the figures. Unless otherwise stated, hydrogen atoms attached to carbon were placed geometrically and allowed to refine with a riding isotropic displacement parameter. Hydrogen atoms attached to a heteroatom were located in a difference Fourier synthesis map and were allowed to refine freely with an isotropic displacement parameter. The asymmetric unit was found to contain one molecule of compound of formula (I) diastereomer 3 (see Figure 17). In the crystal structure there is a significant amount of diffuse electron density corresponding to heavily disordered and partially occupied solvent molecules, located in the large voids between neighboring molecules of compound of formula (I) diastereomer 3. This diffuse electron density was removed during structure refinement using an Olex2 implementation of the Platon Squeeze routine. This approach significantly improved the final model and allowed the least-squares refinement to convergence. Due to the heavily diffuse nature of the electron density, all hydrogen atoms located on heteroatoms were refined at calculated positions (AFIX 47) as riding on their parent atoms. In the structure one disordered carboxylate group was modelled over two sites and refined with 0.60:0.40 ratio. In the structure, 50 % of compound of formula (I) molecules have all stereocenters (C15, C36, C45, C76) in the R configuration and another 50 % in the S configuration (centrosymmetric space group P21/n). This means that the crystal structure of the sample of compound of formula (I) (example 7) contains an equimolar mixture of (R,R,R,R) and (S,S,S,S) diastereomers and confirms the structure compound of formula (I) diastereomer 3.

Instrumental set-up for IR measurements

IR measurements were performed with a Bruker alpha spectrometer in the attenuated total reflectance (ATR)

geometry. No sample preparation was performed, and each individual measurement consisted of 32 scans.

**Instrumental set-up for Raman measurements**

Raman measurements were performed with a Bruker MultiRAM spectrometer. No sample preparation was performed, and each individual measurement consisted of 64 scans using a laser power of 300 mW.

**Instrumental set-up for TGA measurements (compound of formula (I) crystalline Forms I and I)**

Thermogravimetric analysis (TGA) was performed with a Mettler Toledo TGA/DSC 3+. The instrument was purged with nitrogen gas at a flow rate of 50 ml.min$^{-1}$. Approximately 3 - 10 mg of each sample was placed into an aluminum crucible and heated at a heating rate of 10 °C.min $^{-1}$ from 25 °C to 350 °C.

**Instrumental set-up for TGA measurements (compound of formula (II) crystalline Forms I, II and III)**

Thermogravimetric analysis (TGA) was performed with a Mettler Toledo TGA/DSC 3+. The instrument was purged with nitrogen gas at a flow rate of 10 or 20 ml.min$^{-1}$. Approximately 1 - 15 mg of each sample was placed into either an aluminum or an aluminum oxide crucible and heated at a heating rate of 10 °C.min $^{-1}$ from 25 °C.

**Instrumental set-up for DSC measurements**

Differential scanning calorimetry (DSC) was performed with a Mettler Toledo DSC3+. The calorimeter was purged with nitrogen gas at a flow rate of 50 ml.min$^{-1}$. 3 - 10 mg of each sample was placed into an aluminum crucible and heated at a rate of 20 °C.min $^{-1}$ from -10 °C to 250 °C.

**Instrumental set-up for DVS measurements (compound of formula (I) crystalline Forms I and II)**

Water sorption isotherms were determined using a Surface Measurement Systems Ltd DVS1 gravimetric sorption analyzer. 10 - 20 mg of sample was equilibrated at 50% relative humidity and the weight recorded. The isotherm was then recorded by changing the humidity in steps of 10%. The equilibrium criterion was set to a relative mass change of dm / dt = 0.002% / minute.

**Analytical methods**

**HPLC Chromatography**

<u>HPLC-UV Method a:</u>

Instrument: Agilent Series 1260, pump: G1312B , autosampler: G1329B, degasser: G4225A, column oven: G1316C, detector: G1314F; detection wavelength: 198 nm, bandwidth: 8 nm; data rate: 40 Hz; column temperature: 40 °C, column: YMC Triart Phenyl C18 100 mm × 3 mm, 3 μm, eluent A: 0.1 wt-% formic acid in water; eluent B: acetonitrile, flow rate: 1.20 mL/min; gradient (A): 0 min: 98%; 1 min: 98%; 9 min: 20%; 11 min: 20%; equilibration time: 3 min, injection volume: 10 μL.

<u>HPLC-UV Method b: (Assay for compound of formula (II))</u>

Evaluation by hydrolysis to 4-Nitrophenol, sample preparation protocol:

Sample solution 1: dissolve approx. 0.2 mg/mL sample in a mix of 85 % Acetonitrile+15% water (v/v). The sample must be dissolved directly prior to analysis only (max. duration between sample dissolution and injection: 15 min).

Sample solution 2: dissolve approx. 0.2 mg/mL sample in 50 % of the necessary volume of a 14 mmol/L disodium hydrogen phosphate solution and shake it for 2 h at 50 °C. After having let cooled down, fill up to volume (use sample solution within 24 h when stored at room temperature).

The assay of the compound of the formula (II) is determined indirectly via the 4-Nitrophenol content after hydrolysis of the sample (direct determination is not possible due to the inherent instability of the compound of the formula (II) in aqueous media/HPLC conditions). Evaluation of analyses is performed via external standard calibration

against 4-Nitrophenol standard. The content of 4-Nitrophenol is determined for sample solution 1 (before hydrolysis) and sample solution 2 (after hydrolysis). Subsequently, the content of 4-Nitrophenol as determined from sample solution 1 is subtracted from that as determined for sample solution 2. The difference is used for assay calculation of the compound of the formula (II). The assay of the compound of the formula (II) is back-calculated based on the factor considering the molecular weight fraction of the compound of the formula (II) to 4-Nitrophenol.

HPLC-UV parameters:

Instrument: e.g. Agilent Series 1290, detection wavelength: 316 nm, bandwidth: 6 nm; data rate: 10 Hz; column temperature: 40 °C, column: Acquity BEH Phenyl 100 mm × 3 mm, 1.7 μm, eluent A: 10 mmol/L ammonium phosphate buffer, pH 2.4 to 1 L; eluent B: acetonitrile, flow rate: 1.0 mL/min; gradient: 0 min: 0 % B; 1.0 min: 0 % B; 9.0 min: 50 % B; 11.0 min: 75% B; 11.5 min: 0 % B; 15.0 min: 0 % B; equilibration time: contained in gradient, injection volume: 1 μL.

HPLC-UV Method b-2 (Determination of formamide in the compound of the formula (II))

Sample solution: dissolve about 5 mg sample in B/A (see HPLC-UV parameters) 9/1 in a 50 mL volumetric flask and make up with volume. Evaluate by multilevel calibration over a suitable linear regression using a formamide reference standard.

HPLC-UV parameters

Instrument: e.g. Agilent Series 1290; detection wavelength: 195 nm; bandwidth: 4 nm; data rate: 10 Hz; column temperature: 40°C, column: Nucleodur HILIC, 125 mm × 4.6 mm, 3 μm, isocratic, 10% A / 90% B; A: 5 mM Ammonium phosphate buffer pH 2.4, B: Acetonitrile; flow rate: 1 mL/min; run time: 5 min; injection volume: 20 μL

HPLC-UV Method c (assay of the compound of the formula (II) via the benzylamide derivative of the compound of the formula (IV)):

Evaluation by derivatization of the compound of formula (II) to the benzylamide derivative of the compound of the formula (IV), sample preparation protocol:
Derivatization reagent: Benzylamine in DMSO (e.g. 1.75 μL/1 mL)

Preparation of reaction solution for blank: pipette 2 mL derivatization reagent in a small reaction vessel and stir for 30 min at 40 °C on a temperature controlled magnetic block.

Preparation of blank solution: dilute 90 μL of the blank reaction solution with 1410 μL of water.

Preparation of reaction solution for sample: prepare an approx. 10 mg/mL sample solution using derivatization reagent as solvent (e.g. weigh 20 mg substance in a small reaction vessel, add 2 mL derivatization reagent), and stir for 30 min at 40 °C on a temperature controlled magnetic block for complete reaction.

Preparation of sample solution: dilute 90 μL of the sample reaction solution with 1410 μL of water (sample concentration of approx. 0.6 mg/mL)

Evaluation of analyses is performed via external standard calibration against the benzylamide derivative of the compound of the formula (IV) standard. The assay for the compound of the formula (II) is calculated based on the factor considering the molecular weight fraction of the benzylamide derivative of the compound of the formula (IV) to the compound of the formula (II).

HPLC-UV parameters:

Instrument: e.g. Agilent Series 1260; detection wavelength: 200 nm; bandwidth: 4 nm; data rate: 5 Hz; column temperature: 25 °C; column: Waters Atlantis Premier BEH C18AX, 100 mm × 4.6 mm, 2.5 μm; eluent A: 0.1 % formic acid + 0.5 % acetonitrile in water, eluent B: 0.1 % formic acid with 30 % acetonitrile in water; flow rate: 1.0 mL/min; gradient: 0 min: 0 % B, 2.0 min: 0 % B; 14.0 min: 40 % B; 23.0 min: 100 % B; 30.0 min: 100 % B, equilibration time: 5 min at 0 % B; injection volume: 5 μL.

HPLC-UV Method c-1 (i.e., the compound of formula (IV) in the compound of formula (II)):

Evaluation from derivatization solution of HPLC-UV method c, sample preparation protocol:

Preparation of reaction solution for blank: Same as for "HPLC Method c (new, assay)".
Preparation of blank solution: 1:1 dilution of the blank reaction solution with water.

Preparation of reaction solution for sample: Same as for "HPLC Method c (new, assay)".
Preparation of sample solution: 1:1 dilution of the sample reaction solution with water (use sample concentration of approx. 5 mg/mL).

HPLC-UV parameters:

Same as for HPLC Method c

HPLC Method d-1:

Instrument: e.g. Agilent Series 1290, detection wavelength: 198 nm, bandwidth: 4 nm; data rate: 5 Hz; column temperature: 35 °C, column: Acquity BEH Phenyl 100 mm × 3 mm, 1.7 μm, eluent A: 30 mL Acetonitrile filled up with 10 mmol/L aqueous ammonium phosphate buffer, pH 2.4 to 1 L; eluent B: 600 mL acetonitrile + 400 mL aqueous ammonium phosphate buffer, pH 2.4, flow rate: 0.4 mL/min; gradient: 0 min: 100% A; 2.0 min: 100% A; 32.0 min: 93.0 % A; 37.0 min: 0% A; 47.0 min: 0 % A; equilibration time: 5 min, injection volume: 5 μL.

HPLC Method d-2 (alternative method to d-1):

Instrument: Agilent 1260 or 1290; detection wavelength: 200 nm, bandwidth: 6 nm; data rate: 10 Hz; column temperature: 20 °C, column: YMC Triart C18 150 mm × 3 mm, 3μm, aqueous potassium phosphate buffer: 1.36 g potassium hydrogen phosphate + 640 μL phosphoric acid (85%) made up with water to 1L; eluent A: 99% aqueous potassium phosphate buffer + 1% acetonitrile v/v; eluent B: 35 % aqueous potassium phosphate buffer + 65% acetonitrile v/v, flow rate: 0.4 mL/min; gradient: 0 min: 100 % A, 27.0 min: 95 % A, 37.0 min: 50 % A, 50 min: 0% A; 60 min: 0%; equilibration time: at least 10 min, injection volume: 10 μL.

HPLC Method e:

Instrument: Agilent Series 1260, pump: G1312B , autosampler: G1329B, degasser: G4225A, column oven: G1316C, detector: G1314F; detection wavelength: 200 nm, bandwidth: 6 nm; data rate: 10 Hz; column temperature: 20 °C, column: YMC Triart C18 150 mm × 3 mm, 3μm, 1.36 g potassium hydrogen phosphate + 640 μL phosphoric acid (85%) made up with water to 1L; eluent A: 99% aqueous potassium phosphate buffer + 1% acetonitrile v/v; eluent B: 95% aqueous potassium phosphate buffer + 5% acetonitrile v/v, flow rate: 0.4 mL/min; gradient: 0 min: 100 % A, 27.0 min: 95 % A, 37.0 min: 50 % A, 50 min: 0% A; 60 min: 0%; equilibration time: at least 10 min, injection volume: 10 μL.

**Determination of residual solvents**

*Formamide content (method a; GC):*

Instrument: Agilent GC HP 7890A o GC HP 7890B, autosampler: Agilent GC Headspace sampler 7693, injection temperature: 220 °C, injection volume: 0.5 μL; detection temperature: 300 °C; data rate: 10 Hz; column: Rxi-624 Sil MS 22 m × 0.18 mm × 1 μm, column flow: 0.7 mL/min, split flow: 3.5 mL/min, split ratio: 5; liner: SLG Focus-Liner part no 092219; analysis method: 50 °C, initial time 2.0 min, heat ramp 10 °C/min to 150 °C, heat ramp 70 °C/min to 250 °C, hold time 2.57 min; carries gas: hydrogen.

*Residual Solvent content (i.e. Ethanol) via GC-Head-Space (method a)*

Instrument: Agilent GC HP 7890A or similar instrument, autosampler: Perkin-Elmer HS 40 XL sampler or Agilent GC Headspace sampler 7697 A, injection temperature: 160 °C, injection volume: 80 μL; detection temperature: 300 °C; data rate: 20 Hz; column: Rxi-624 Sil MS 20 m × 0.18 mm × 1 μm, column flow: 1.2 mL/min, split flow: 21.6 mL/min, split ratio: 18; liner: transfer-liner Agilent part no 18740-80200; analysis method: 40 °C, initial time 4.5 min, heat ramp 14 °C/min to 70 °C, heat ramp 90 °C/min to 220 °C, hold time 1.69 min; carries gas: hydrogen.

*Ethanol content (method b)*

Headspace gas chromatography based on USP <467>.

**Water content determination**

*Water content (method a):*

Instrument: 870 KF-Titrino Plus, Metrohm AG, Herisau, Switzerland; solvent: Hydranal® solvent for the volumetric determination of water content according to Karl-Fischer; titrant: Hydranal®-titrant 5.

*Water content (method b):*

According to Pharm. Eur. 2.5.32 (Karl-Fischer-method; coulometric)

**Example 1 - Synthesis of the Compound of Formula (II)**

Synthesis of Gadolinium 2,2',2"-[10-(1-{2-(4-nitrophenoxy)-2-oxoethyl)amino}-1-oxopropan-2-yl)-1,4,7,10-tetraazacyclododecane-1,4,7-triyl]triacetate

**Reaction Scheme**

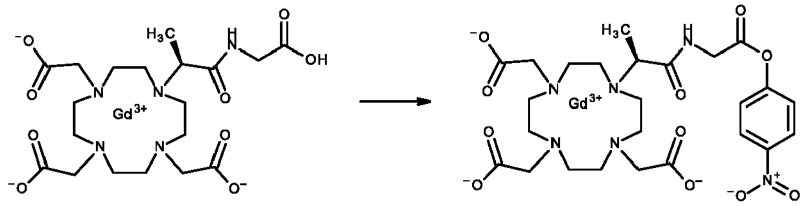

(IV) (II)

A 36 L reactor was charged with 4-Nitrophenol (0.994 kg, CAS: 100-02-7, purchased from Amarjyot Chemical Corporation, India) and acetonitrile (11.0 kg) was added. The mixture was stirred for 15 min at 25 °C (jacket temperature) to obtain a clear solution and Gadolinium 10-[4-carboxy-1-methyl-2-oxo-3-azabutyl]-1,4,7,10-tetraazacyclododecan-1,4,7-triacetic acid (2.25 kg, CAS: 208252-78-2, see DE 19652386, Schering AG, supplied as dihydrate, 5.6 wt-% water) was added as solid. The source container and the addition funnel were rinsed with acetonitrile (1.29 kg). The mixture was stirred for an additional 15 min at 25 °C (jacket temperature) and subsequently cooled to 0 °C (internal temperature). Formamide (4.21 kg) was added, the piping was rinsed with acetonitrile (0.890 kg) and the mixture was stirred for 3 h at this temperature. Then, a solution of N,N-diisopropylcarbodiimide (0.676 kg, CAS: 693-13-0, purchased from Kemilabs, Hungary) in acetonitrile (0.670 kg) was added within 8 min. The source container and the piping were rinsed with acetonitrile (0.630 kg). The mixture was warmed to 23 °C (internal temperature) and subsequently stirred for 19 h. A solution of acetonitrile (4.56 kg) and water (0.810 kg) was added and the mixture was stirred for 3 h. The mixture was filtered and the product was washed with acetonitrile (three times, 3.51 kg, 3.99 kg, 3.51 kg). The solid was dried under reduced pressure and elevated temperature to yield the title compound (2.33 kg, 87%) as a colorless to slightly yellow crystalline solid.

**Analytics**

| | |
|---|---|
| HPLC (method a), $R_t$: | 4.23 min. |
| HPLC (method b), $R_t$: | 4.52 min. |
| Assay (method b): | 78.3 wt-% |

Mass spectrometry:

| Compound | MW [Da] | RT [min] | m/z detected | Adduct | Deviation [ppm] |
|---|---|---|---|---|---|
| $C_{25}H_{33}N_6O_{11}Gd$ (the compound of formula (II)) | 751 | 1.74 | 750.1379 | [M-H]- | 1 |

Formamide (method b): 11.9 wt-%
Water (method b): 2.2 wt-%
Solid state form: Form I

The following examples have been carried out similarly as described above:

| Example | compound of the formula (IV) [kg] | Yield [%] | Assay [wt-%, method b or c] | Formamide content [wt-%, method a (GC) or b-2] | Solid state form [all crystalline] |
|---|---|---|---|---|---|
| 1.1**, $ | 28.3 | 77 | 69.5 [b] | 8.9 [b-2] | Form III |
| 1-2** | 28.3 | 78 | 66.6 [b] | 8.8 [b-2] | Form III |
| 1-3** | 28.3 | 81 | 65.7 [b] | 7.7 [b-2] | Form III |
| 1.4 | 2.25 | 65 | 73.1 [b] | 5.6 [a] | Form II |
| 1.5 | 2.25 | 86 | 75.9 [b] | 11.5 [a] | Form I |
| 1.6 | 2.25 | 86 | 77.7 [b] | 10.4 [a] / 12.0 [b-2] | Form I |
| 1.7 | 2.25 | 86 | 76.5 [b] | 12.6 [a] | Form I |
| 1.8 | 2.25 | 87 | 77.3 [b] | 10.2 [a] | Form I |
| 1.9 | 2.25 | 88 | 79.6 [b] | 11.8 [a] | Form I |
| 1.10*** | 15.0 | 84 | 80.3 [c] | 11.7 [b-2] | Form I |
| 1.11***/**** | 15.0 | 86 | 79.4 [c] | 12.0 [b-2] | Form I |
| 1. 12***/**** | 440 | 76§ | 83.2 [c] | 12.0 [b-2] | Form I |
| 1.13***/**** | 440 | 89 | 83.9 [c] | 11.5[b-2] | Form I |
| 1.14 | 0.130 | 88 | 82.5 [c] | 10.9 [a] | Form I |

** Different order of addition: The reactor was charged with 4-Nitrophenol and Gadolinium 10-[4-carboxy-1-methy-2-oxo-3-azabutyl]-1,4,7,10-tetraazacyclododecan-1,4,7-triacetic acid; then acetonitrile has been added to obtain a suspension which was carried forward as described above.

*** The reaction was seeded with 1 wt-% Gadolinium 2,2',2"-[10-(1-{2-(4-nitrophenoxy)-2-oxoethyl)amino}-1-oxopropan-2-yl)-1,4,7,10-tetraazacyclododecane-1,4,7-triyl]triacetate (Form I) upon addition of formamide.

****Instead of adding a solution of acetonitrile and water, the two components were added sequentially. Water was dosed within 45 to 60 mins.

§ Actual yield higher. Due to production line hold-up, not all material could be isolated.

$ Water content of the mixture upon addition of all reagents, solvents and water: 3.2 wt-%

**Example 2 - Synthesis of the compound of formula (I) crude**

[4,10-bis(carboxylatomethyl)-7-{3,6,12,15-tetraoxo-16-[4,7,10-tris-(carboxylatomethyl)-1,4,7,10-tetraazacyclododecan-1-yl]-9,9-bis({[({2-[4,7,10-tris-(carboxylatomethyl)-1,4,7,10-tetraazacyclododecan-1-yl]propanoyl}amino)acetyl]-amino}methyl)-4,7,11,14-tetraazaheptadecan-2-yl}-1,4,7,10-tetraazacyclododecan-1-yl]acetate

## Reaction Scheme:

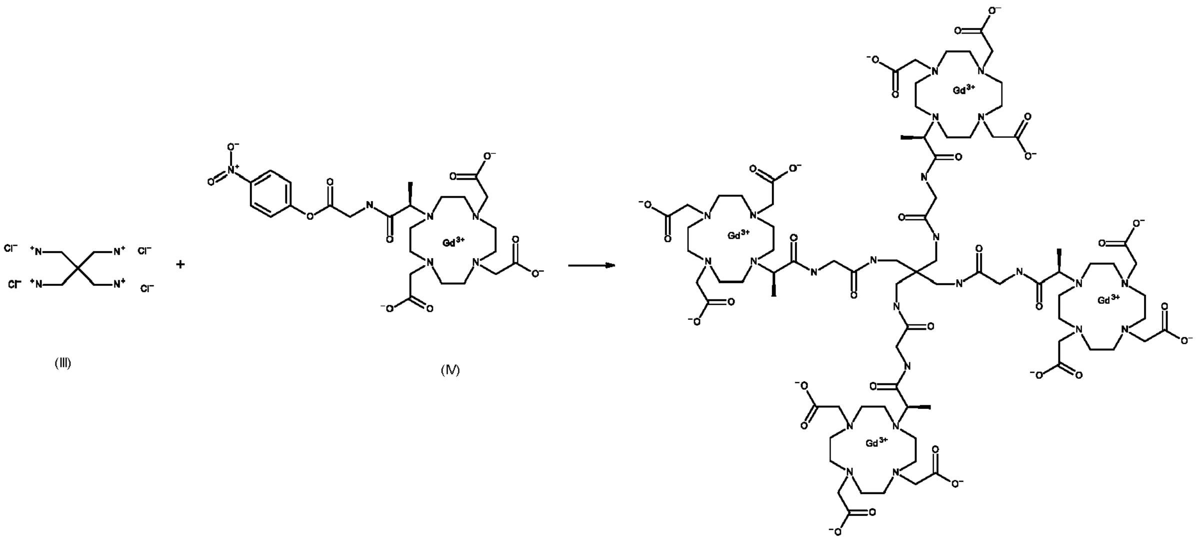

(III)

(IV)

(I)

A 26 L reactor was charged with dimehtylsulfoxide (7.26 kg). 2,2-bis(aminomethyl)propane-1,3-diamine tetrahydrochloride (0.148 kg, see W. Hayes et al., Tetrahedron 2003, 59, 7983) was added and the mixture was stirred for 15 min. Gadolinium 2,2',2"-[10-(1-{2-(4-nitrophenoxy)-2-oxoethyl)amino}-1-oxopropan-2-yl)-1,4,7,10-tetraazacyclododecane-1,4,7-triyl]triacetate (2.20 kg | 77 wt-%) was added and the mixture was stirred for 30 min. Subsequently, N,N-diisopropylethylamine (0.303 kg) was added. The mixture was heated to 50 °C (internal temperature) and stirred for 4 h. The reaction was cooled to 23 °C and water (6.6 kg) was slowly added maintaining an internal temperature below 40 °C. The pH was adjusted to 12.5 by slow addition of aqueous sodium hydroxide (7.38 wt-%, 3.63 kg) and the mixture was stirred for 1 h at 20 °C. The pH was adjusted to 6.5 - 7.5 by slow addition of aqueous hydrochloric acid (10 wt-%, 2.1 kg). The mixture was consecutively washed with methyl-tert-butyl ether (2 × 4.88 kg) and the organic layer of each wash was discarded. The aqueous layer was filtered and subsequently concentrated under reduced pressure (50 mbar) until an internal temperature of ~58 °C was reached. The water content was determined by volumetric Karl-Fischer titration to be ~16 wt-%. The mixture was cooled to room temperature and ethanol (containing ~1 % methylethylketone, 5.5 kg) was added. Subsequently, acetone (11 kg) was added within 2 h. The resulting suspension was stirred for 30 min and filtered. The resulting solid was washed with acetone (two times, 2.75 kg each) and subsequently dried under reduced pressure and elevated temperature. The title compound was obtained as a colorless solid (2.13 kg, 155% based on employed 2,2-bis(aminomethyl)propane-1,3-diamine tetrahydrochloride, 94% assay corrected).

Typically, such material has an assay of 50 - 80%. Apart from the major impurity the compound of the formula (IV) originating from the compound of the formula (II) and other organic impurities, the material also contains inorganic salts such as sodium chloride, residual solvents such as dimethylsulfoxide, formamide and water.

Synthesis of the compound of the formula (I) based upon the presented synthesis route results in a mixture of three isomer types: (RRRR/SSSS), (RRRS/SSSR), (RRSS) the selective isolation of which are described in examples 5, 6 and 7. Given the reaction mechanism a statistical distribution of isomers would be expected yielding a ratio of ~1:5:4 in terms of (RRRR/SSSS):(RRRS/SSSR):(RRSS) that is approximately observed in reaction mixtures as outlined for this batch.

**Analytics:**

| | | | |
|---|---|---|---|
| Purity (area-%, method d-1): | 89.2% | | |
| Assay HPLC (method d-1): | 60.6% | | |
| Diastereomer Distribution: | Diastereomer 1 ($R_t$: 20.3 min): | 40.5% | |
| | Diastereomer 2 ($R_t$: 20.9 min): | 50.2% | |
| | Diastereomer 3 ($R_t$: 22.4 min): | 9.3 % | |
| Water (KF-Fischer, volumetric, method a): | 4 wt-% | | |

The following examples have been carried out similarly as described above; the ratio of the compound of the formula (IV) / the compound of the formula (III) is outlined below.

| Example | compound of the formula (II) [kg] \| Assay [wt-%] | compound of the formula (III) [kg] | Yield [kg \| % based the compound of the formula (III)] | Assay [wt-% , method d-1] | Yield corrected based on the compound of the formula (III) [%] | HPLC Purity [Area-%, method d-1] |
|---|---|---|---|---|---|---|
| 2.1 | 3.00 \| 68 | 0.186 | 2.38 \| 139 | 55.8 [d-1] | 84 | 75.7 [d-1] |
| 2.2* | 25.51 70 | 1.45 | 21.4 \| 158 | 50.7 [d-1] | 80 | 70.3 [d-1] |
| 2.3* | 25.5 \| 67 | 1.45 | 23.5 \| 174 | 51.2 [d-1] | 89 | 69.2 [d-1] |
| 2.4* | 25.5 \| 66 | 1.45 | 23.7 \| 176 | 50.8 [d-1] | 89 | 70.3 [d-1] |
| 2.5 | 2.201 76 | 0.148 | 2.03 \| 148 | 67.1 [d-1] | 99 | 88.8 [d-1] |
| 2.6 | 2.201 77 | 0.148 | 2.06 \| 150 | 61.1 [d-1] | 95 | 89.7 [d-1] |
| 2.7 | 1.00 \| 73 1.20 \| 78 | 0.148 | 1.92 \| 141 | 62.9 [d-1] | 88 | 87.5 [d-1] |
| 2.8 | 2.20 \| 77 | 0.155 | 2.17 \| 150 | 64.0 [d-1] | 96 | 92.8 [d-1] |
| 2.9** | 0.025 \| 78 | 1.68 $10^{-3}$ | 15*$10^{-3}$ \| 96 | 80.5 [d-1] | 78 | 91.9 [d-1] |
| 2.10*** | 0.025 \| 77 | 1.68 $10^{-3}$ | 19.3*$10^{-3}$\| 124 | 72.3 [d-1] | 89 | 89.3 [d-1] |
| 2.11$ | 0.025 \| 77 | 1.68 $10^{-3}$ | 18.5*$10^{-3}$ \| 119 | 74.7 [d-1] | 88 | 89.1 [d-1] |
| 2.12 | 0.0251 83 | 1.85 $10^{-3}$ | 20.7*$10^{-3}$\| 121 | 70.7 [d-1] | 85 | 95.3 [d-1] |
| 2.13$$ | 0.025 \| 77 | 1.68 $10^{-3}$ | 21.9*$10^{-3}$\| 140 | 65.9 [d-1] | 92 | 89.1 [d-1] |
| 2.14$$$ | 0.0251 77 | 1.68 $10^{-3}$ | 20.6*$10^{-3}$\| 132 | 66.9 [d-1] | 88 | 87.8 [d-1] |
| 2.15$$$$ | 0.025 \| 77 | 1.68 $10^{-3}$ | 22.2*$10^{-3}$\| 142 | 66.0 [d-1] | 94 | 87.7 [d-1] |

(continued)

| Example | compound of the formula (II) [kg] \| Assay [wt-%] | compound of the formula (III) [kg] | Yield [kg \| % based the compound of the formula (III)] | Assay [wt-% , method d-1] | Yield corrected based on the compound of the formula (III) [%] | HPLC Purity [Area-%, method d-1] |
|---|---|---|---|---|---|---|
| 2.16[$$$$$] | 0.050 \| 81 | 3.44 $10^{-3}$ | 38.6*$10^{-3}$\| 121 | 74.9 [d-1] | 91 | 89.5 [d-1] |
| 2.17[§] | 400 \| 83 | 27.5 | 372 \| 146 | 62.8 [d-1] | 92 | 89.8 [d-1] |
| 2.18 | 400 \| 81 | 27.5 | 391 \| 148 | 63.6 [d-1] | 99 | 90.9 [d-1] |

* The manufacture was differing in the sense that the reactor was charged with the solids the compound of the formula (III) and the compound of the formula (II) prior to addition of dimethylsulfoxide.

** The reaction was carried out similarly as described above, but no treatment with aqueous sodium hydroxide and aqueous hydrochloric acid took place.

*** The reaction was carried out similarly as described above, but basic treatment upon addition of aqueous sodium hydroxide was carried out at 50 °C and pH = 8.5

[$] The reaction was carried out similarly as described above, but basic treatment upon addition of aqueous sodium hydroxide was carried out at 50 °C and pH = 9.5

[$$]The reaction was carried out similarly as described above, but triethylamine was employed instead of diisopropylethylamine

[$$$] The reaction was carried out similarly as described above, but tributylamine was employed instead of diisopropylethylamine

[$$$$] The reaction was carried out similarly as described above, but no washings with tert-butyl methyl ether took place.

[$$$$$] The reaction was carried out similarly as described above, but basic treatment with upon addition of aqueous sodium hydroxide was carried out at 50 °C and pH = 9; no washings with tert-butyl methyl ether took place.

[§] Distillation was carried out at 75 mbar until reaching an internal temperature of 66 °C. The water content was determined to be 16.9 wt-%.

**Example 3: Synthesis of the compound of the formula (I) | aqueous solution: Synthesis of [4,10-bis(carboxylatomethyl)-7-{3,6,12,15-tetraoxo-16-[4,7,10-tris-(carboxylatomethyl)-1,4,7,10-tetraazacyclododecan-1-yl]-9,9-bis({[({2-[4,7,10-tris-(carboxylatomethyl)-1,4,7,10-tetraazacyclododecan-1-yl]propanoyl}amino)acetyl]-amino}methyl)-4,7,11,14-tetraazaheptadecan-2-yl}-1,4,7,10-tetraazacyclododecan-1-yl]acetate | aqueous solution**

## Reaction Scheme:

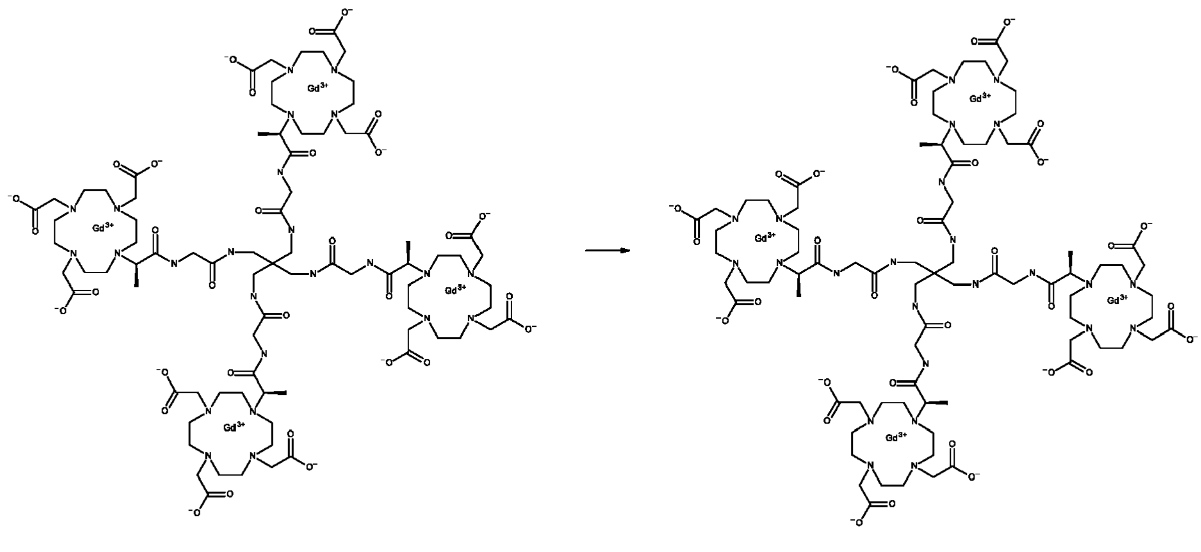

(I) (I)

The compound of the formula (I) crude (32.5 kg, ~51 wt-% assay, 16.5 kg the compound of the formula (I)) was dissolved in water (190 kg) in a total of four portions. Each portion was filtered and stored in a container. Overall, the reactor and the piping were washed with water (14 kg). The mixture was transferred to a second reactor via a filter and the containers and the piping was rinsed with water (21 kg). The solution was diafiltrated using a 2 kDa Membrane (12 $m^2$ surface, Hydrosart®, purchased from Satorius AG, Germany) exchanging the water volume of the retentate overall seven times and maintaining a constant mass in the retentate vessel. After completion, the mass of the retentate was concentrated by ultrafiltration, the mixture was stored in containers and the system was rinsed with water. Overall, 127.9 kg solution with an assay of ~12.2 wt-% of the compound of the formula (I) was obtained (~95% yield).

**Analytics:**

| | | |
|---|---|---|
| Purity (area-%, method d-1): | 92.5% | |
| Diastereomer content (area-%, method d-1): | | |
| | Diastereomer 1 ($R_t$: 20.7 min): | 36.5 area-% |
| | Diastereomer 2 ($R_t$: 21.4 min): | 46.0 area-% |
| | Diastereomer 3 ($R_t$: 23.1 min): | 9.0 area-% |

The following examples have been carried out similarly as described above; the membrane size was adjusted to the scale.

| Example | Scale compound of the formula (I) crude [kg] | Purity the compound of the formula (I) crude or starting solution in water [Area-%] [method d-1 or d-2 | Yield [assa y correc ted, %] | HPLC Purity the compound of the formula (I) upon completion of UF/DF [Area-% / wt-%, \|method d-1 or d-2] |
|---|---|---|---|---|
| 3.1 | 9.4 (~15 wt-% in water,) 2.83 2.10 | 64 66 68 [d-2] | 88 | 95.4 / 17.6 [d-2] |
| 3.2 | 32.5 | 77 [d-1] | 95 | 91.8 / 13.1 [d-1] |
| 3.3 | 0.118 | 89 [d-1] | 88 | 96.8/14.6 [d-1] |
| 3.4** | 0.123 | 89 [d-1] | 99 | 97.0/16.8 [d-1] |
| 3.5** | 0.165 | 89 [d-1] | 99 | 97.5/15.0 [d-1] |

(continued)

| Example | Scale compound of the formula (I) crude [kg] | Purity the compound of the formula (I) crude or starting solution in water [Area-%] [method d-1 or d-2 | Yield [assa y correc ted, %] | HPLC Purity the compound of the formula (I) upon completion of UF/DF [Area-% / wt-%, \|method d-1 or d-2] |
|---|---|---|---|---|
| 3.6** | 0.159 | 87 [d-1] | 98 | 96.3 / 15.4 [d-1] |
| 3.7*** | 5.1 | 76 [d-1] | 93 | 95.9 / 15.4 [d-1] |
| 3.8***\|$ | 4.4 | 90 [d-1] | 93 | 98.8 / 16.0 [d-1] |
| 3.9$\|$$ | 4.9 | 86 [d-1] | 93 | 98.7 [d-1] |
| 3.10$$$ | 0.089 | 86 [d-1] | 91 | 95.4 / 12.2 [d-1] |
| 3.11$\|$$ | 500 | 87 [d-1] | >95§ | 97.3 / 14.4 [d-1] [d-1] |
| 3.12$\|$$ | 500 | 91 [d-1] | >95§ | 97.8/14.4 [d-1] |

** Due to the high purity of the compound of the formula (I) crude, only four cycles of diafiltration were carried out before concentrating the aqueous solution.
*** The UF/DF was carried out at 40 °C.
$Due to the high purity of the compound of the formula (I) crude, only fives cycles of diafiltration were carried out before concentrating the aqueous solution.
$$The UF/DF was carried out at 35 °C.
$$$ Only three cycles of diafiltration were carried out
§ The aqueous solution was collected in a vessel equipped with fill level sensor and not with a balance. The solution was processed further. In addition, hold-up remains in the pipes and vessels. Thus, the yield is given as >95%.

**Example 4. Synthesis of the compound of the formula (I)**

[4,10-bis(carboxylatomethyl)-7-{3,6,12,15-tetraoxo-16-[4,7,10-tris-(carboxylatomethyl)-1,4,7,10-tetraazacyclododecan-1-yl]-9,9-bis({[({2-[4,7,10-tris-(carboxylatomethyl)-1,4,7,10-tetraazacyclododecan-1-yl]propanoyl}amino)acetyl]-amino}methyl)-4,7,11,14-tetraazaheptadecan-2-yl}-1,4,7,10-tetraazacyclododecan-1-yl]acetate

## Reaction Scheme:

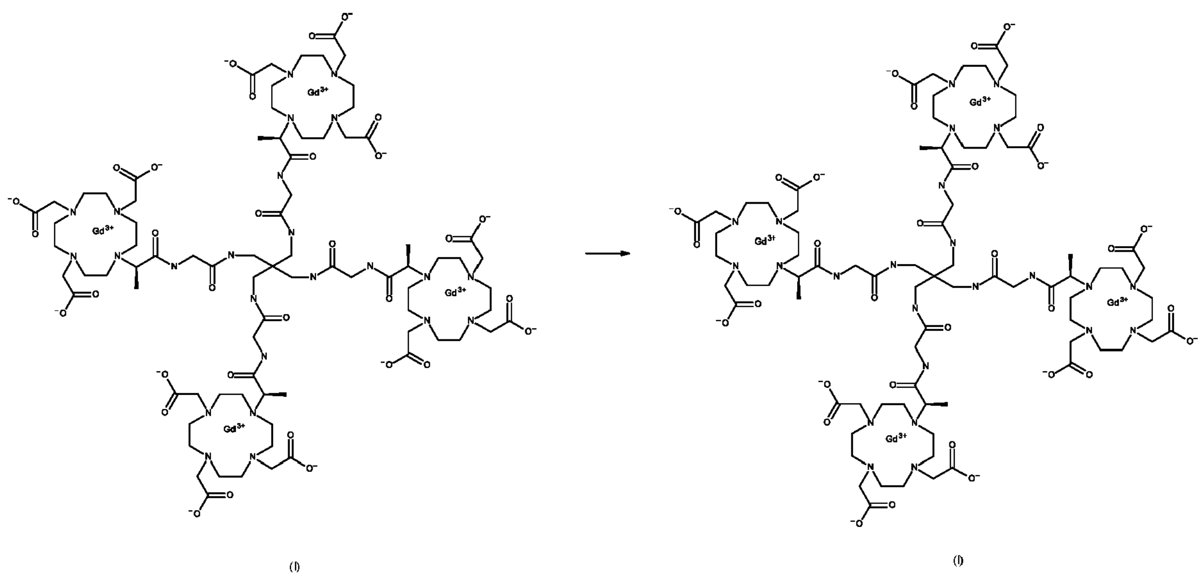

A solution of the compound of the formula (I) in water (128 kg, Assay: ~12.3 wt-%, ~15.7 kg compound of the formula (I)) in containers was transferred to a 250 L reactor and the containers as well as the piping was rinsed with water (overall 10.0 kg). The resulting solution was concentrated under reduced pressure (50 - 70 mbar) at elevated temperature (jacket temperature: 75 °C) to give a solution of ~20 wt-% the compound of the formula (I) in water. The solution was transferred to a second reactor and the source reactor and the piping was rinsed with water (5.2 kg). Activated charcoal (0.830 kg, type Norit A Supra DAB) was added and the mixture was heated to 60 °C and stirred for 1 h. The mixture was cooled to 20 °C and subsequently filtered and transferred to a third reactor. The source reactor and the piping were washed with water (12.4 kg). The resulting aqueous solution was concentrated under reduced pressure (50 - 70 mbar) and elevated temperature (jacket temperature: 75 °C) to give an ~50 wt-% solution of the compound of the formula (I) in water. The mixture was warmed to 60 °C and ethanol (containing -1% methylethylketone, 78.5 kg) was added within 2 h. The water content of the mixture was determined by volumetric Karl-Fischer titration to be -13.5 wt-%. The resulting suspension was stirred for an additional 2 h at this temperature and subsequently cooled to 20 °C within 90 min. The mixture was stirred for 15 min, filtered and washed with ethanol (containing ~1% methylethylketone, two times, 29.9 kg, 16.1 kg). The resulting solid was dried in an agitated Nutsch dryer at elevated temperature and reduced pressure. Such material was transferred to an oven containing two trays filled with water. The material was equilibrated for 20 h at reduced pressure (30 mbar) and slightly elevated temperature (36 °C, equaling a relative humidity of -50%) to yield the title compound (14.7 kg, 94%, 83% assay corrected) as a colorless crystalline solid.

**Analytics:**

| | | | |
|---|---|---|---|
| HPLC purity (method d-1, area-%): | 99.8% | | |
| HPLC (method d-1): | | | |
| Diastereomer Distribution: | | Diastereomer 1 ($R_t$: 20.1 min): | 46.4% |
| | | Diastereomer 2 ($R_t$: 20.7 min): | 52.6% |
| | | Diastereomer 3 ($R_t$: 22.3 min): | 1.0% |
| Assay: | 86.9 wt-%. | | |

Mass spectrometry [LC-MS, modified method d-1 using formic acid instead of phosphate buffer]:
The detected masses comply with the sum formula of the compound of the formula (I) diastereomers

| Compound | MW [Da] | RRT | m/z detected | Adduct | Deviation [ppm] |
|---|---|---|---|---|---|
| the compound of the formula (I), Diastereomer 1 Sum formula: $C_{81}H_{128}N_{24}O_{32}Gd_4$ | 2579 | 1.00 | 861.2130 | $[M+3H]^{3+}$ | 3 |
| the compound of the formula (I), Diastereomer 2 Sum formula: $C_{81}H_{128}N_{24}O_{32}Gd_4$ | 2579 | 1.03 | 861.2128 | $[M+3H]^{3+}$ | 3 |
| the compound of the formula (I), Diastereomer 3 Sum formula: $C_{81}H_{128}N_{24}O_{32}Gd_4$ | 2579 | 1.12 | 861.2112 | $[M+3H]^{3+}$ | 4 |

| | |
|---|---|
| Water (method b): | 12.5 wt-% |
| Ethanol (method b): | <0.050 wt-% |
| Solid state: | Mixture; Form II and Form I |

**Example 4.1: Selective manufacture of Form II**

A solution of the compound of the formula (I) in water (8.6 kg, ~15.7 wt-%, ~1.35 kg compound of the formula (I)) in containers was transferred to a 26 L reactor and the piping was rinsed with a solution of compound of the formula (I) in Water (1.05 kg, ~2.6 wt-%, ~0.027 kg compound of the formula (I)). Activated charcoal (0.059 kg, type Norit SX Plus 20, was suspended in a solution of the compound of the formula (I) in water (1.5 kg, ~2.6 wt-%, ~0.039 kg compound of the formula (I)) and added to the mixture. The mixture was heated to 60 °C and stirred for 1 h. The mixture was cooled

to 22 °C and subsequently filtered. The source reactor and the filter were washed with water (1.35 kg). The resulting solution (11.9 kg) was transferred to a 50 L reactor and subsequently concentrated under reduced pressure (75 mbar) and elevated temperature (jacket temperature: 80 °C) to give an ~50 wt-% solution of the compound of the formula (I) in water (collected distillate: 9.68 kg). The mixture was warmed to 60 °C and ethanol (containing -1% methylethylketone, 6.8 kg) was added within 2 h. The water content of the mixture was determined by volumetric Karl-Fischer titration to be ~8-9 wt-%. The resulting suspension was stirred for an additional 2 h at this temperature and subsequently cooled to 25 °C within 45 min. The mixture was stirred for 30 min, filtered, and washed with ethanol (containing ~1% methylethylketone, two times, 1.35 kg each). The resulting solid was dried in an agitated Nutsch dryer at elevated temperature (initial jacket temperature: 80 °C) and reduced pressure (pump pressure: 20 mbar). Upon reaching a pressure of 27 mbar (internal pressure in the dryer) and a product temperature of 47.5 °C, water steam was applied.§ The material was dried at approx. 25 - 29 mbar (pressure in the dryer) and a product temperature of 64 - 68 °C (equaling a r.H. of ~9 - 12%) for 3 h. Drying in the presence of water steam was continued at 25 - 29 mbar (pressure in the dryer). The jacket temperature was reduced to 30 °C and the product temperature gradually declined to a final product temperature of 30 °C (equaling a r.H. of -66%) within 4.5 h to yield the title compound (1.2 kg, 89%, 78% assay corrected) as a colorless crystalline solid.

§The water steam was generated in a pressure reactor at 130 °C jacket temperature | 1200-1500 mbar,abs. The hot water steam passed into the nutsch dryer using a heated hose. Flow rate controlled by a valve.

Analytics:

| | | |
|---|---|---|
| HPLC purity (method d-1, area-%): | 99.9% | |
| HPLC (method d-1): | | |
| Diastereomer Distribution: | Diastereomer 1 ($R_t$: 20.1 min): | 46.7% |
| | Diastereomer 2 ($R_t$: 20.7 min): | 52.3% |
| | Diastereomer 3 ($R_t$: 22.3 min): | 1.1% |
| Assay: | 87.2 wt-%. | |
| Water (method b): | 13.2 wt-% | |
| Ethanol (method b): | n.d. | |
| Solid state: | Form II | |

**Example 4.2: Selective manufacture of Form I**

A solution of the compound of the formula (I) in water (15.2 kg, ~16.0 wt-%, ~2.43 kg compound of the formula (I)) in containers was transferred to a 26 L reactor and the piping was rinsed with a solution of the compound of the formula (I) in water (3.05 kg, ~4.4 wt-%, ~0.134 kg compound of the formula (I)). Activated charcoal (0.106 kg, type Norit A Supra DAB) was suspended in a solution of the compound of the formula (I) in water (1.6 kg, ~4.4 wt-%, ~0.070 kg compound of the formula (I)) and added to the mixture. The mixture was heated to 60 °C and stirred for 1 h. The mixture was cooled to 22 °C and subsequently filtered. The source reactor and the filter were washed with water (1.2 kg). The resulting solution (20.8 kg) was transferred to a 50 L reactor and subsequently concentrated under reduced pressure (75 mbar) and elevated temperature (jacket temperature: 80 °C) to give an ~50 wt-% solution of the compound of the formula (I) in water (collected distillate: ~16 kg). The mixture was warmed to 60 °C and ethanol (containing ~1% methylethylketone, 12 kg) was added within 2 h. The water content of the mixture was determined by volumetric Karl-Fischer titration to be ~15 wt-%. The resulting suspension was stirred for an additional 2 h at this temperature and subsequently cooled to 25 °C within 45 min. The mixture was stirred for 30 min, filtered, and washed with ethanol (containing ~1% methylethylketone, two times, 2.4 kg each).The wet material was dried in an agitated Nutsch dryer in the presence of water steam§ at 29 - 32 mbar (pressure in the dryer) and a product temperature of 31 - 38 °C (equaling a r.H. of ~44 - 69%) for 24 h with a final product temperature of 31 °C at 29 mbar (pressure in the dryer, equaling a r.H. of ~65%) to yield the title compound (2.28 kg, 87%, 71% assay corrected)§§ as a colorless crystalline solid.

§The water steam was generated in a pressure reactor at 130 °C jacket temperature | 1200-1500 mbar,abs. The hot water steam passed into the nutsch dryer using a heated hose. Flow rate controlled by a valve.
§§ Actual yield was higher, but the agitated nutsch dryer could not be fully emptied. Remainder material was dissolved in water.

Analytics:

| | | | |
|---|---|---|---|
| HPLC purity (method d-1, area-%): | >99% | | |
| HPLC (method d-1): | | | |
| Diastereomer Distribution: | Diastereomer 1 ($R_t$: 20.1 min): | 48.7% | |
| | Diastereomer 2 ($R_t$: 20.7 min): | 50.4% | |
| | Diastereomer 3 ($R_t$: 22.3 min): | 0.9% | |
| Assay: | 82.4 wt-% | | |
| Water (KF-Fischer, method b): | 17.2 wt-% | | |
| Ethanol (method b): | 0.222 wt-% | | |
| Solid state: | Form I | | |

The following examples have been carried out similarly as the examples described above. The drying | equilibration conditions partially differ to the ones as described above.

<table>
<tr><th>Example</th><th>Scale of the compound of the formula (I) [kg, based on wt-% in water]</th><th>Yield [%] | Yield [assay corrected, %]</th><th>HPLC Purity [Area-% / wt-%, | method d-1 or d-2]</th><th>Diastereomer content [area-%, diastereomer 1 | diastereomer 2 | diastereomer 3]</th><th>Water content [wt-%, method a | method b]</th><th>ethanol content [wt-%] | method a or method b</th><th>Solid state</th></tr>
<tr><td>4.3*</td><td>4.05</td><td>70 | 67</td><td>99.8 / 95.6 [d-2]</td><td>46.5 | 52.3 | 1.2 [d-2]</td><td>2.7 [b]</td><td>0.122 [b]</td><td>Form II</td></tr>
<tr><td>4.4</td><td>15.7</td><td>831 76</td><td>99.8 / 87.0 [d-1]</td><td>46.0 | 52.9 | 1.1 [d-1]</td><td>12.6 [b]</td><td>n.d. [b]</td><td>Mixture; Form I and Form II</td></tr>
<tr><td>4.5[$$$]</td><td>0.100</td><td>73** | 65</td><td>>99.9 / 88.2 [d-1]</td><td>49.2 | 50.2 |0.6 [d-1]</td><td>12.5 [a]</td><td>0.003 [a]</td><td>Mixture; Form I and Form II</td></tr>
<tr><td>4.6***</td><td>0.123</td><td>81 | assay not determined</td><td>>99.9 / 78.7 [d-1]</td><td>52.2 | 47.1 | 0.6 [d-1]</td><td>19.4 [b]</td><td>n.d. [b]</td><td>Form I</td></tr>
<tr><td>4.7****</td><td>0.100</td><td>93**| 82</td><td>99.8 / 88.4 [d-1]</td><td>48.0 | 51.3 | 10.6 [d-1]</td><td>12.1 [a]</td><td>0.003 [a]</td><td>Form II</td></tr>
<tr><td>4.8***</td><td>0.115</td><td>82** | 66</td><td>99.8 / 80.3 [d-1]</td><td>50.2 | 48.9 | 0.7 [d-1]</td><td>20.0 [a]</td><td>n.d. [a]</td><td>Form I</td></tr>
<tr><td>4.9[$,§]</td><td>2.56</td><td>84[$$] | 75</td><td>99.8 / 86.7 [d-1]</td><td>48.1 | 50.8 | 1.1 [d-1]</td><td>12.6 [b]</td><td><0.050 [b]</td><td>Form II</td></tr>
<tr><td>4.10[$, §§]</td><td>1.35</td><td>84 | 73</td><td>>99.9 / 86.8 [d-1]</td><td>48.9 | 50.3 |0.9 [d-1]</td><td>13.2 [b]</td><td><0.050 [b]</td><td>Form II</td></tr>
<tr><td>4.11 [$, §§§]</td><td>1.27</td><td>871 77</td><td>>99.9 / 88.5 [d-1]</td><td>48.8 | 50.3 | 0.8 [d-1]</td><td>11.7 [b]</td><td>n.d. [b]</td><td>Form II</td></tr>
<tr><td>4.12 [$, §§§§]</td><td>1.27</td><td>881 76</td><td>>99.9 / 86.3 [d-1]</td><td>48 | 51 | 1 [d-1]</td><td>14.3 [b]</td><td>n.d. [b]</td><td>Form II</td></tr>
</table>

(continued)

| Example | Scale of the compound of the formula (I) [kg, based on wt-% in water] | Yield [%] \| Yield [assay corrected, %] | HPLC Purity [Area-% / wt-%, \| method d-1 or d-2] | Diastereomer content [area-%, diastereomer 1 \| diastereomer 2 \| diastereomer 3] | Water content [wt-%, method a \| method b] | ethanol content [wt-%] \| method a or method b | Solid state |
|---|---|---|---|---|---|---|---|
| 4.13$$$$ | 0.025 | 91 181 | 99.9 / 88.8 [d-1] | 45.3 \| 53.6 \| 1.1 [d-1] | n/a | 0.16 [a] | - |
| 4.14$$$$$ | 0.025 | 861 78 | 99.7 / 92.0 [d-1] | 43.1 \| 54.0 \| 2.6 [d-1] | n/a | 0.84 [a] | - |
| 4.15# | 289 | 71 \| 63 | >99.9 / 89.3 [d-1] | 47.8 \| 51.5 \| 0.8 [d-1] | 9.42 [b] | 0.004 [b] | Form II |
| 4.16## | 360 | 58 \| 52 | >99.9 / 89.9 [d-1] | 60.3 \| 39.4 \| 0.4 [d-1] | 9.75 [b] | 0.003 [b] | Form II |
| 4.17### | 290 | 68 \| 61 | 100.0 / 89.3 [d-1] | 47.8 \| 51.5 \| 0.8 [d-1] | 10.18 [b] | 0.006 [b] | Form II |

**[0289]** * Crystallization and isolation procedure differed slightly: upon concentration under vacuum, the mixture was cooled to room temperature. Then ethanol was added and the mixture was heated to 60 °C resulting in crystallization of the product. The product was isolated, washed with ethanol and dried under reduced pressure. As the ethanol level was exceeding specification limits (~2 wt-% detected), the product was exposed to air for several hours resulting in absorption of water. The product was again dried under reduced pressure - however, not in the presence of water - to yield the final compound.

** Normalized yield. Not the full wet product was equilibrated

*** The wet product was directly equilibrated in the presence of water in a glass tumble dryer at 30 °C jacket temperature | ~30 °C product temperature | 20 mbar pump pressure (equaling a r.H. of ~48 %). Due to technical setup, no measurement of pressure in the dryer.

**** Drying was conducted directly in the presence of water in a glass tumble dryer. First at 70 °C jacket temperature | ~62 °C product temperature! 20 mbar pump pressure (equaling a r.H. of ~9%) for 1.75 h followed by 30 °C jacket temperature | 20 mbar pump pressure for 2.25 h (cooling) + 1.25 h (holding time) with a final product temperature of 29°C (equaling a r.H. of -50%). Due to technical setup, no measurement of pressure in the dryer.

§ Upon reaching the required parameters, drying was conducted directly in the presence of water in an agitated nutsch dryer. First at 80 °C jacket temperature | 68 - 73 °C product temperature | 20 mbar pump pressure | ~32 mbar pressure in the dryer (equaling a r.H. of-9 - 11%) for 4.5 h followed by 31 - 40 °C jacket temperature | 20 mbar pump pressure | ~30 mbar pressure in the dryer for 5 h with a final product temperature of 31 °C (equaling a r.H. of -67%).

§§ Upon reaching the required parameters, drying was conducted directly in the presence of water in an agitated nutsch dryer. First at 72 °C jacket temperature | 61 - 65 °C product temperature | 20 mbar pump pressure | 22 - 24 mbar pressure in the dryer (equaling a r.H. of 9 - 11%) for 3 h followed by 30 °C jacket temperature! 20 mbar pump pressure | ~21 mbar pressure in the dryer for 4.5 h with a final product temperature of 31 °C (equaling a r.H. of -48%).

§§§ Upon reaching the required parameters, drying was conducted directly in the presence of water in an agitated nutsch dryer. First at 63 °C jacket temperature | 55 - 57 °C product temperature | 20 mbar pump pressure | 30 - 31 mbar pressure in the dryer (equaling a r.H. of 18 - 19%) for 3 h followed by 30 °C jacket temperature! 20 mbar pump pressure | ~28 mbar pressure in the dryer for 1.5 h with a final product temperature of 38 °C (equaling a r.H. of -43%) to obtain a mixture (Form I and Form II). Drying was continued in the presence of water, first at 72 °C jacket temperature | 60 - 63 °C product temperature | 20 mbar pump pressure | 27 - 31 mbar pressure in the dryer (equaling a r.H. of 12 - 15%) for 2 h followed by 30 °C jacket temperature! 20 mbar pump pressure | ~27 mbar pressure in the dryer for 2.5 h with a final product temperature of 33 °C (equaling a r.H. of -54%) to obtain Form II.

§§§§ Upon reaching the required parameters, drying was conducted directly in the presence of water in an agitated nutsch dryer. First at 93 - 95 °C jacket temperature | 78 - 82 °C product temperature | 21 - 24 mbar pump pressure | 29 - 33 mbar pressure in the dryer (equaling a r.H. of 6 - 7%) for 3 h followed by 30 °C jacket temperature! 20 mbar pump pressure | ~27 mbar pressure in the dryer for 4 h with a final temperature of 32 °C (equaling a r.H. of -56%) to obtain a mixture (Form I and Form II). Drying was continued in the presence of water, first at 80 °C jacket

temperature | 58 - 71 °C product temperature | 20 mbar pump pressure | 30-31 mbar pressure in the dryer (equaling a r.H. of 9 - 17 %) for 3 h followed by 30 °C jacket temperature | 20 mbar pump pressure | ~28 mbar pressure in the dryer for 2.5 h with a final product temperature of 33 °C (equaling a r.H. of ~55 %) to obtain Form II.

$ Equilibration took place in an agitated nutsch dryer. The water steam was generated in a pressure reactor at 125 - 130 °C jacket temperature | 1200 - 1500 mbar,abs. The hot water steam passed into the nutsch dryer using a heated hose. Flow rate controlled by a valve.

$$ Actual yield was higher, but the agitated nutsch dryer could not be fully emptied. Remainder material was dissolved in water.

$$$ Upon reaching the required parameters, drying was conducted directly in the presence of water in a glass tumble dryer. First at 65 - 68 °C jacket temperature | 54 - 63 °C in the dryer | 20 mbar pump pressure (equaling a r.H. of ~9 - 13%) for 2 h followed by ~30 °C jacket temperature | 20 mbar pump pressure for 3 h (cooling) + 2 h (holding time) with a final product temperature of 29 °C (equaling a r.H. of -50%) to obtain a mixture (Form I and Form II). Due to technical setup, no measurement of pressure in the dryer.

$$$$ The reaction was carried out similarly as above, but crystallization was carried out by slowly adding a mixture of ethanol and iso-propanol (w/w = 4:1) within 2 h. Accordingly, the washing of the isolated wet product was carried out with a mixture of ethanol and iso-propanol (w/w = 4:1). No drying the presence of water steam was carried out. In addition to ethanol, 0.16 wt-% isopropanol was detected. The solid state form was not determined.

$$$$$ The reaction was carried out similarly as above, but crystallization was carried out by slowly adding a mixture of ethanol and iso-propanol (w/w = 3:2) within 2 h. Accordingly, the washing of the isolated wet product was carried out with a mixture of ethanol and iso-propanol (w/w = 3:2). No drying the presence of water steam was carried out. In addition to ethanol, 0.73 wt-% iso-propanol was detected. The solid state form was not determined.

# Upon full addition of Ethanol for crystallization, the water content was determined by volumetric Karl-Fischer Titration to be -14 wt-%. Upon reaching the required parameters (product temperature of 45 °C | pressure in the dryer of <40 mbar), drying was conducted in the presence of water steam (~15 kg/h) in an agitated nutsch dryer with a jacket temperature until reaching a product temperature of 64 °C at 35 mbar (equaling a r.H. of -15%) with an overall exposure to water steam for ~12 h. The jacket temperature was lowered to 38 °C and drying was continued in the presence of water steam until reaching a product temperature of 45 °C at 35 mbar (equaling a r.H of -35%) within ~7 h Throughout the process, water steam was applied in a pressure range of ~30 to 45 mbar. In regular frequency (~20 to 40 min), water steam was switched off to carry out a cleaning of the vapour filters leading for a period of ~8 min to reduced relative humidity. Actual yield was higher, but the agitated nutsch dryer could not be fully emptied. The remainder material was dissolved in water.

## Upon full addition of Ethanol for crystallization, the water content was determined by volumetric Karl-Fischer Titration to be 23 wt-%. Upon reaching the required parameters (product temperature of 45 °C | pressure in the dryer of <40 mbar), drying was conducted in the presence of water steam (~15 kg/h) in an agitated nutsch dryer with a jacket temperature until reaching a product temperature of 65 °C at 45 mbar (equaling a r.H. of -18%) with an overall exposure to water steam for ~500 min. The jacket temperature was lowered to 43 °C and drying was continued in the presence of water steam until reaching a product temperature of 50 °C at 45 mbar (equaling a r.H of -37%) within ~330 min Throughout the process, water steam was applied in a pressure range of ~35 to 55 mbar. In regular frequency (~20 to 40 min), water steam was switched off to carry out a cleaning of the vapour filters leading for a period of ~8 min to reduced relative humidity. Actual yield was higher, but the agitated nutsch dryer could not be fully emptied. The remainder material was dissolved in water.

### Upon reaching the required parameters (product temperature of 50 °C | pressure in the dryer of <50 mbar), drying was conducted in the presence of water steam (~15 kg/h) in an agitated nutsch dryer with a jacket temperature until reaching a product temperature of 65 °C at 45 mbar (equaling a r.H. of -18%) with an overall exposure to water steam for ~430 min. The jacket temperature was lowered to 43 °C and drying was continued in the presence of water steam until reaching a product temperature of 50 °C at 45 mbar (equaling a r.H of ~37%) within ~300 min Throughout the process, water steam was applied in a pressure range of ~40 to 55 mbar. In regular frequency (~20 to 40 min), water steam was switched off to carry out a cleaning of the vapour filters leading for a period of ~8 min to reduced relative humidity. Actual yield was higher, but the agitated nutsch dryer could not be fully emptied. The remainder material was dissolved in water.

The isomers of the compound of the formula (I) can be separated as outlined below to obtain material for characterization purposes:

**Example 5: Isolation of the compound of the formula (I) diastereomer 1 (RRSS)**

Examples 5.1, 5.2 and 5.3 describe enrichment of material toward the compound of the formula (I) diastereomer 1:

**Example 5.1:** The compound of the formula (I) (38 g, obtained by a crystallization as described above, HPLC purity

>99%, diastereomer 1 = ~50%, diastereomer 2 = ~ 48.5%, diastereomer 3 = 1%) was dissolved in water (38 mL). Ethanol (containing ~1% methylethylketone, 38 mL) was added and the mixture was heated at reflux. Additional ethanol (containing ~1% methylethylketone, 342 mL) was added in three portions and the mixture was refluxed for 4 h, allowed to cool to room temperature and stirred for 18 h. The resulting precipitate was filtered off and washed with ethanol (containing ~1% methylethylketone, 10 mL) to yield a wet product (50 g)

The so obtained wet product was dissolved in water (50 mL). Ethanol (containing ~1% methylethylketone, 500 mL) was added and the mixture was heated at reflux for 2 h. The resulting suspension was allowed to cool to room temperature and the precipitate was filtered off and washed with ethanol (containing ~1% methylethylketone) to yield a wet product (47 g)

The so obtained wet product was dissolved in water (50 mL). Ethanol (containing ~1% methylethylketone, 500 mL) was added and the mixture was heated at reflux for 2 h. The resulting suspension was allowed to cool to room temperature and stirred for 18 h. The precipitate was filtered off and washed with ethanol (containing ~1% methylethylketone) to yield a wet product (42 g).

HPLC purity (method e, area-%): >99 %
diastereomer 1 = ~54%
diastereomer 2 = ~46%
diastereomer 3 = not detected.

The so obtained wet product was dissolved in water (126 mL). Ethanol (containing ~1% Methylethylketone, 230 mL) was added and the mixture was heated at reflux for 2 h. Since no crystallization was observed, additional ethanol (containing ~1% methylethylketone, 230 mL) was added and the mixture was heated at reflux for 2 h. The resulting suspension was allowed to cool to room temperature and stirred for 18 h. The precipitate was filtered off and washed with ethanol (containing ~1% methylethylketone) to yield a wet product (27 g).

HPLC purity (method e, area-%): >99 %
diastereomer 1 = ~69%
diastereomer 2 = ~31%
diastereomer 3 = not detected.

The so obtained wet product was dissolved in water (126 mL). Ethanol (containing ~1% methylethylketone, 230 mL) was added and the mixture was heated at reflux for 2 h. Since no crystallization was observed, additional ethanol (containing ~1% methylethylketone, 230 mL) was added and the mixture was heated at reflux for 2 h. The resulting suspension was allowed to cool to room temperature and stirred for 18 h. The precipitate was filtered off and washed with ethanol (containing ~1% methylethylketone) to yield a wet product which was dried under reduced pressure and elevated temperature to yield the compound of the formula (I) (27 g, HODA 6004-2-9) as colorless solid.

HPLC purity (method e, Area-%): >99 %
diastereomer 1 = ~82%
diastereomer 2 = ~18%
diastereomer 3 = not detected.

**Example 5.2.** Similarly as described for example 5.1, the compound of the formula (I) (21.3 g obtained by a crystallization as described above, HPLC purity >98%, diastereomer 1 = ~49%, diastereomer 2 = ~ 48%, diastereomer 3 = ~1%) was repeatedly re-crystallized from mixtures of ethanol (containing ~1% methylethylketone) and water to yield a colorless solid (7.1 g).

HPLC purity (method e, area-%): >98 %
diastereomer 1 = ~87%
diastereomer 2 = ~11%
diastereomer 3 = not detected

**Example 5.3.** Similarly as described for example 5.1, the compound of the formula (I) (26 g wet product, obtained by a crystallization as described above, HPLC purity >99%, diastereomer 1 = ~59%, diastereomer 2 = ~ 40%, diastereomer 3 = ~1%) was repeatedly re-crystallized from mixtures of ethanol (containing ~1% methylethylketone) and water to yield a colorless solid (18.8 g).

HPLC purity (method e, area-%): >98 %

diastereomer 1 = ~77%
diastereomer 2 = ~23%
diastereomer 3 = not detected

Preparation of the title compound:

The three batches described above were combined and dissolved in water (100 mL). Ethanol (containing ~1% methylethylketone, 500 mL) was added and the mixture was heated at reflux for 4 h. The mixture was cooled to room temperature and stirred for 1 h. The suspension was filtered and the solid was washed with ethanol (containing ~1% methylethylketone, 50 mL). The residue was dried under reduced pressure and elevated temperature to yield a mixture of diastereomers of the compound of the formula (I) as colorless solid (41.6 g).

HPLC purity (method e, area-%): >99 %
diastereomer 1 = ~81%
diastereomer 2 = ~19%
diastereomer 3 = not detected.

Such material (41.5 g) was dissolved in water (120 mL). Ethanol (containing ~1% methylethylketone, 420 mL) was added and the mixture was heated at reflux for 4 h. The mixture was cooled to room temperature and stirred for 1 h. The suspension was filtered and the solid was washed with ethanol (containing ~1% methylethylketone, 50 mL). The residue was dried under reduced pressure and elevated temperature to yield a mixture of diastereomers the compound of the formula (I) (36.5 g) as colorless solid.

HPLC purity (method e, area-%): >99 %
diastereomer 1 = ~88%
diastereomer 2 = ~12%
diastereomer 3 = not detected.

Such material (36.5 g) was dissolved in water (90 mL) and filtered. The filter was washed with water (18 mL). Ethanol (containing ~1% methylethylketone, 360 mL) was added and the mixture was heated at reflux for 4 h. The mixture was cooled to room temperature and stirred for 1 h. The suspension was filtered and the solid was washed with ethanol (containing ~1% methylethylketone, 50 mL). The residue was dried under reduced pressure and elevated temperature to yield the title compound (32.6 g).

**Analytics**:

Prior to analytical characterization, the material was equilibrated by storage in a desiccator over a saturated solution of potassium carbonate for at least 48 h.

| | | |
|---|---|---|
| HPLC purity (method d-2, area-%): | >99.9% | |
| HPLC (method d-2): | | |
| Diastereomer distribution | | |
| | Diastereomer D1: | 94.1% |
| | Diastereomer D2: | 5.9% |
| | Diastereomer D3: | n.d. |
| Water content (method b): | 11.2 wt-% | |
| Residual Ethanol: (method b) | n.d. | |
| Relaxivity R1 [in water]: | 10.3 [L / (mmol*sec)] | |
| Relaxivity R2 [in water]: | 11.7 [L / (mmol*sec)] | |
| Solubility data in water: | >=593 mg/mL | |
| Solid state: | Form II | |

The stereochemical configuration and molecular structure was further verified by single crystal x-ray diffraction. For details see section Instrumentation and Methods and Table 4/Figure 16.

**Example 6. Isolation of the compound of the formula (I) diastereomer 2 (RRRS and SSSR)**

Since diastereomers 2 and 3 of the compound of the formula (I) are better soluble than diastereomer 1 of the compound of the formula (I) in the crystallization media water and ethanol, several mother liquors from crystallization experiments carried out similarly as described above in example 5 to enrich diastereomer 1 of the compound of the formula (I) were combined and concentrated to dryness under reduced pressure and elevated temperatures.

A mixture of isomers the compound of the formula (I) (69.4 g) was dissolved in water (150 mL). Ethanol (containing ~1% methylethylketone, 250 mL) was added and the mixture was heated at reflux. Additional ethanol (containing ~1% methylethylketone, 250 mL) was added. Subsequently, 100 mL of solvent was distilled off and the mixture was stirred at reflux for 2 h. The mixture was cooled to room temperature and stirred for 1 h. The suspension was filtered and the solid was washed with ethanol (containing ~1% methylethylketone, 70 mL). The residue was dried under reduced pressure and elevated temperature to yield a mixture of isomers the compound of the formula (I) (32.5 g) as colorless solid.

HPLC purity (method e, area-%): >99 %
diastereomer 1 = ~25%
diastereomer 2 = ~75%
diastereomer 3 = not detected.

The mother liquor was concentrated to yield a residue (36.7 g).

HPLC purity (method e, area-%): >99 %
diastereomer 1 = not detected
diastereomer 2 = ~92%
diastereomer 3 = ~8%.

To such material (36.7 g) was added ethanol (containing ~1% methylethylketone, 50 mL) and the mixture was stirred at 60 °C for 2 h forming a white suspension. The mixture was cooled to room temperature and stirred for 1 h. The suspension was filtered and the solid was dried under reduced pressure and elevated temperature to yield a mixture of isomers of the compound of the formula (I) (25.4 g) as colorless solid.

HPLC purity (method d-2, area-%): 98.7 %
diastereomer 1 = 1.9%
diastereomer 2 = 95.3%
diastereomer 3 = 1.6%.

In order to reduce the high ethanol content of such material (~5 wt-%), it was stored at ambient atmosphere for 24 h and subsequently dried under reduced pressure and elevated temperature to yield the title compound (23.8 g).

**Analytics:**

Prior to analytical characterization, the material was equilibrated by storage in a desiccator over a saturated solution of potassium carbonate for at least 48 h.

| | | |
|---|---|---|
| HPLC purity (method d-2, area-%): | 99.4% | |
| HPLC (method d-2): | | |
| Diastereomer distribution | | |
| | Diastereomer D1: | 1.9 % |
| | Diastereomer D2: | 96.0% |
| | Diastereomer D3: | 1.6%. |
| Water content (method b): | 15.1 wt-% | |
| Residual Ethanol: (method b) | n.d. | |
| Relaxivity R1 [in water]: | 10.3 [L / (mmol*sec)] | |
| Relaxivity R2 [in water]: | 11.9 [L / (mmol*sec)] | |
| Solubility in water | >=548 mg/mL | |
| Solid state: | Form II with amorphous | content |

**Example 6.1**

This example was carried out similarly as described above to obtain the compound of the formula (I) diastereomer 2 by repeated re-crystallizations and combinations of mother liquors.

| | | |
|---|---|---|
| HPLC purity (method d-1, area-%): | 99.8 % | |
| HPLC (method d-1): | | |
| Diastereomer distribution | | |
| | Diastereomer D1: | 4.6% |
| | Diastereomer D2: | 95.2% |
| | Diastereomer D3: | 0.05% |
| Water content (method a): | 9.3 wt-% | |
| Residual ethanol: (method a) | 0.2 wt-% | |

**Example 7. Isolation of the compound of the formula (I) diastereomer 3 (RRRR and SSSS)**

The mother liquor and washing liquors of a crystallization carried out similarly as described above (carried out on 2.5 kg scale) were concentrated under reduced pressure to a highly viscous residue (~2-3 L). The residue was split into two parts and to each part was added ethanol (containing ~1% methylethylketone 500 mL). The mixtures were concentrated under reduced pressure at 60 °C to yield a syrup. Additional ethanol (containing ~1% methylethylketone 1 L) was added and the mixture was concentrated under reduced pressure yielding a mixture of crystalline material and amorphous agglomerations. The crystalline material of both trials was separated manually (~600 g) and not carried forward. The remaining amorphous agglomerations of one part described above were dissolved in water (25 mL). Ethanol (containing ~1% methylethylketone, 1 L) was added and the mixture was heated to reflux. Since no crystallization was observed, seeding material was added (5 g, wet product, purity (method e) = ~99%, diastereomer 1 = ~26%, diastereomer 2 = ~70%, diastereomer 3 = ~3%,) and the mixture was heated to reflux for 5 h distilling off a total of 250 mL solvent. The mixture was slowly cooled to room temperature and stirred for 72 h. The precipitate was filtered off and washed with ethanol (containing ~1% methylethylketone, 25 mL). The filtrate was concentrated under reduced pressure and elevated temperature. To the obtained residue was added acetone (1 L) and the resulting suspension was stirred for 1 h at room temperature. The precipitate was filtered off, washed with acetone and subsequently dried under reduced pressure and elevated temperature to yield a colorless solid (207 g).

HPLC purity (method d-2, area-%): 64.4 %
diastereomer 1 = 2.1%
diastereomer 2 = 14.8%
diastereomer 3 = 47.5 %.

Such material was dissolved in water (500 mL) and ion exchange resin (211 g, type MB 6113, purchased from Merck KGaA, Germany) was added. The source container was rinses with water (250 mL) and the mixture was stirred for 3 h at room temperature. The resin was filtered off and washed with water. The filtrate was concentrated under reduced pressure. To the resulting residue was added ethanol (containing ~1% methylethylketone, 250 mL) and the mixture was heated to 60 °C. Then, acetone (250 mL) was added and the mixture was stirred for 1 h forming a suspension. The mixture was allowed to cool to room temperature. The precipitate was filtered off, washed with acetone and dried under reduced pressure and elevated temperature to yield a solid (71.5 g).

HPLC purity (method d-2, area-%): 76.5 %
diastereomer 1 = 2.6%,
diastereomer 2 = 19.4%
diastereomer 3 = 54.5%.

Such material was further purified by preparative HPLC

Instrument: Agilent Series 1260 Infinity, pump: G1361A, autosampler: G2260A, fraction collector: G1364C, detector: G1315D; column: YMC Triart Actus C18, column size: 250 × 30 mm, 5 μm, band width: 12 nm, detection: DAD, wavelength: 210 nm; flowrate: 36 mL/min; eluent A: 0.1% formic acid in water; eluent B: 0.1% formic acid in water:acetonitrile = 9:1; gradient: isocratic, 65% A, 35% B.

Overall 140 runs with 0.2 g of material in 2 mL of water were carried out.

The combined product containing fractions from chromatography were concentrated under reduced pressure and elevated temperature (bath temperature: 60 °C) to yield a residue which was subsequently dissolved in water (50 mL) showing a slightly acidic pH due to formic acid resulting from chromatography. Ion exchange resin (16 g, type IRA 67, purchased from Merck KGaA, Germany) was added and the mixture was stirred for 1 h adjusting the pH to ~7. The resin was filtered off and washed with water. The filtrate was concentrated under reduced pressure and elevated temperature to yield a colorless foam-like solid (13.1 g)

**Analytics:**

Prior to analytical characterization, the material was equilibrated by storage in a desiccator over a saturated solution of potassium carbonate for at least 48 h.

| | | |
|---|---|---|
| HPLC purity (method d-2, area-%): | 96.2 % | |
| HPLC (method d-2): | | |
| Diastereomer distribution: | Diastereomer D1: | <0.03% |
| | Diastereomer D2: | 0.4% |
| | Diastereomer D3: | 99.6%. |
| Water content (method b): | 17.8 wt-% | |
| Residual Ethanol: (method b) | n.d. | |
| Relaxivity R1 [in water]: | 10.3 [L / (mmol*sec)] | |
| Relaxivity R2 [in water]: | 11.8 [L / (mmol*sec)] | |
| Solubility in water | >=565 mg/mL | |
| Solid state: | amorphous | |

The retention time of the compound of the formula (I) diastereomer 3 matches the one of a sample that was prepared via selective chemical synthesis as described in patent EP3303307B1, examples 3-1 and 3-2.

The stereochemical configuration and molecular structure was further verified by single crystal x-ray diffraction. For details see section Instrumentation and Methods and Table 5/Figure 17.

**Crystalline Forms**

**Crystalline forms of the compound of formula (I)**

**Table 1. XRPD data for the crystalline Form I and Form II of the compound of formula (I)**

| Diffraction angle (2θ, °) | |
|---|---|
| Form I | Form II |
| 6.4 | 5.4 |
| 6.8 | 7.1 |
| 9.1 | 7.3 |
| 10.1 | 9.1 |
| 11.4 | 10.2 |
| 12.0 | 10.8 |
| 12.9 | 11.3 |
| 13.6 | 13.3 |
| 14.4 | 14.8 |
| 15.9 | 15.1 |
| 16.4 | 15.6 |
| 17.2 | 17.0 |
| 17.7 | 17.7 |
| 18.3 | 19.4 |

(continued)

| Diffraction angle (2θ, °) | |
|---|---|
| Form I | Form II |
| 19.5 | 20.6 |
| 20.3 | 21.0 |
| 22.5 | 21.8 |
| 22.9 | 22.9 |
| 23.4 | 24.1 |
| 23.5 | 24.8 |
| 25.2 | 25.4 |
| 25.5 | 26.2 |
| 26.0 | 28.7 |
| 27.5 | 29.5 |
| 28.0 | 30.5 |
| 29.0 | 33.2 |
| 30.5 | 34.8 |
| 30.9 | 36.0 |
| 31.2 | |
| 32.0 | |
| 32.7 | |
| 33.4 | |
| 33.9 | |
| 34.9 | |
| 35.8 | |
| 36.5 | |

**Table 2. IR data for the crystalline Forms I and II of the compound of formula (I)**

| Band maxima ($cm^{-1}$) | |
|---|---|
| Form I | Form II |
| 438 | 406 |
| 494 | 430 |
| 564 | 447 |
| 608 | 495 |
| 713 | 508 |
| 789 | 549 |
| 832 | 562 |
| 906 | 633 |
| 935 | 667 |
| 1002 | 716 |
| 1083 | 790 |
| 1129 | 832 |
| 1153 | 882 |
| 1238 | 905 |
| 1278 | 933 |
| 1309 | 994 |
| 1383 | 1082 |
| 1455 | 1107 |
| 1557 | 1153 |
| 1594 | 1239 |

(continued)

| Band maxima ($cm^{-1}$) | |
|---|---|
| Form I | Form II |
| 1661 | 1277 |
| 2868 | 1316 |
| 2986 | 1380 |
| 3313 | 1435 |
| | 1472 |
| | 1557 |
| | 1596 |
| | 1660 |
| | 2871 |
| | 2983 |
| | 3291 |

**Table 3. Raman data for the crystalline Forms I and II of the compound of formula (I)**

| Band maxima ($cm^{-1}$) | |
|---|---|
| Form I | Form II |
| 208 | 225 |
| 238 | 275 |
| 280 | 311 |
| 311 | 355 |
| 356 | 375 |
| 387 | 390 |
| 412 | 452 |
| 435 | 497 |
| 441 | 512 |
| 453 | 561 |
| 478 | 614 |
| 500 | 632 |
| 512 | 658 |
| 547 | 722 |
| 568 | 769 |
| 611 | 792 |
| 659 | 833 |
| 725 | 868 |
| 773 | 907 |
| 791 | 935 |
| 804 | 981 |
| 834 | 1035 |
| 846 | 1085 |
| 866 | 1110 |
| 909 | 1137 |
| 938 | 1161 |
| 982 | 1191 |
| 1003 | 1207 |
| 1020 | 1244 |
| 1060 | 1277 |
| 1086 | 1297 |

(continued)

| Band maxima ($cm^{-1}$) | |
|---|---|
| Form I | Form II |
| 1110 | 1308 |
| 1138 | 1317 |
| 1159 | 1327 |
| 1191 | 1371 |
| 1222 | 1390 |
| 1241 | 1429 |
| 1305 | 1472 |
| 1336 | 1485 |
| 1371 | 1557 |
| 1393 | 1624 |
| 1422 | 2738 |
| 1460 | 2888 |
| 1488 | 2938 |
| 1550 | 2951 |
| 1614 | 2978 |
| 1668 | 3068 |
| 2736 | |
| 2890 | |
| 2948 | |
| 2965 | |
| 2979 | |
| 3061 | |
| 3309 | |

**Crystalline Forms of the diastereomers of the compound of formula (I)**

As described above, the compound of formula (I) can exist in different configurations, i.e. (R, R, R, R) and (S, S, S, S) (Diastereomer 3); (R, S, S, S) and (S, R, R, R) (Diastereomer 2); (S, S, R, R) (Meso form, Diastereomer 1) either in pure form or as mixtures of two or more of these diastereomeric configurations.

The absolute configurations of diastereomers 1 and 3 of the compound of formula (I) were determined by single crystal diffraction (SCD). For diastereomer 1, the asymmetric part of the unit cell is shown in Figure 16. The crystal data and structure refinement are shown in Table 4. For diastereomer 3, the asymmetric part of the unit cell is shown in Figure 17. The crystal data and structure refinement are shown in Table 5.

**Table 4. Crystal data and structure refinement for Diastereomer 1 of the compound of formula (I).**

| | | |
|---|---|---|
| Empirical formula (asymmetric part) | $C_{25.35}H_{49.30}GdN6O_{11.5}$ | |
| Chemical formula (grown) | $C_{81}H_{128}Gd_4N_{24}O_{32}$, 10.2 ($C_2H_6O$), 4($H_2O$) | |
| Formula weight | 780.25 | |
| Temperature | 100(2) K | |
| Wavelength | 1.54184 Å | |
| Crystal size | 0.120 × 0.080 × 0.030 mm | |
| Crystal system | Tetragonal | |
| Space group | $I\bar{4}$ | |
| Unit cell dimensions | a = 19.31424(12) Å | $\alpha$= 90° |
| | *b* = 19.31424(12) Å | $\beta$= 90° |
| | *c* = 17.61367(16) Å | $\gamma$ = 90° |
| Volume | 6570.60(10) $Å^3$ | |
| Z | 8 | |

(continued)

| | |
|---|---|
| Density (calculated) | 1.578 Mg/m$^3$ |
| Absorption coefficient | 13.620 mm$^{-1}$ |
| F(000) | 3198 |
| Data collection method | omega scans |
| Theta range for data collection | 3.396 to 68.217° |
| Index ranges | $-23 \leq h \leq 22$, $-23 \leq k \leq 23$, $-21 \leq l \leq 21$ |
| Reflections collected | 127661 |
| Independent reflections | 6022 [R(int) = 0.0718] |
| Coverage of independent reflections | 100.0 % |
| Data / restraints / parameters | 6022 / 22 / 418 |
| Goodness-of-fit on $F^2$ | 1.107 |
| Final R indices | |
| 5999 data; I>2σ(I) | R1 = 0.0307, wR2 = 0.0861 |
| all data | R1 = 0.0308, wR2 = 0.0862 |
| Absolute structure parameter | 0.0010(18) |
| Extinction coefficient | n/a |
| Largest diff. peak and hole | 0.809 and -0.480 $e$Å$^{-3}$ |

**Table 5. Crystal data and structure refinement for Diastereomer 3 of the compound of formula (I)**.

| | | |
|---|---|---|
| Empirical formula | $C_{81}H_{128}Gd_4N_{24}O_{32}$ | |
| Formula weight | 2579.07 | |
| Temperature | 100(2) K | |
| Wavelength | 1.54184 Å | |
| Crystal size | 0.170 x 0.150 x 0.040 mm | |
| Crystal system | Monoclinic | |
| Space group | $P2_1/n$ | |
| Unit cell dimensions | $a$ = 18.7196(3) Å | $\alpha$= 90° |
| | $b$ = 17.06182(19) Å | $\beta$= 90.3934(14)° |
| | $c$ = 43.4523(6) Å | $\gamma$ = 90° |
| Volume | 13877.9(3) Å$^3$ | |
| Z | 4 | |
| Density (calculated) | 1.234 Mg/m$^3$ | |
| Absorption coefficient | 12.718 mm$^{-1}$ | |
| $F$(000) | 5176 | |
| Theta range for data collection | 2.564 to 68.251° | |
| Index ranges | $-22 \leq h \leq 22$, $-14 \leq k \leq 20$, $-52 \leq l \leq 52$ | |
| Reflections collected | 253873 | |
| Independent reflections | 25376 [R(int) = 0.1197] | |
| Coverage of independent reflections | 99.7 % | |
| Data / restraints / parameters | 25376 / 2659 1 1293 | |
| Goodness-of-fit on $F^2$ | 1.082 | |
| Final R indices | | |
| 18704 data; I>2σ(I) | R1 = 0.0678, $w$R2 = 0.1904 | |
| all data | R1 = 0.0870, $w$R2 = 0.2034 | |
| Absolute structure parameter | n/a | |
| Extinction coefficient | 0.000084(11) | |
| Largest diff. peak and hole | 1.099 and -1.385 $e$Å$^{-3}$ | |

## Crystalline forms of the compound of formula (II)

**Table 6. XRPD data for the crystalline Forms I, II and III of the compound of formula (II)**

| Diffraction angle (2θ, °) | | |
|---|---|---|
| Form I | Form II | Form III |
| 8.0 | 4.2 | |
| 9.0 | 5.5 | |
| 10.4 | 6.0 | |
| 11.7 | 6.6 | |
| 12.8 | 7.0 | |
| 14.9 | 8.3 | |
| 17.0 | 10.3 | |
| 17.4 | 10.7 | 5.6 |
| 18.6 | 11.1 | 5.8 |
| 18.9 | 11.5 | 7.2 |
| 19.3 | 11.7 | 7.7 |
| 22.7 | 12.2 | 9.6 |
| 23.0 | 12.6 | 10.2 |
| 23.5 | 13.0 | 11.1 |
| 24.5 | 13.3 | 11.3 |
| 24.8 | 13.9 | 12.1 |
| 25.8 | 14.9 | 12.5 |
| 26.4 | 16.0 | 13.0 |
| 26.9 | 16.6 | 14.3 |
| 28.3 | 17.1 | 15.1 |
| 29.8 | 20.3 | 16.3 |
| 30.4 | 22.5 | 16.7 |
| 30.9 | 23.2 | 19.8 |
| 32.6 | 23.9 | 20.5 |
| | 24.3 | 21.4 |
| | 25.0 | 22.0 |
| | 25.8 | 25.4 |
| | 26.1 | 26.3 |
| | 28.0 | 27.0 |
| | 28.7 | 27.6 |
| | 29.8 | 29.0 |
| | 30.4 | 30.4 |
| | 30.9 | 33.8 |
| | 31.3 | 34.8 |
| | 31.7 | 39.3 |
| | 32.3 | |
| | 33.2 | |
| | 33.8 | |
| | 35.4 | |
| | 35.7 | |
| | 37.8 | |
| | 38.6 | |

**Table 7. IR data for the crystalline Forms I, II and III of the compound of formula (II)**

| Band maxima ($cm^{-1}$) | | |
|---|---|---|
| Form I | Form II | Form III |
| 451 | 438 | 439 |
| 503 | 447 | 494 |
| 531 | 496 | 612 |
| 562 | 530 | 709 |
| 569 | 614 | 747 |
| 634 | 713 | 756 |
| 647 | 790 | 789 |
| 667 | 822 | 854 |
| 715 | 862 | 925 |
| 775 | 928 | 1019 |
| 835 | 1019 | 1083 |
| 854 | 1085 | 1111 |
| 864 | 1131 | 1131 |
| 907 | 1153 | 1152 |
| 936 | 1213 | 1208 |
| 996 | 1321 | 1291 |
| 1019 | 1346 | 1329 |
| 1085 | 1489 | 1347 |
| 1137 | 1521 | 1489 |
| 1154 | 1612 | 1521 |
| 1211 | 1683 | 1558 |
| 1241 | 1774 | 1569 |
| 1317 | 2854 | 1575 |
| 1343 | 2976 | 1607 |
| 1390 | 3070 | 1652 |
| 1447 | | 1688 |
| 1522 | | 1694 |
| 1557 | | 1699 |
| 1598 | | 1777 |
| 1652 | | 2873 |
| 1688 | | 2981 |
| 1779 | | 3076 |
| 2872 | | 3243 |
| 2992 | | |
| 3120 | | |

**Table 8. Raman data for the crystalline Forms I, II and III of the compound of formula (II)**

| Band maxima ($cm^{-1}$) | | |
|---|---|---|
| Form I | Form II | Form III |
| 283 | 313 | 235 |
| 317 | 382 | 300 |
| 374 | 449 | 383 |
| 502 | 495 | 448 |
| 629 | 627 | 499 |

(continued)

| Band maxima ($cm^{-1}$) | | |
|---|---|---|
| Form I | Form II | Form III |
| 748 | 750 | 636 |
| 836 | 783 | 823 |
| 865 | 823 | 864 |
| 937 | 864 | 933 |
| 999 | 899 | 1113 |
| 1047 | 929 | 1167 |
| 1111 | 1016 | 1215 |
| 1139 | 1053 | 1295 |
| 1166 | 1111 | 1332 |
| 1213 | 1168 | 1350 |
| 1346 | 1215 | 1463 |
| 1396 | 1295 | 1522 |
| 1463 | 1335 | 1592 |
| 1488 | 1348 | 2883 |
| 1525 | 1461 | 2932 |
| 1594 | 1478 | 2964 |
| 2884 | 1491 | 3078 |
| 2987 | 1518 | |
| 3087 | 1592 | |
| | 2885 | |
| | 2935 | |
| | 2960 | |
| | 3085 | |

## Claims

**1.** A process for the isolation of a compound of general formula (I),

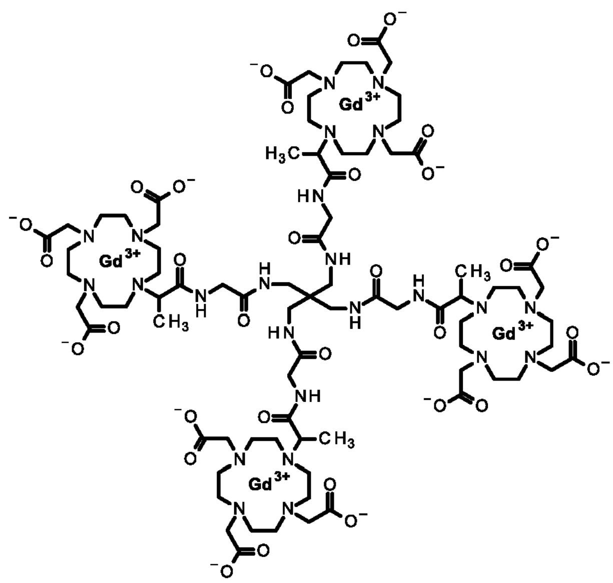

**(I)**

or its stereoisomers, tautomers, N-oxides, hydrates, solvates, or salts thereof, or mixtures of same, said process

comprising

(i) providing a mixture comprising a compound of formula (I), water and at least a first organic solvent,
(ii) removing water from the mixture provided in (i),
(iii) adding a second organic solvent,
(iv) optionally adding a third organic solvent,
(v) isolating the compound of formula (I).

**2.** The process according to claim 1, wherein the second organic solvent in step (iii) is an alcohol selected from the list consisting of ethanol, n-propanol and iso-propanol, and wherein the third organic solvent in step (iv) is acetone.

**3.** The process according to any of claims 1 or 2, wherein the second organic solvent in step (iii) is ethanol.

**4.** The process according to any of claims 1 to 3, wherein step (ii) comprises removing water from the mixture provided in (i) until the water content of the mixture lies between 10% and 20% by weight (w/w).

**5.** The process according to any of claims 1 or 4, wherein the at least first organic solvent in step (i) is DMSO.

**6.** The process according to any of claims 1 to 5, wherein the isolation of the compound of formula (I) in step (v) comprises the following steps:

(v-1) providing an aqueous mixture comprising the compound of formula (I),
(v-2) adding an organic solvent,
(v-3) drying the solid obtained in (v-2).

**7.** The process according to claim 6, wherein the organic solvent in (v-2) is ethanol, and wherein in (v-3) drying the solid obtained in (v-2) is carried out at a relative humidity of from 18% to 70%, preferably of from 30% to 65%.

**8.** The process according to any of claims 6 or 7, wherein the organic solvent in (v-2) is ethanol, and wherein in (v-3) drying the solid obtained in (v-2) is carried out first at a relative humidity of from 0% to 25%, preferably of from 2% to 18%, more preferably of from 5% to 16% followed by a second drying process by adjusting the relative humidity until a final value of from 18% to 70%, preferably of from 25% to 70%, more preferably of from 30% to 70% is reached.

**9.** A crystalline Form I of the compound of formula (I) **characterized by** having a powder X-ray diffractogram comprising reflections at 2-Theta angles of (6.8 ± 0.2)°, (9.1 ± 0.2)° and (11.4 ± 0.2)°, preferably comprising reflections at 2-Theta angles of (6.8 ± 0.2)°, (9.1 ± 0.2)°, (10.1 ± 0.2)°, (11.4 ± 0.2)° and (12.0 ± 0.2)°, more preferably comprising reflections at 2-Theta angles of (6.8 ± 0.2)°, (9.1 ± 0.2)°, (10.1 ± 0.2)°, (11.4 ± 0.2)°, (12.0 ± 0.2)°, (14.4 ± 0.2)° and (23.5 ± 0.2)°, when measured at room temperature with Cu-Kalpha1 radiation having a wavelength of 0.15419 nm.

**10.** A crystalline Form II of the compound of formula (I) **characterized by** having a powder X-ray diffractogram comprising reflections at 2-Theta angles of (10.2 ± 0.2)°, (10.8 ± 0.2)° and (11.3 ± 0.2)°, preferably comprising reflections at 2-Theta angles of (7.3 ± 0.2)°, (10.2 ± 0.2)°, (10.8 ± 0.2)°, (11.3 ± 0.2)° and (13.3 ± 0.2)°, more preferably comprising reflections at 2-Theta angles of (7.1 ± 0.2)°, (7.3 ± 0.2)°, (10.2 ± 0.2)°, (10.8 ± 0.2)°, (11.3 ± 0.2)°, (13.3 ± 0.2)° and (15.1 ± 0.2)°, when measured at room temperature with Cu-Kalpha1 radiation having a wavelength of 0.15419 nm.

**11.** A process for the preparation of the crystalline Form I of a compound of formula (I) according to claim 9

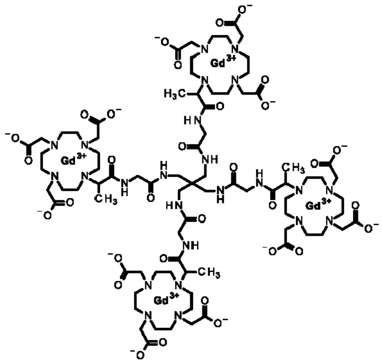

(I)

said process comprising:

(i) providing an aqueous mixture comprising the compound of formula (I),
(ii) adding an organic solvent,
(iii) drying the solid obtained in (ii) at a relative humidity of from 18% to 70%, preferably of from 30% to 65%.

**12.** A process for the preparation of the crystalline Form II of a compound of formula (I) according to claim 10

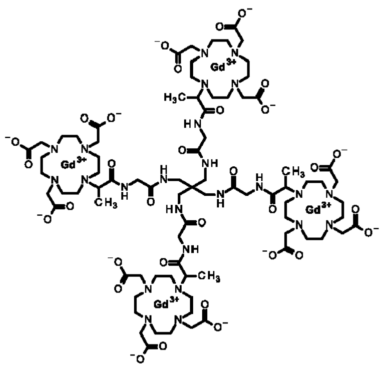

(I)

said process comprising:

(i) providing an aqueous mixture comprising the compound of formula (I),
(ii) adding an organic solvent,
(iii) drying the solid obtained in (ii) first at a relative humidity of from 0% to 25%, preferably of from 2% to 18%, more preferably of from 5% to 16% followed by a second drying process by adjusting the relative humidity until a final value of from 18% to 70%, preferably of from 25% to 70%, more preferably of from 30% to 70% is reached.

**13.** The process according to any of claims 11 or 12, wherein the organic solvent in step (ii) is ethanol.

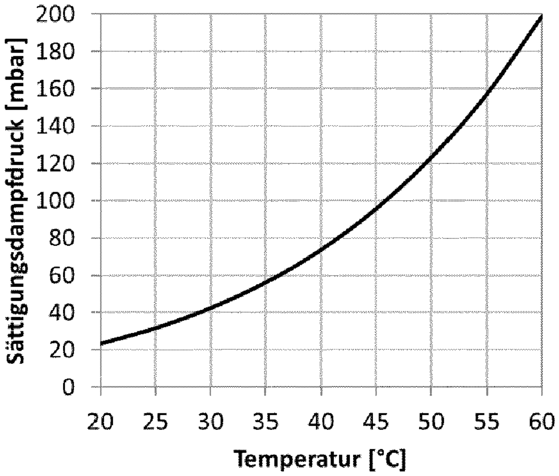


Figure 1

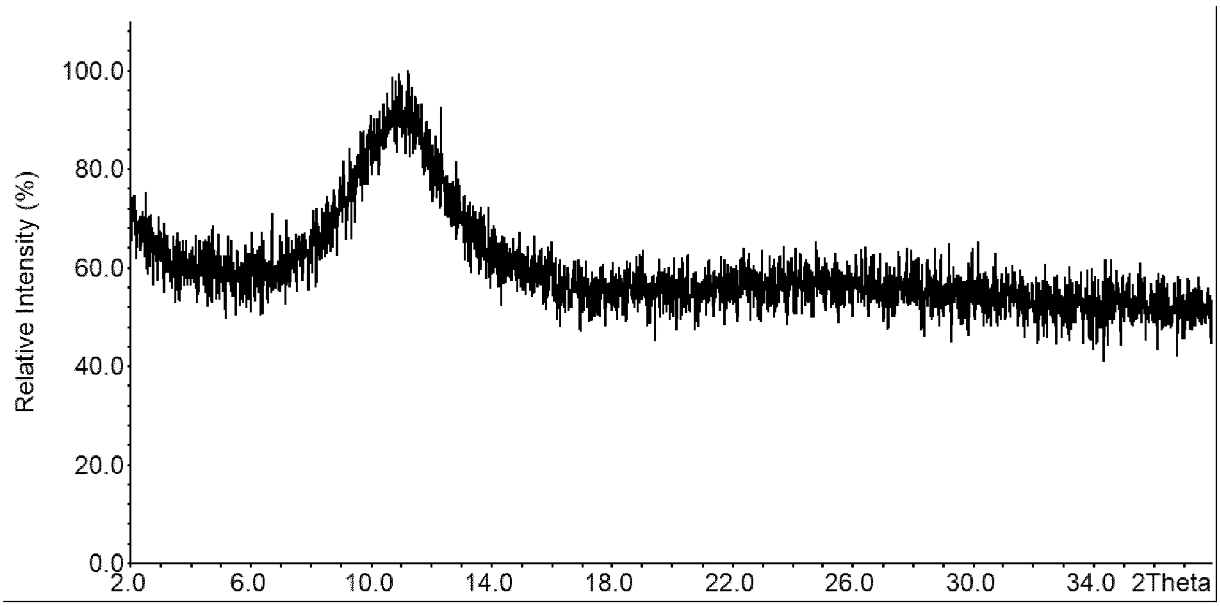


Figure 2

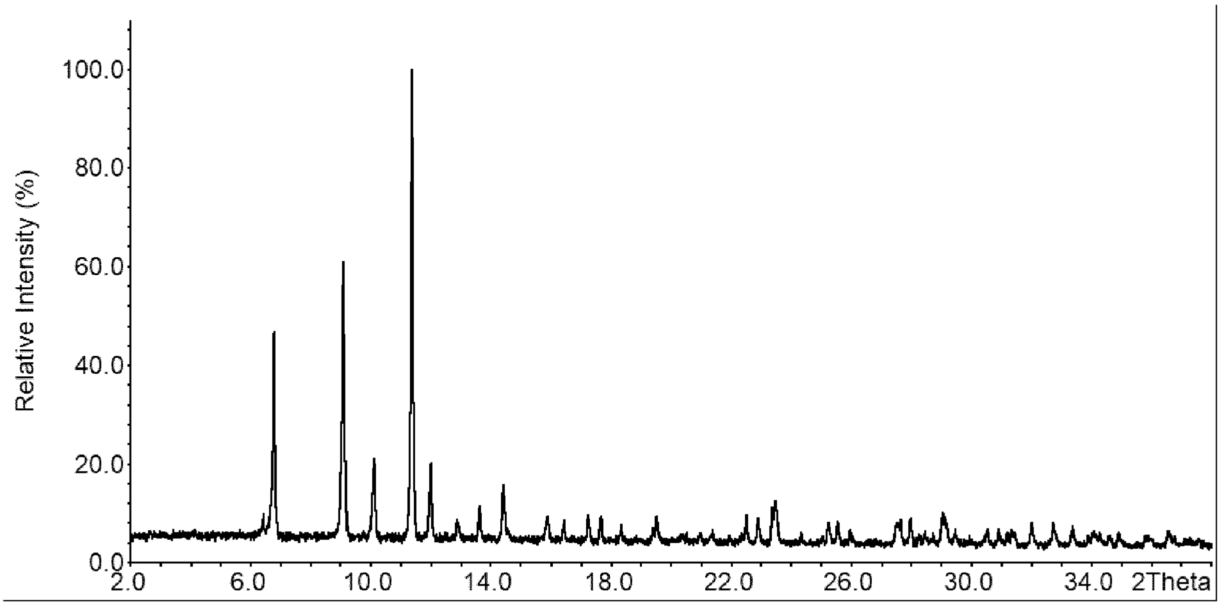


Figure 3

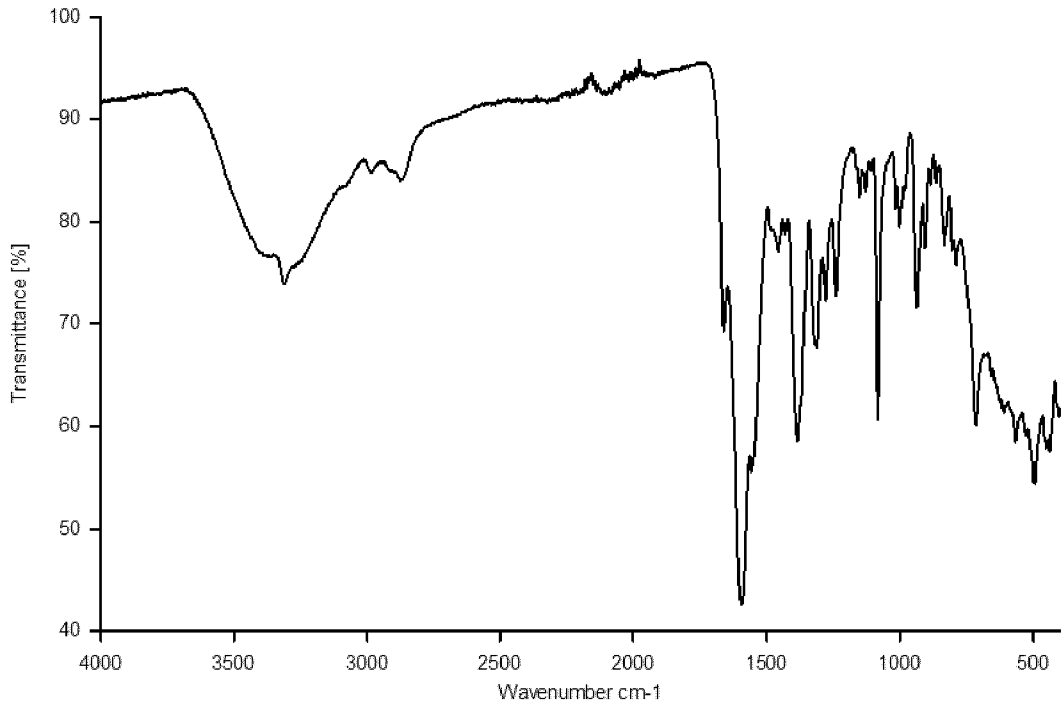


Figure 4

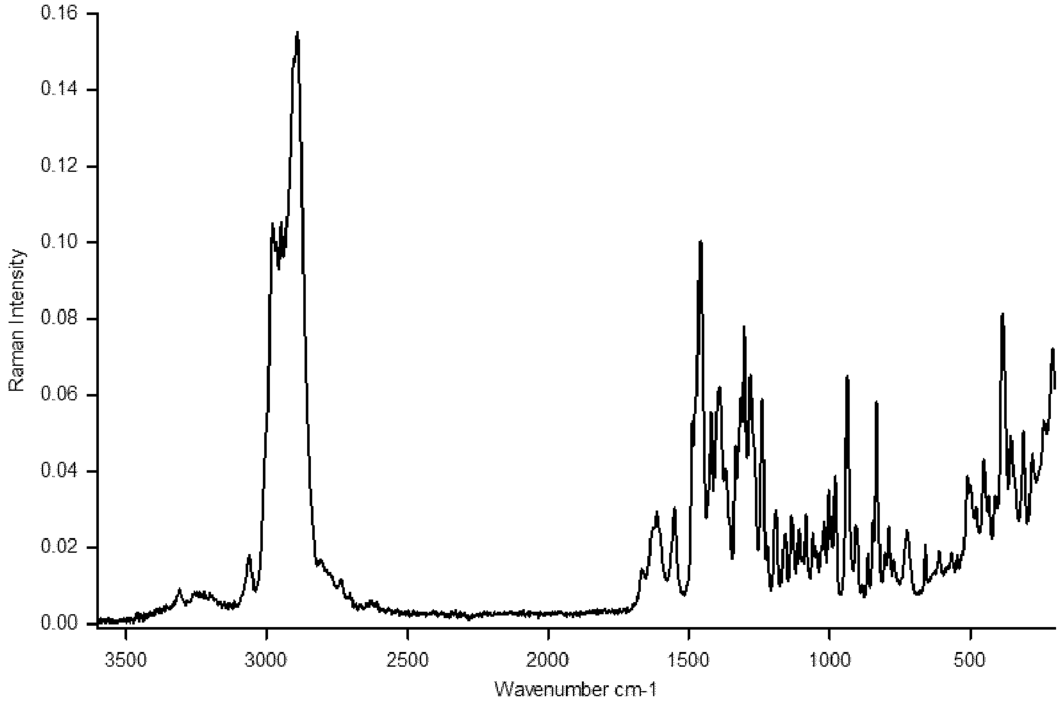


Figure 5

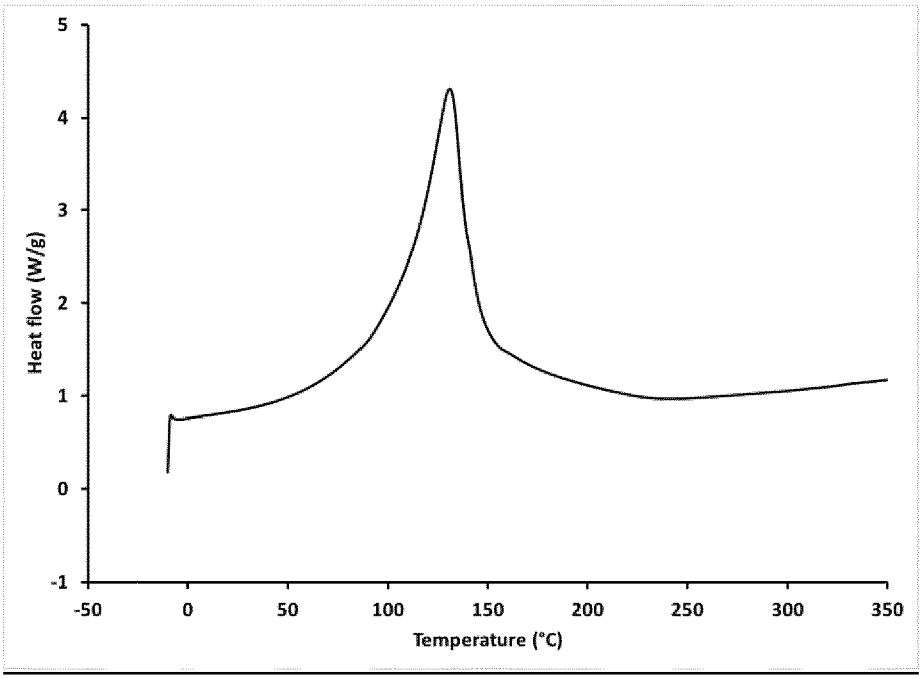


Figure 6

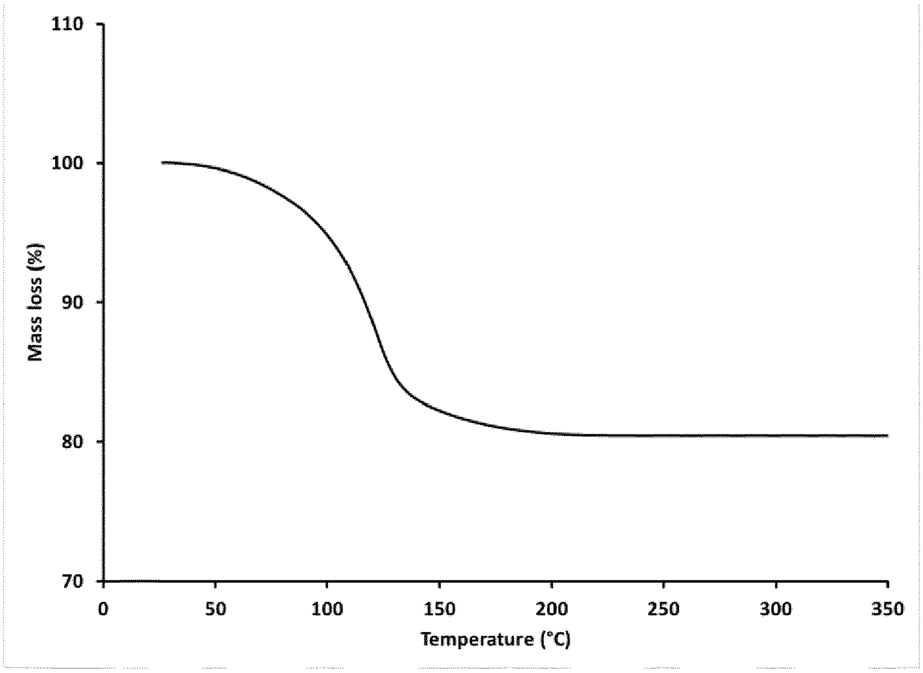


Figure 7

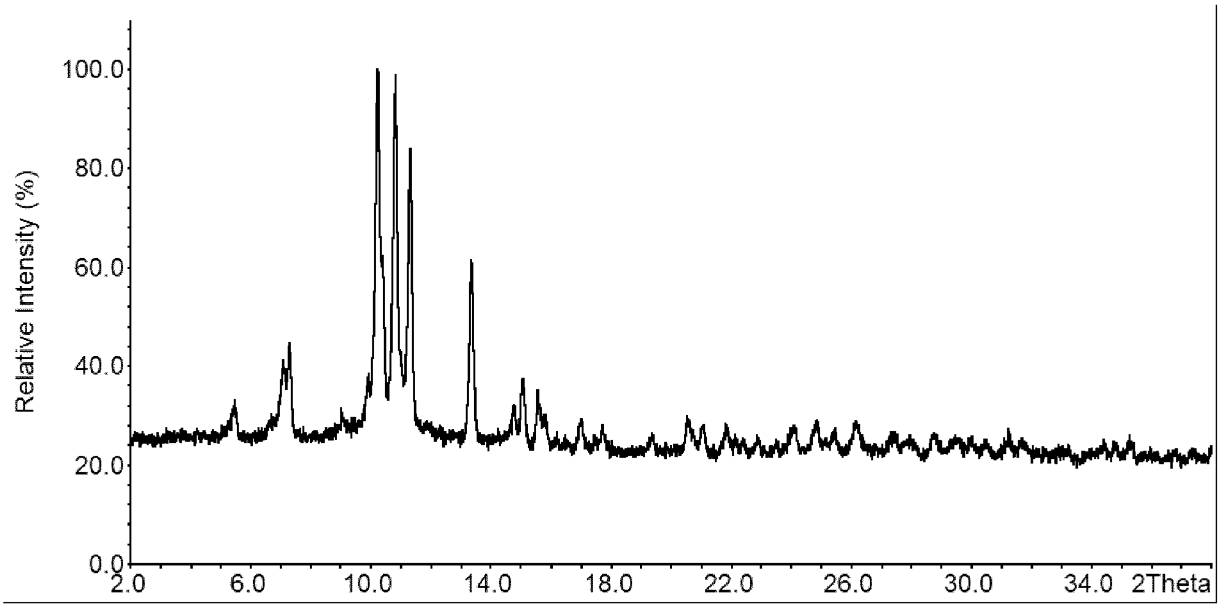


Figure 8

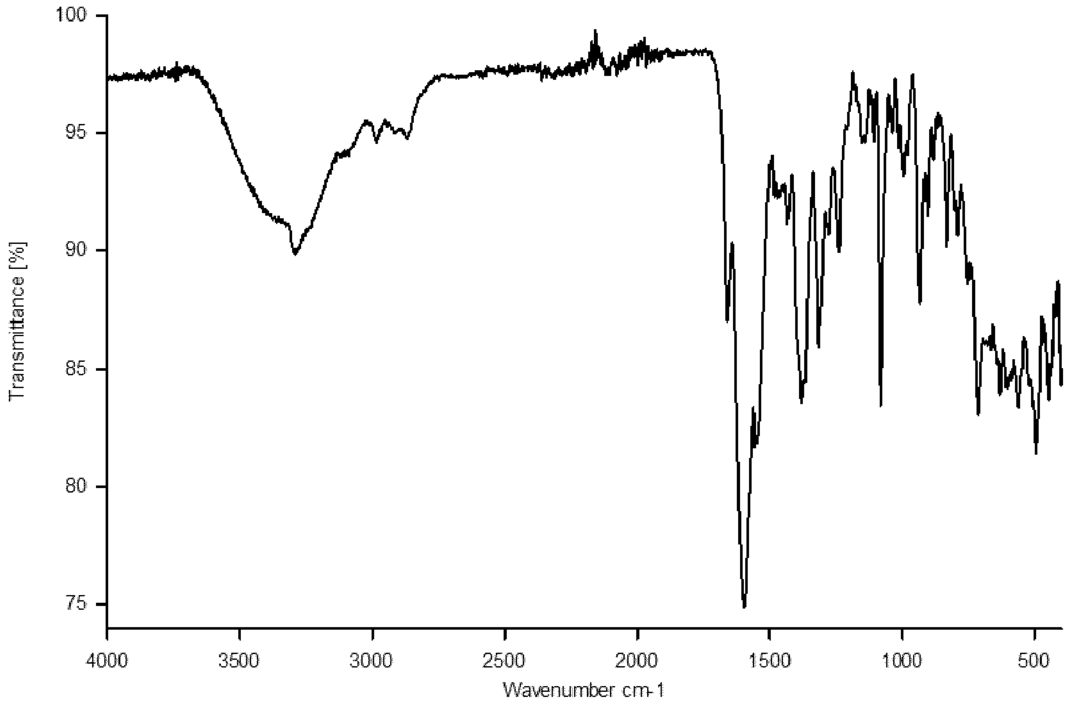


Figure 9

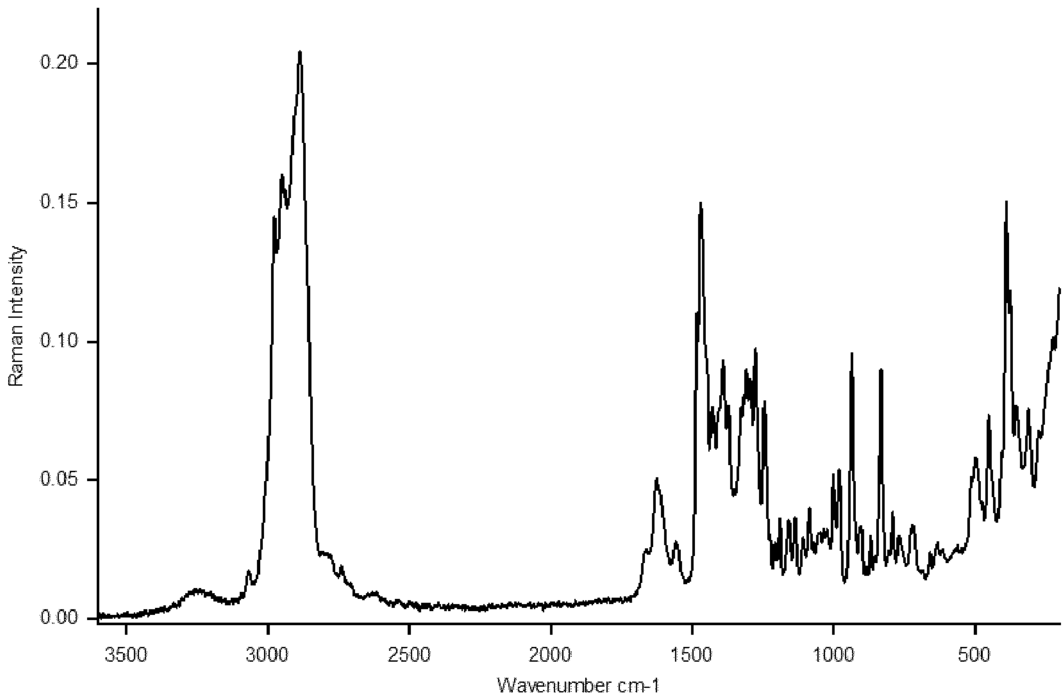


Figure 10

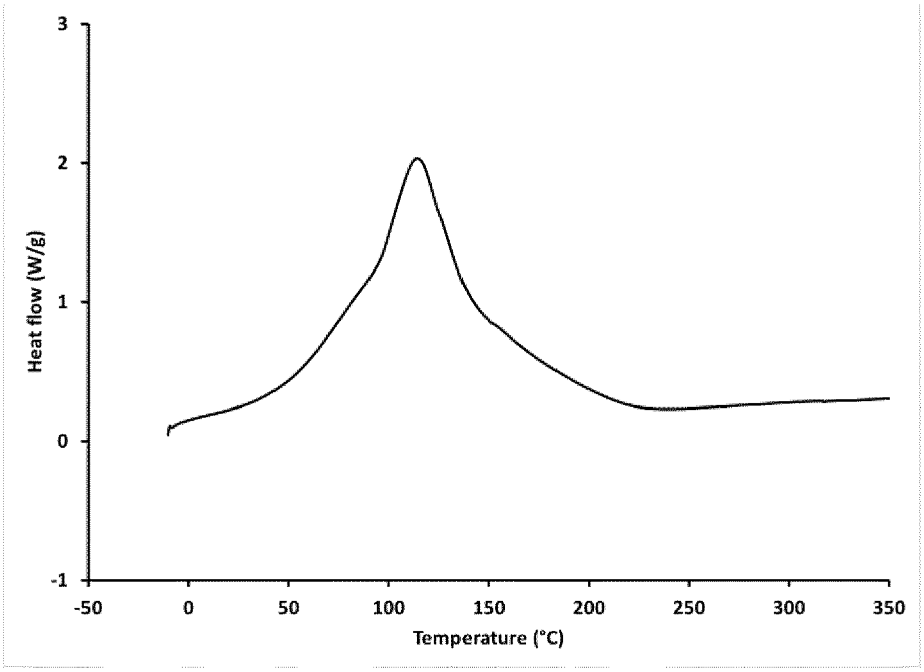


Figure 11

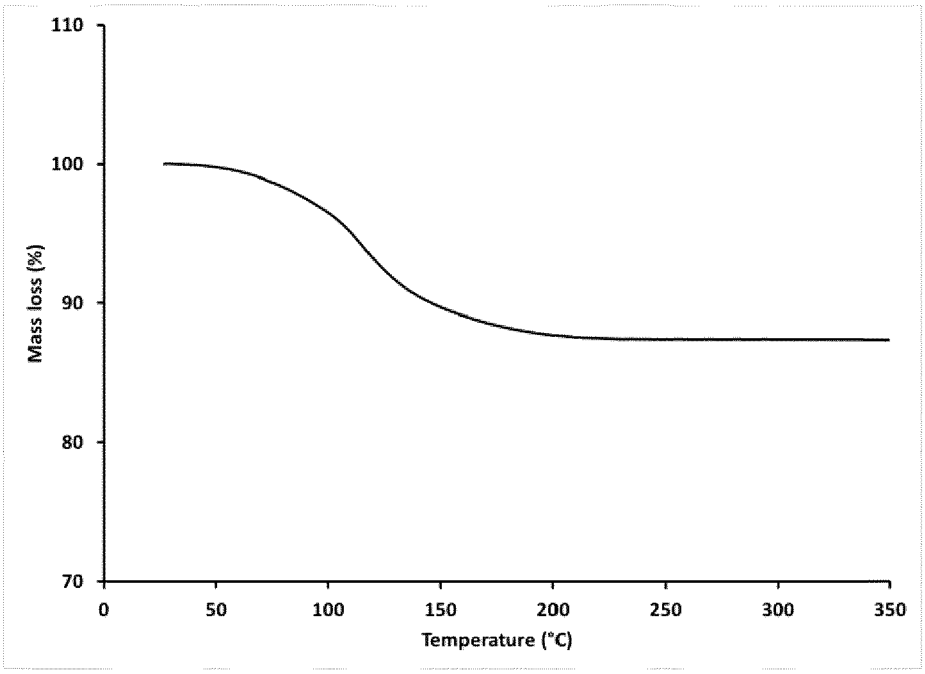


Figure 12

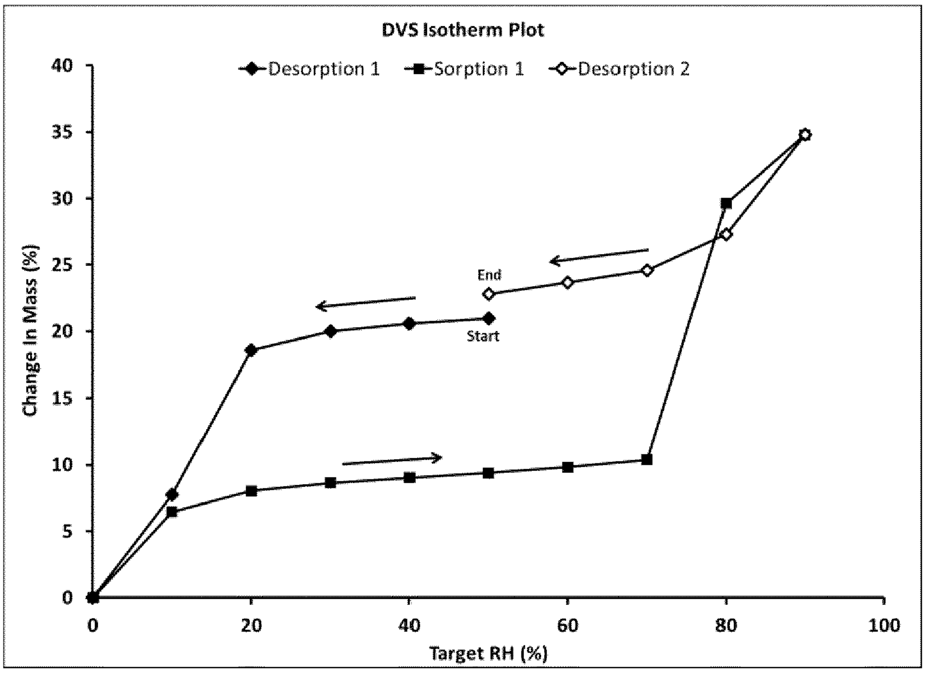


Figure 13

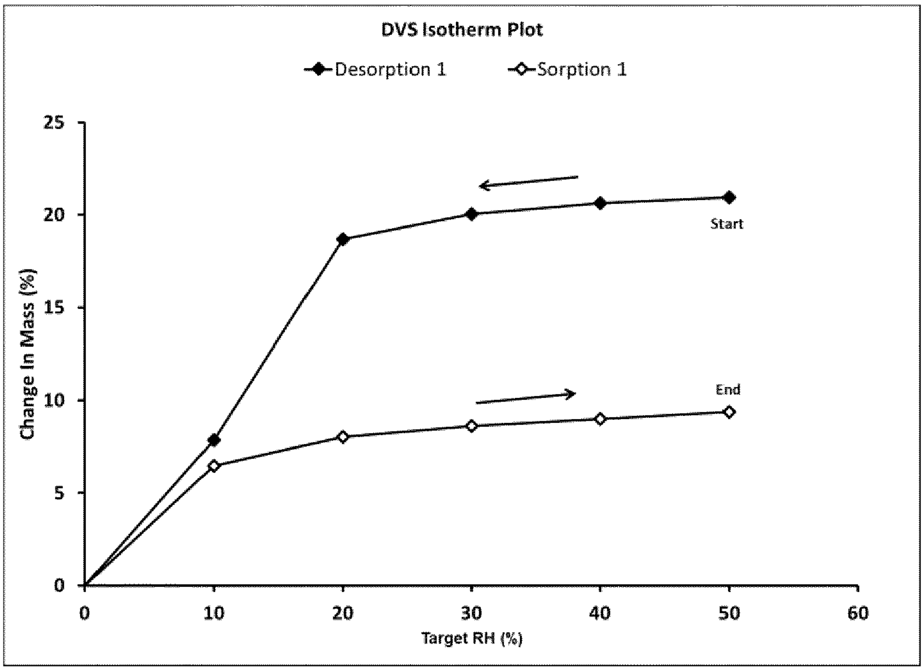


Figure 14

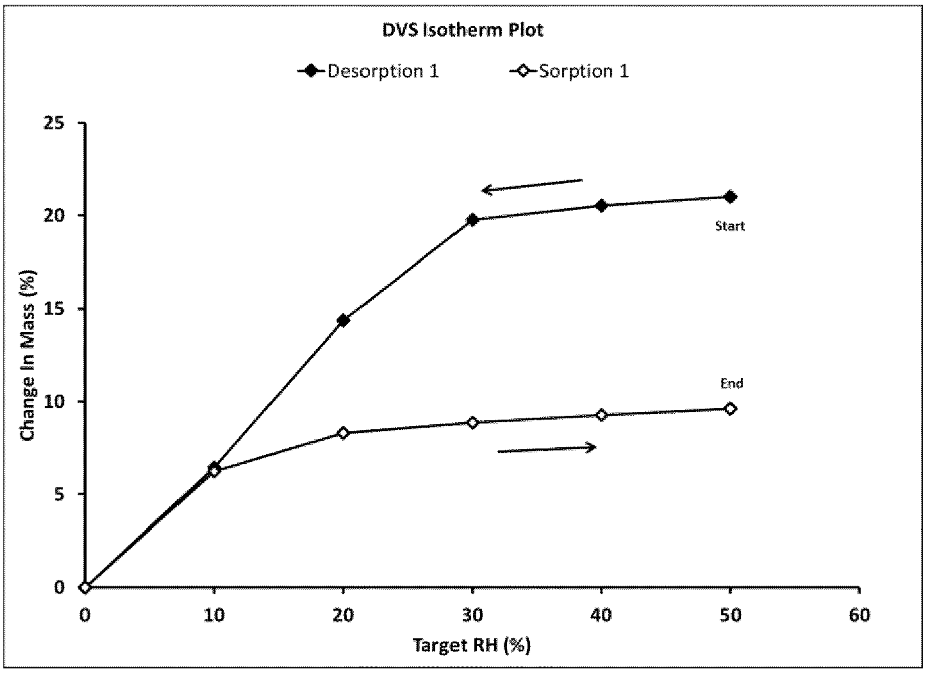


Figure 15

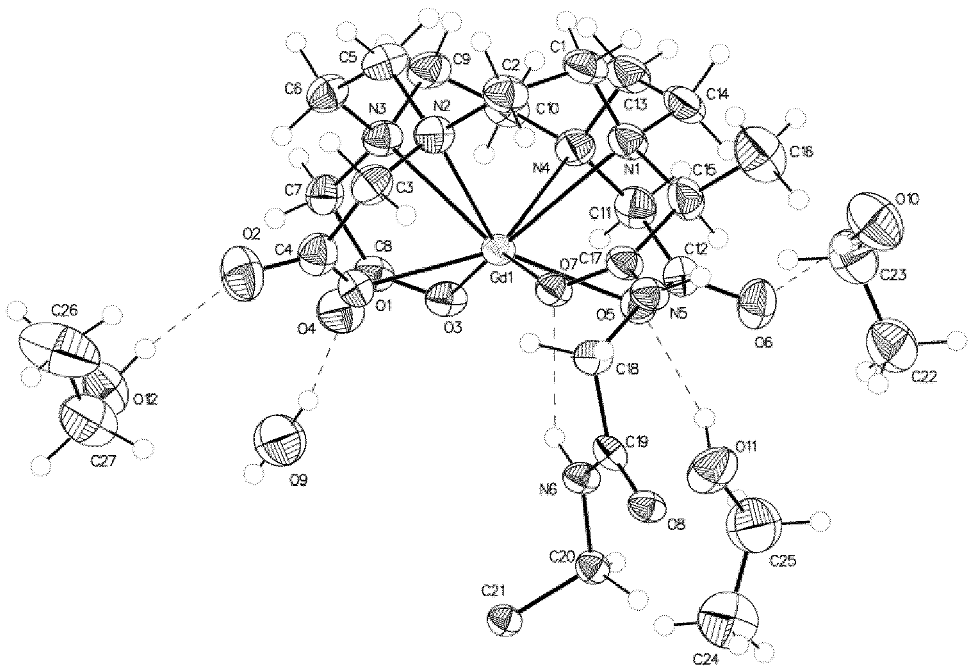

Figure 16

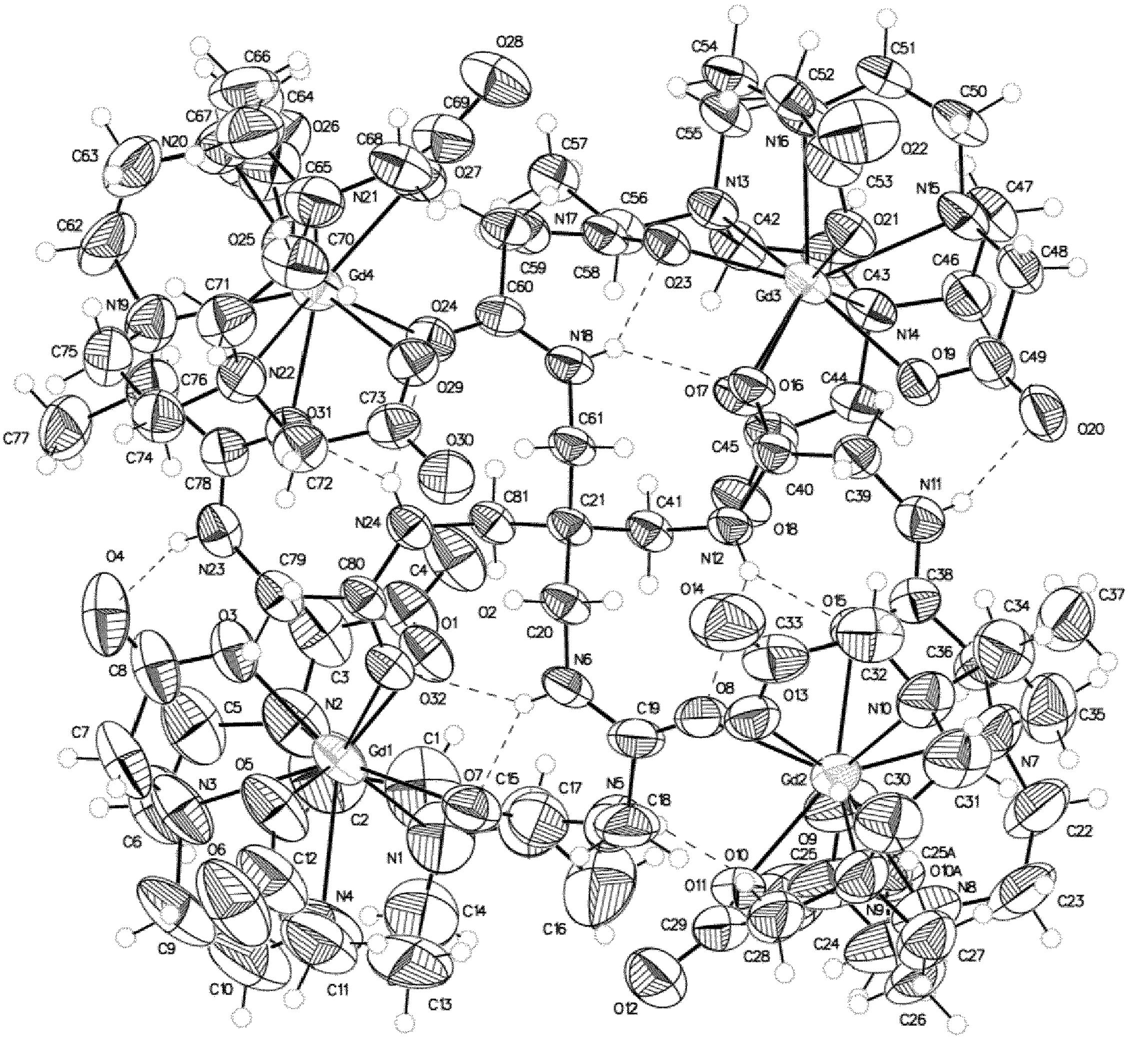

Figure 17

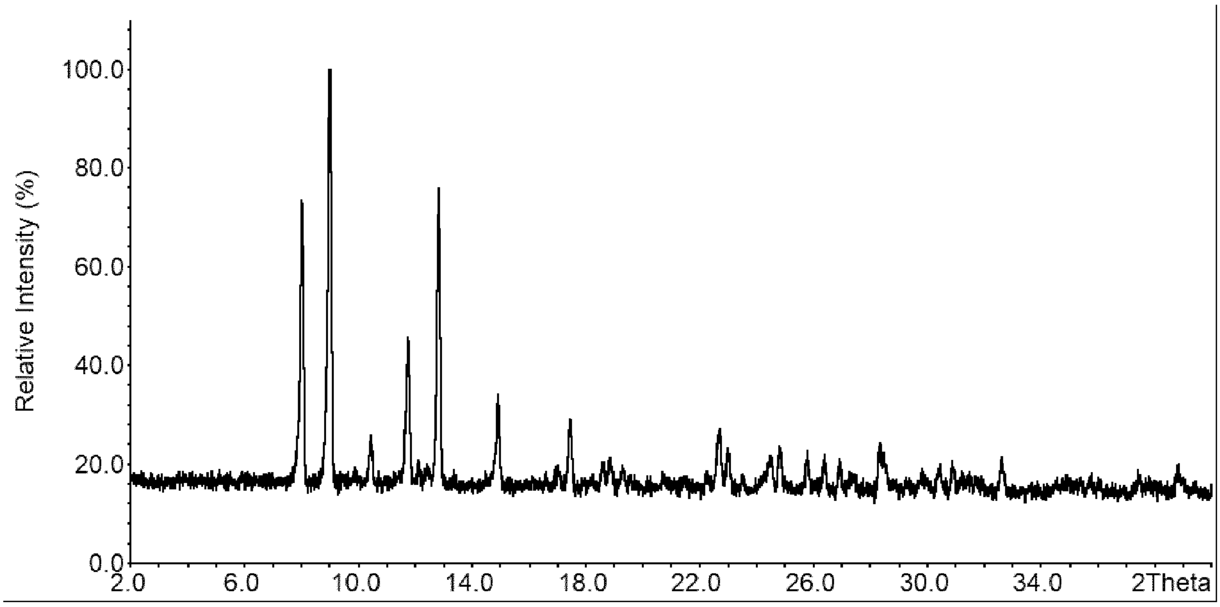


**Figure 18**

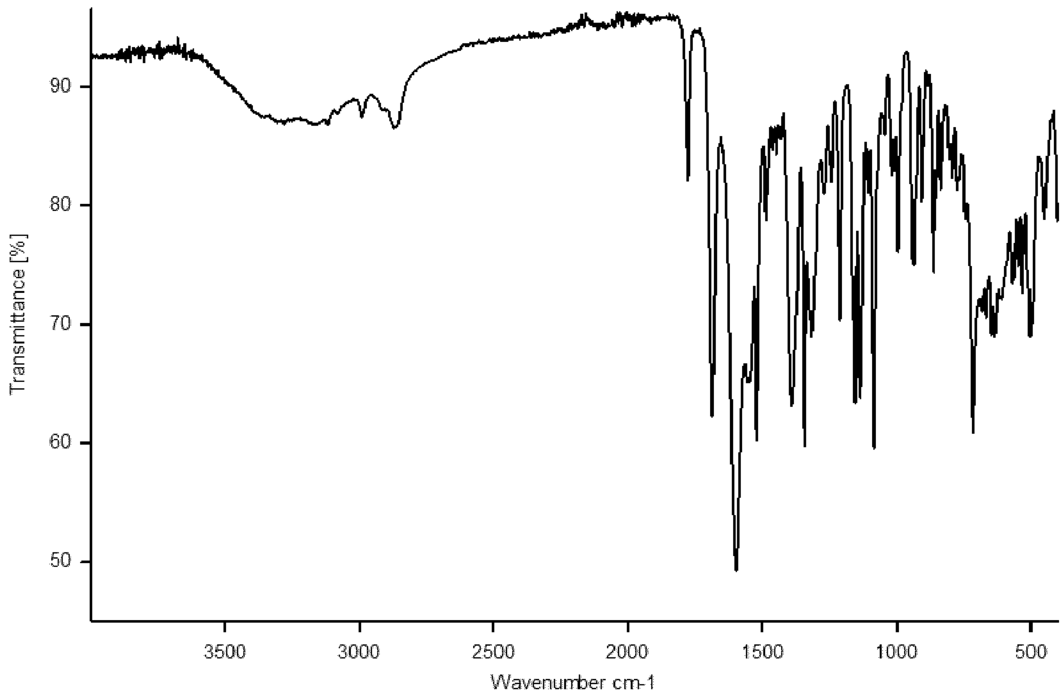


**Figure 19**

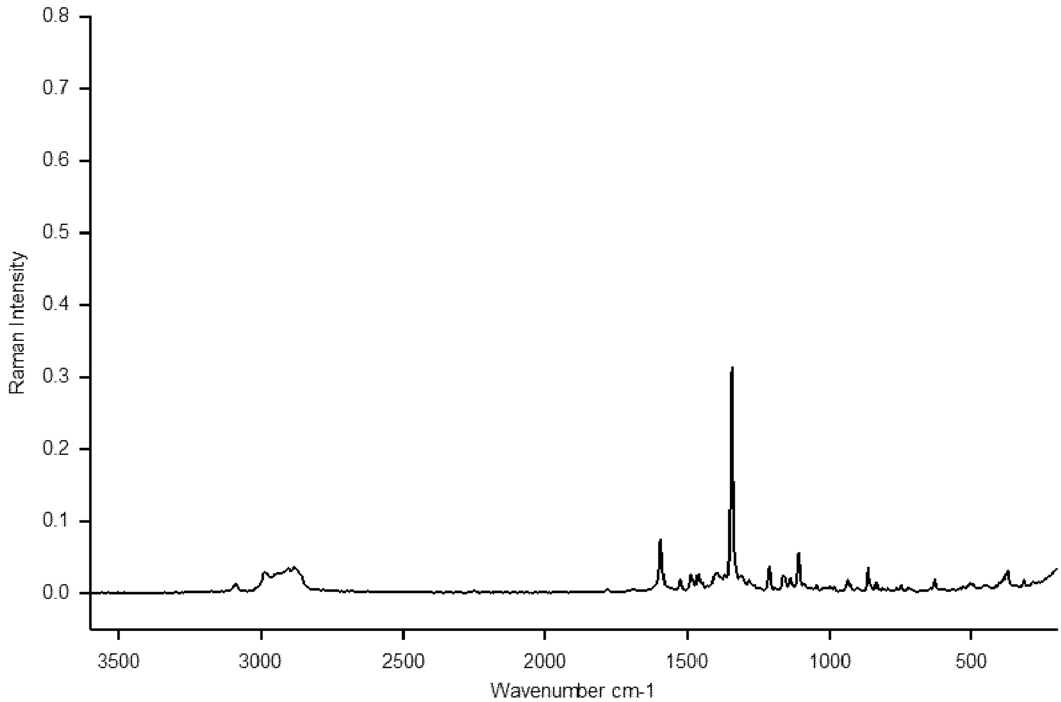


Figure 20

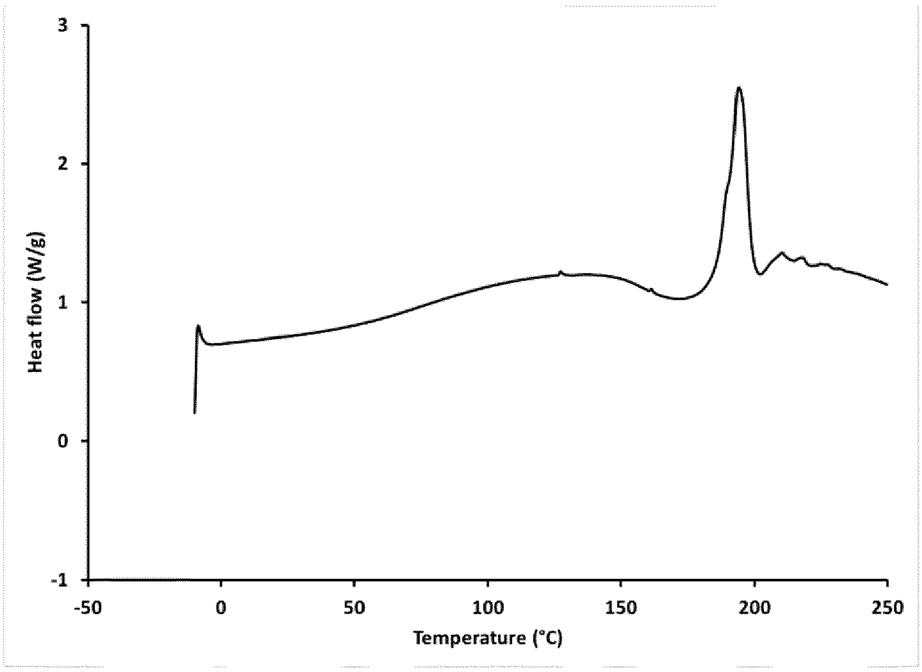


Figure 21

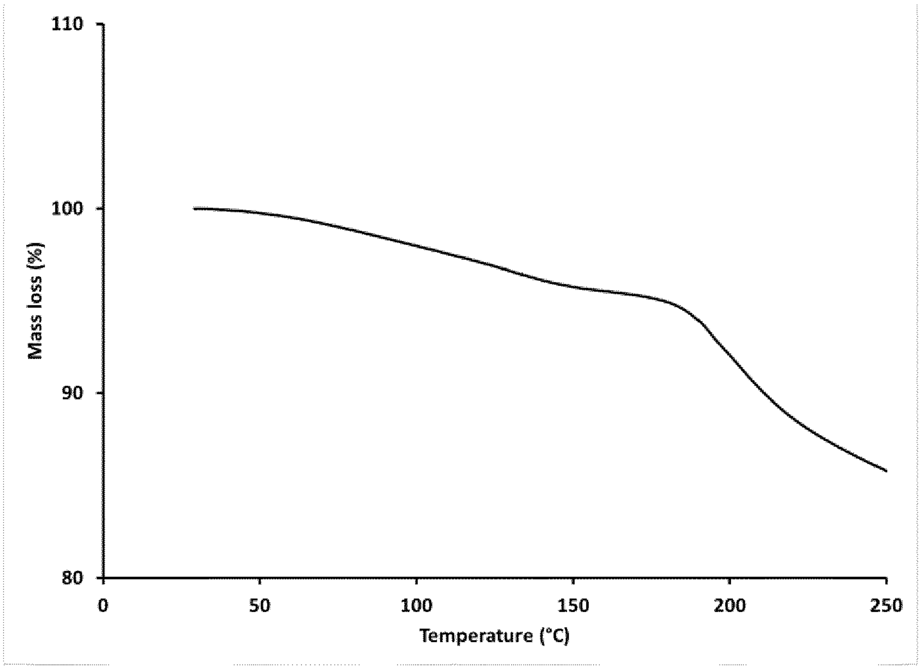


Figure 22

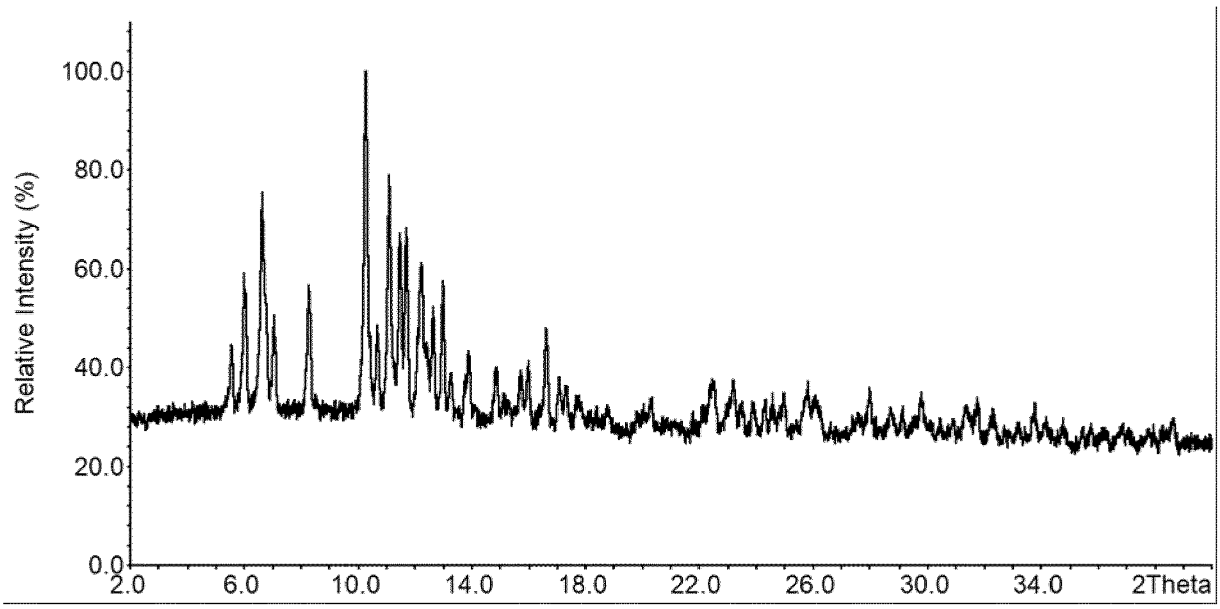


Figure 23

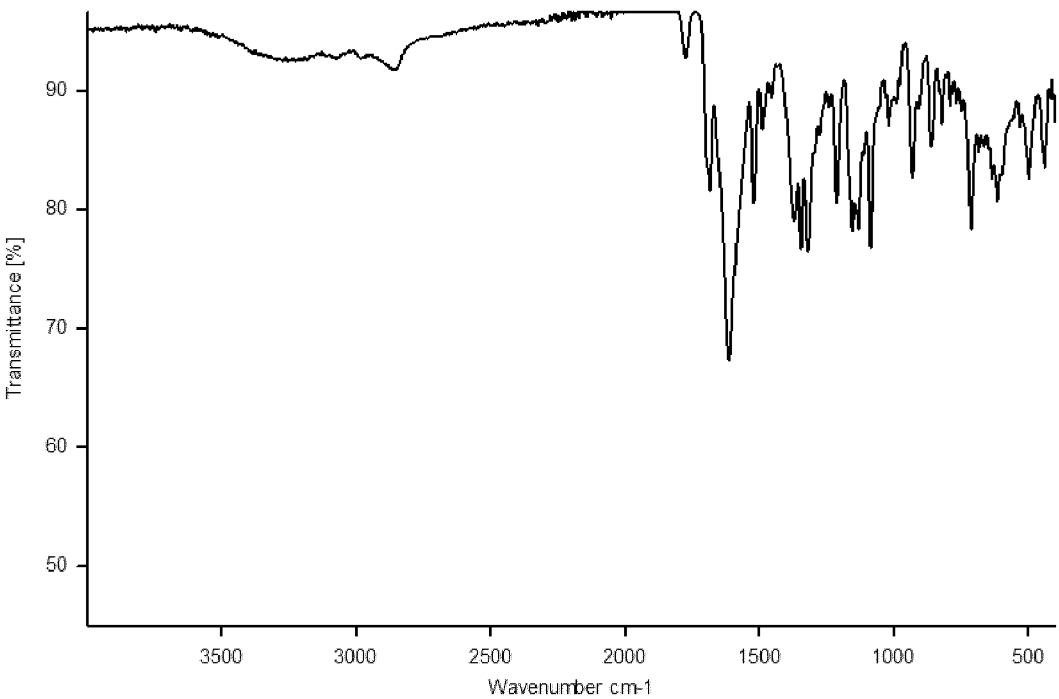


Figure 24

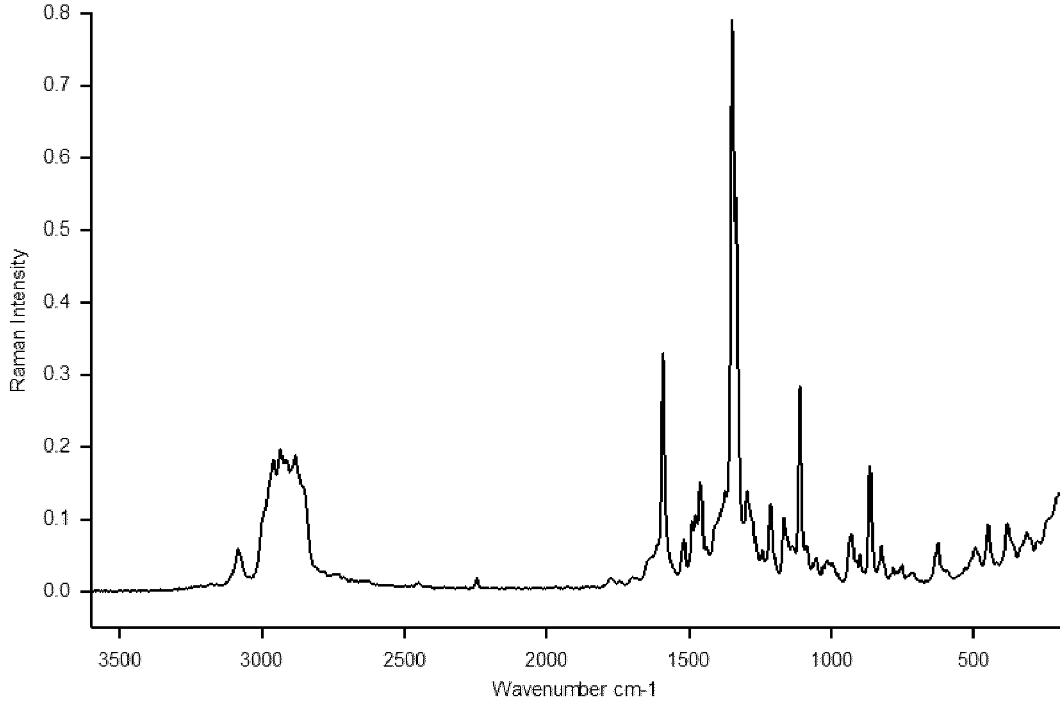


Figure 25

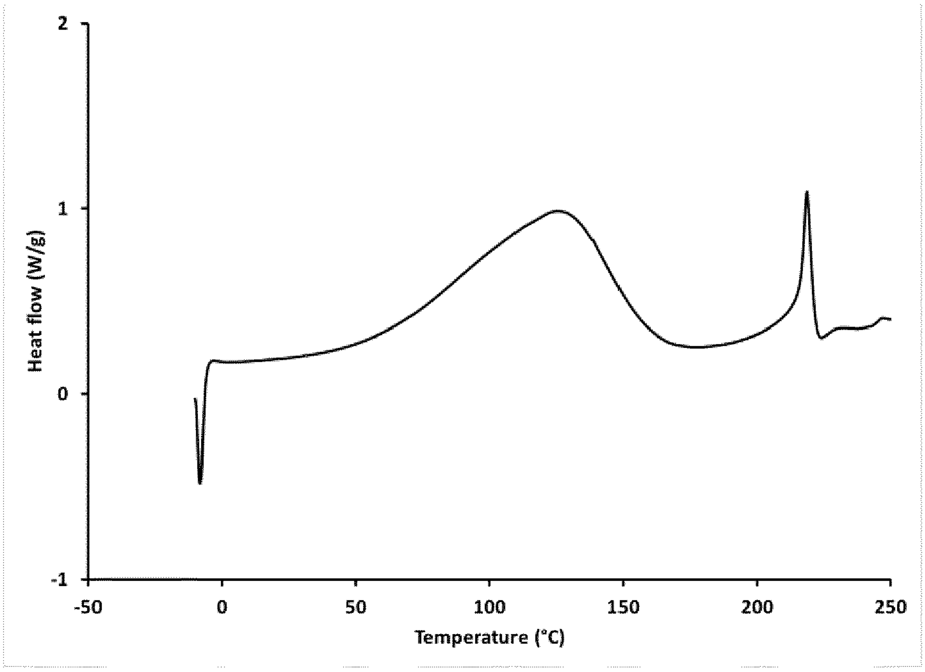


Figure 26

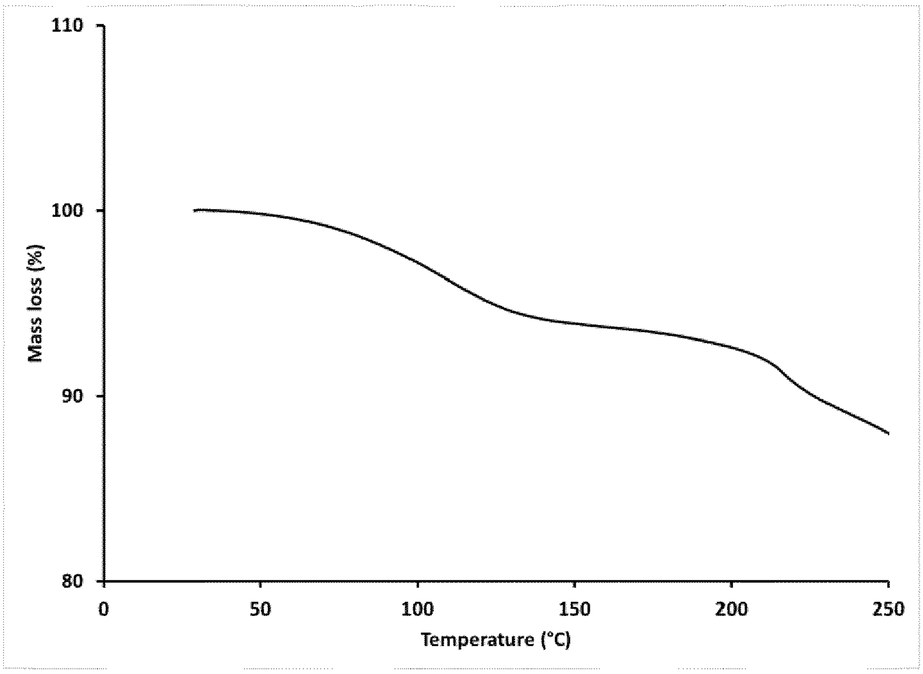


Figure 27

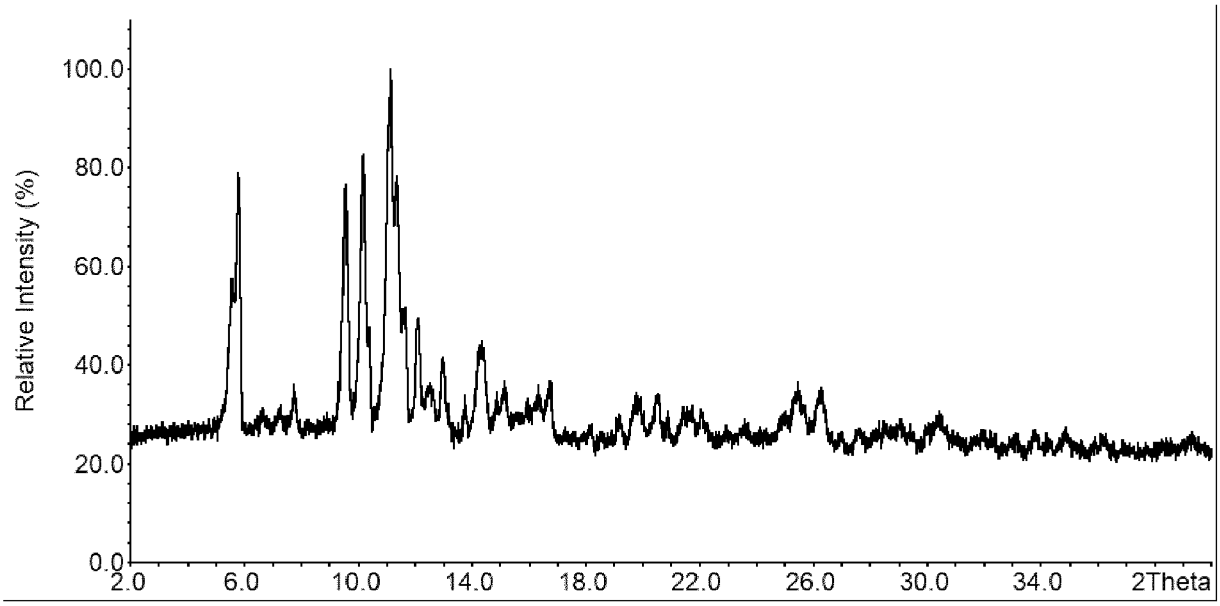


Figure 28

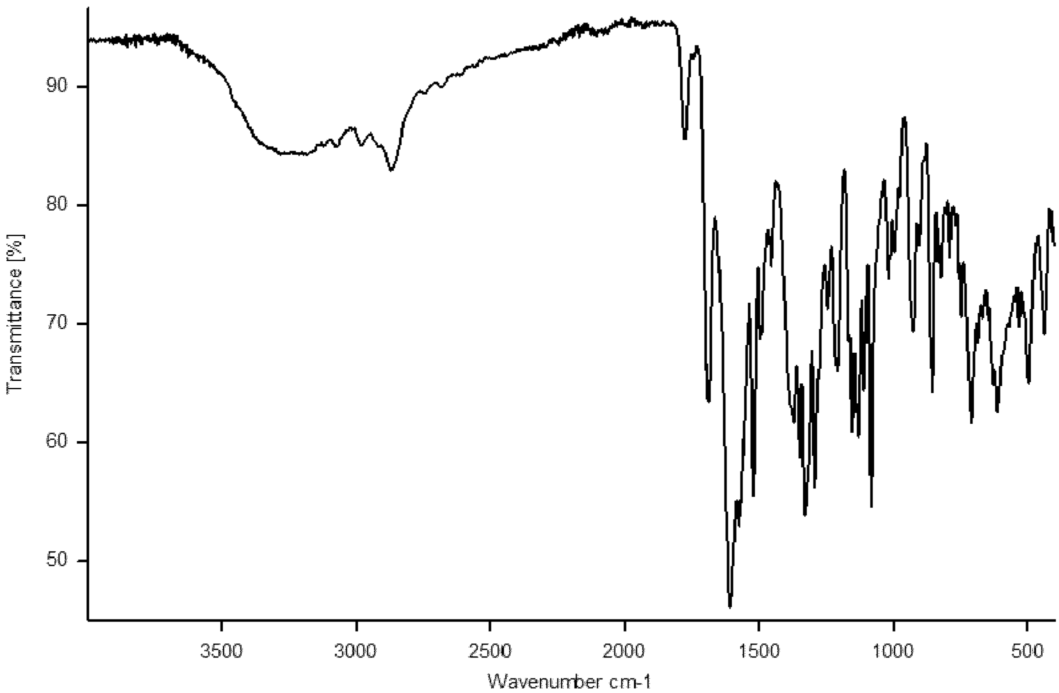


Figure 29

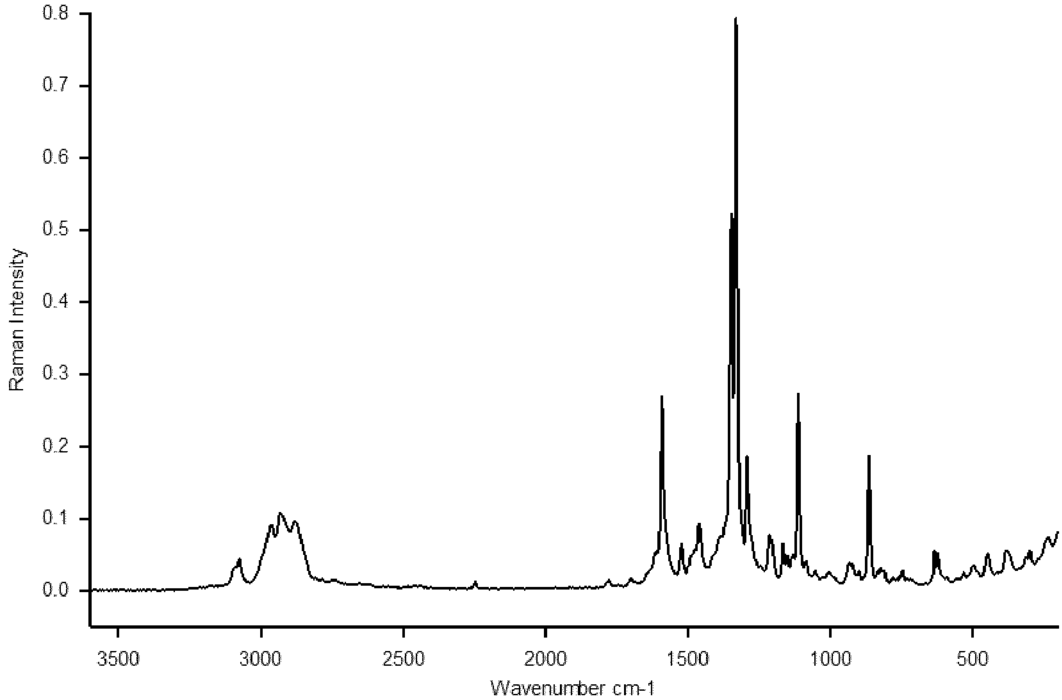


Figure 30

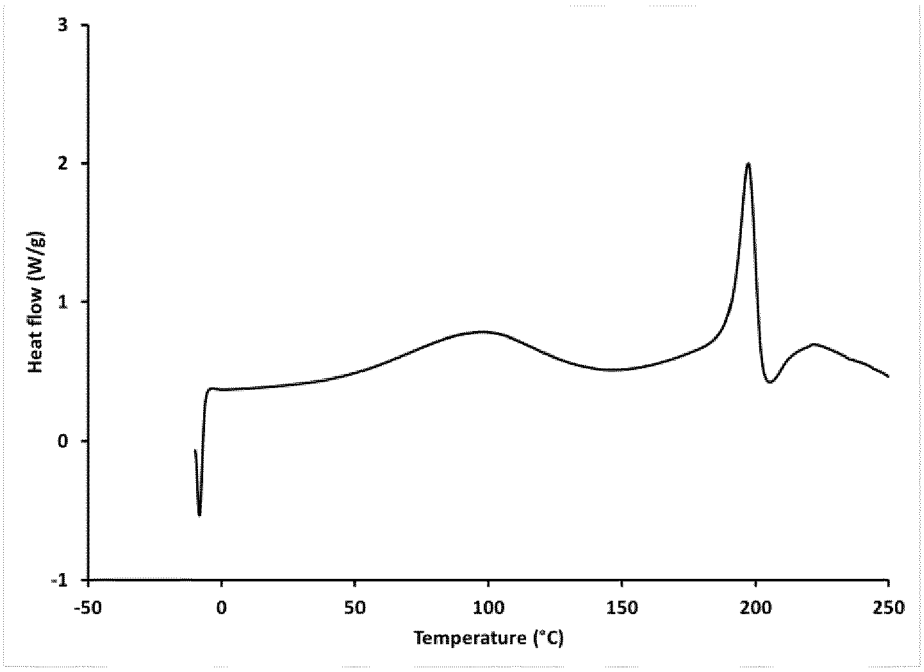


Figure 31

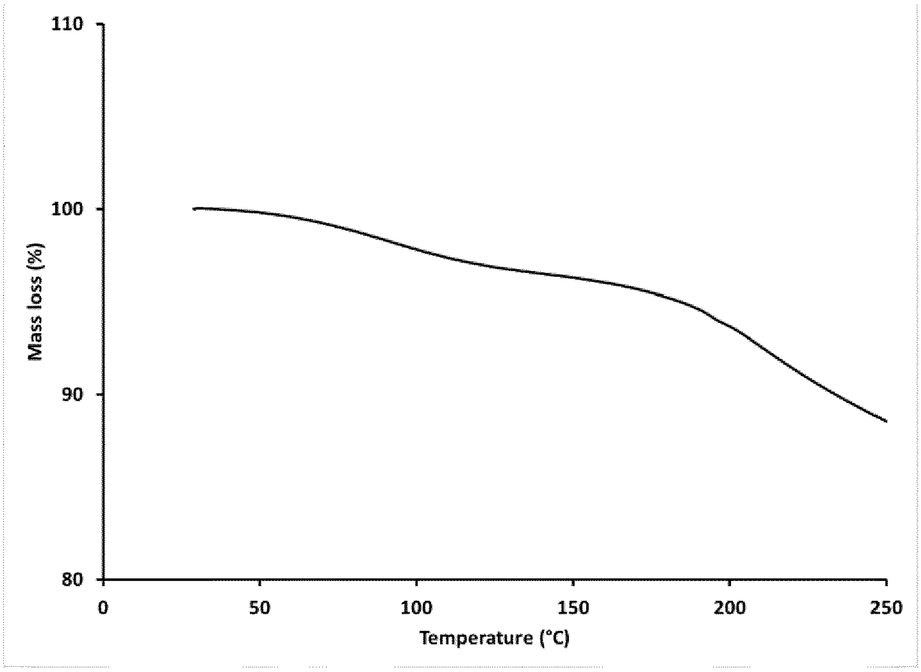


Figure 32

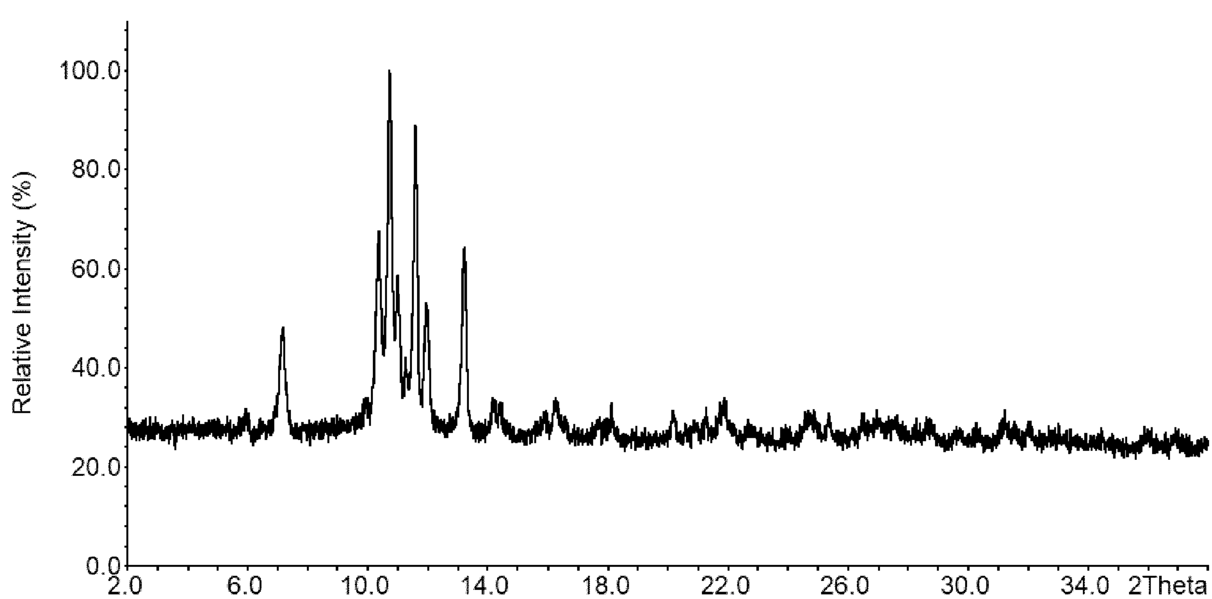


Figure 33

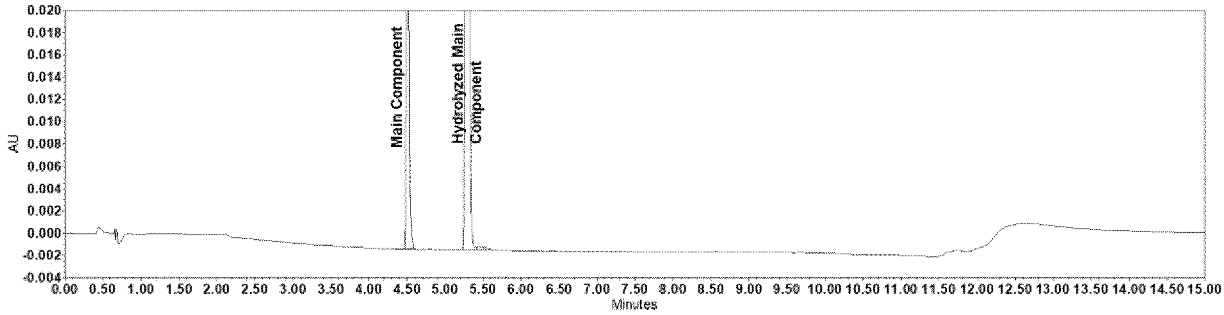


Figure 34a

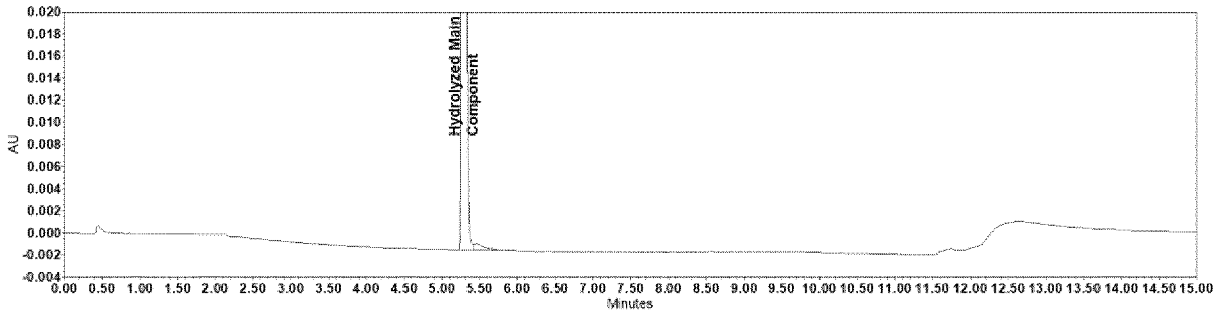


Figure 34b

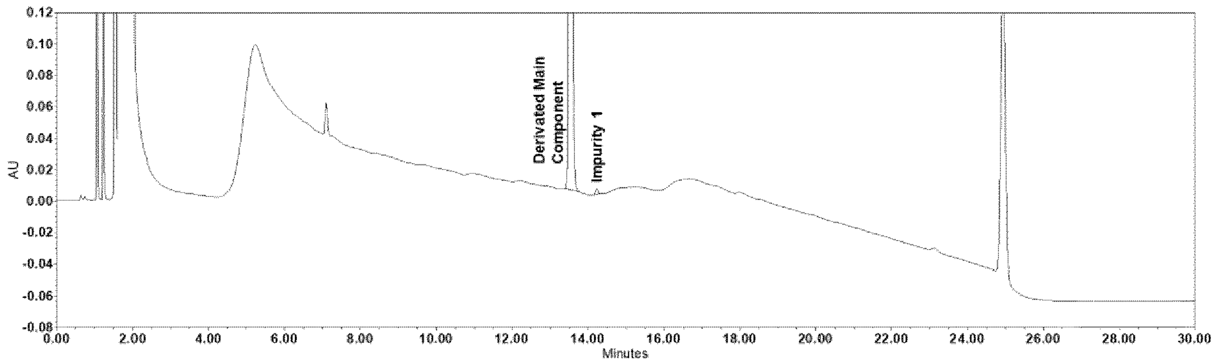


Figure 35a

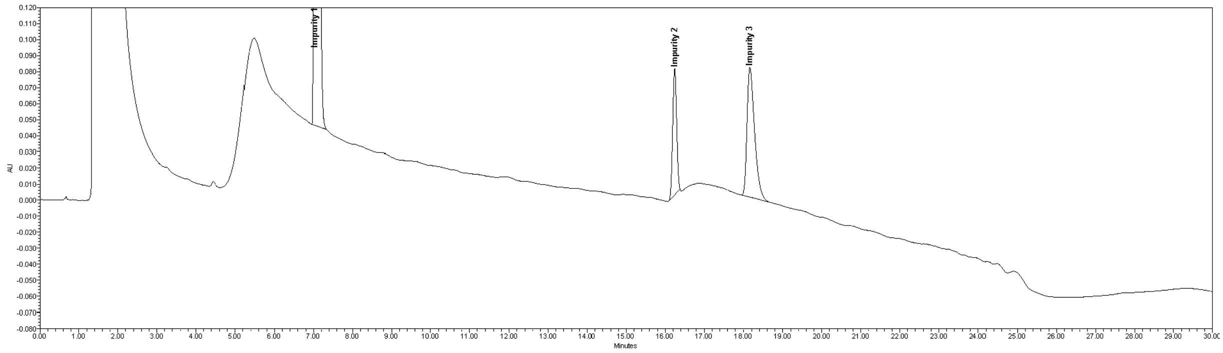


Figure 35b

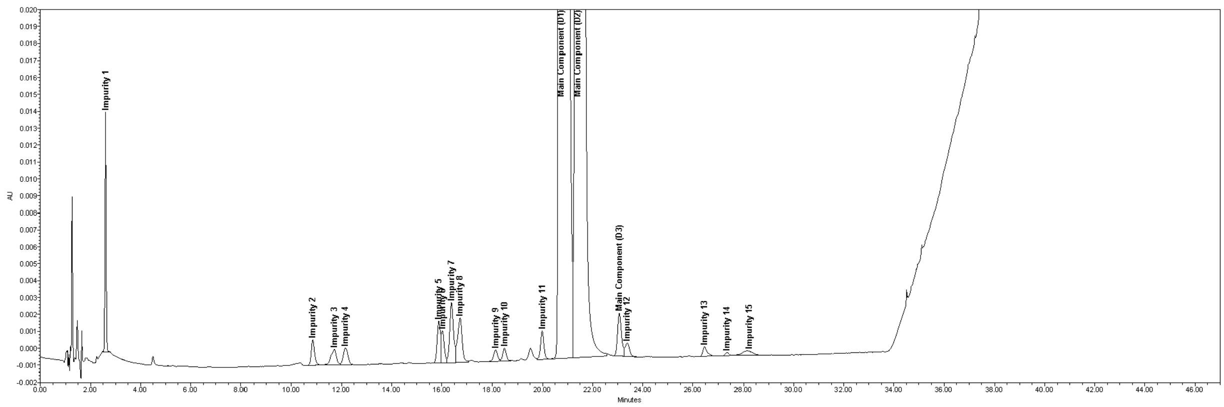


Figure 36a

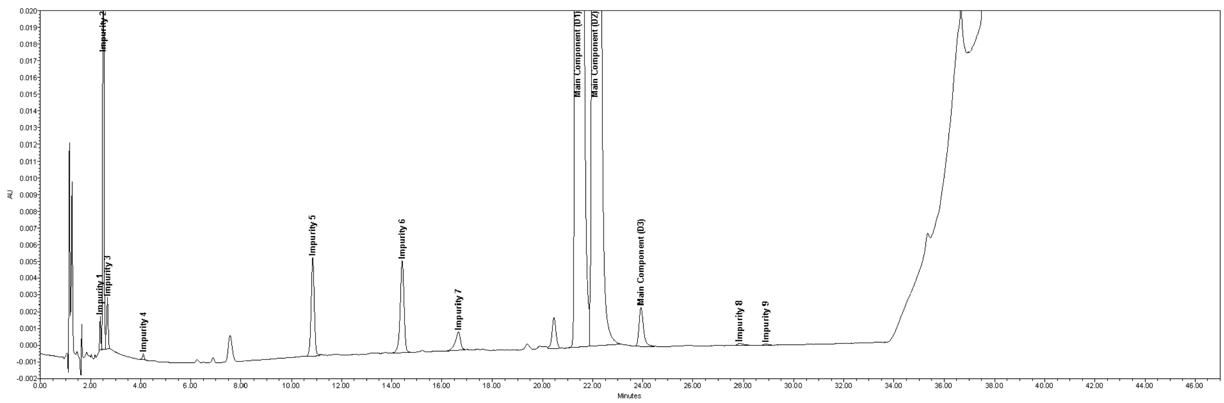


Figure 36b

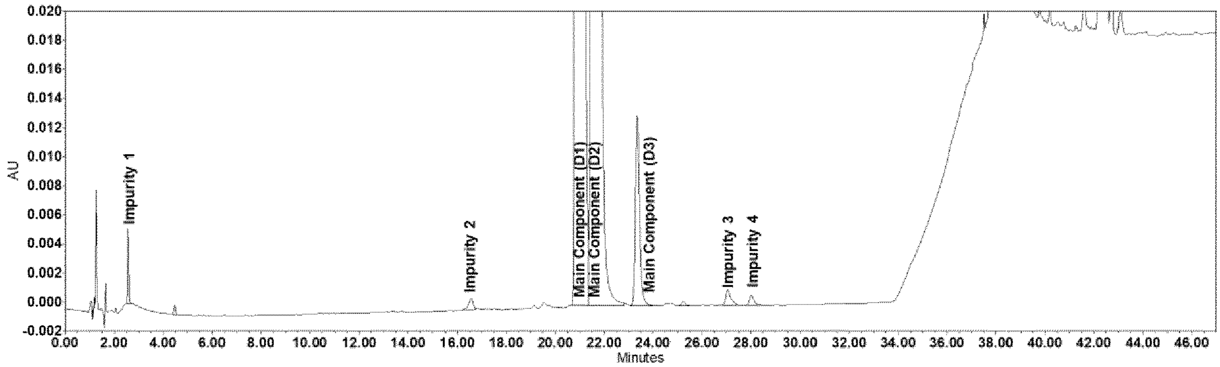


Figure 36c

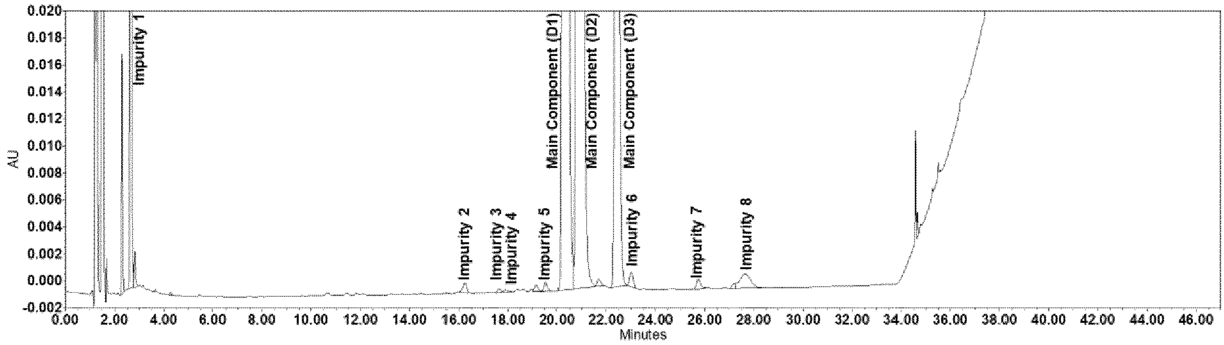


Figure 37

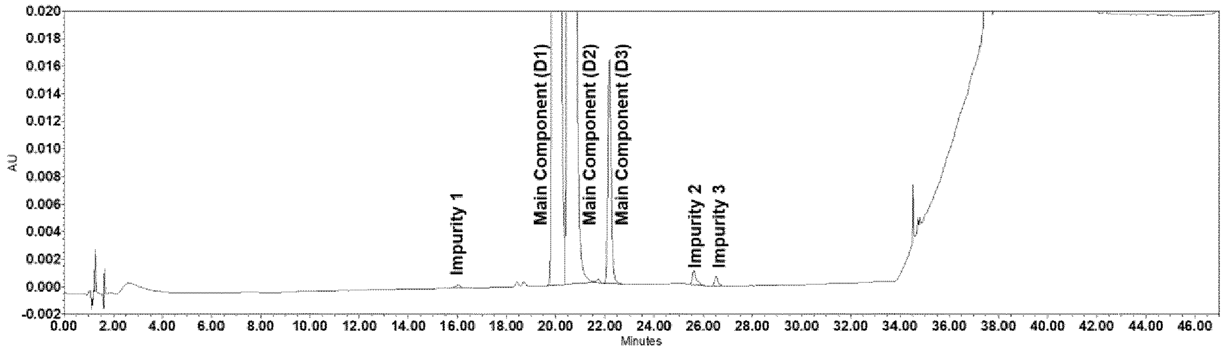


Figure 38

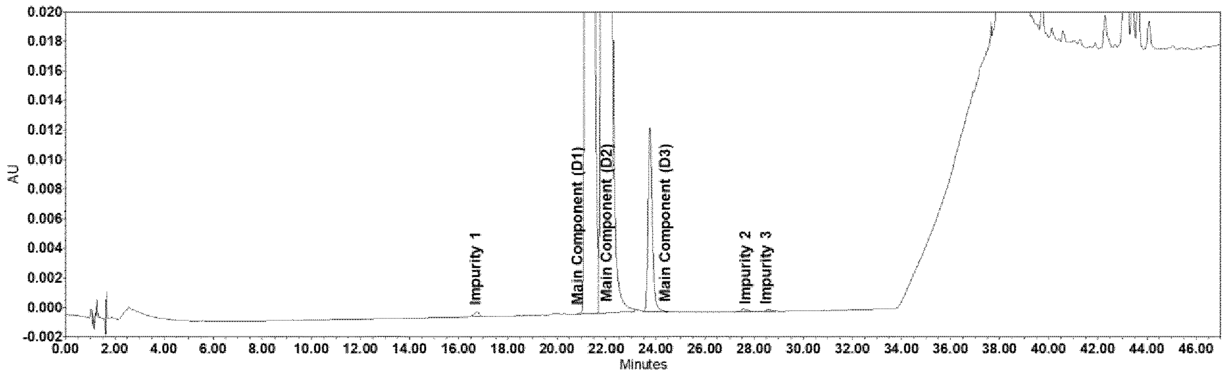
0.020
0.018
0.016
0.014
0.012
0.010
0.008
0.006
0.004
0.002
0.000
-0.002
AU
Impurity 1
Main Component (D1)
Main Component (D2)
Main Component (D3)
Impurity 2
Impurity 3
0.00 2.00 4.00 6.00 8.00 10.00 12.00 14.00 16.00 18.00 20.00 22.00 24.00 26.00 28.00 30.00 32.00 34.00 36.00 38.00 40.00 42.00 44.00 46.00
Minutes

**Figure 39**

Europäisches Patentamt
European Patent Office
Office européen des brevets

# EUROPEAN SEARCH REPORT

Application Number
EP 22 20 3296

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2020/104602 A1 (BAYER AG [DE]; BAYER PHARMA AG [DE]) 28 May 2020 (2020-05-28)<br>* pages 13, 15; compounds I-A *<br>* claims 3, 4, 24 *<br>----- | 1-13 | INV.<br>A61K49/08<br>A61K49/10<br>A61K49/12 |
| X,D | WO 2016/193190 A1 (BAYER PHARMA AG [DE]) 8 December 2016 (2016-12-08)<br>* pages 98, 104; examples 3-1, 3-2 *<br>----- | 1-13 | |

TECHNICAL FIELDS SEARCHED (IPC)

A61K

The present search report has been drawn up for all claims

1

| Place of search | Date of completion of the search | Examiner |
|---|---|---|
| Munich | 8 March 2023 | Panday, Narendra |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................
& : member of the same patent family, corresponding document

EPO FORM 1503 03.82 (P04C01)

# ANNEX TO THE EUROPEAN SEARCH REPORT ON EUROPEAN PATENT APPLICATION NO. EP 22 20 3296

This annex lists the patent family members relating to the patent documents cited in the above-mentioned European search report.
The members are as contained in the European Patent Office EDP file on
The European Patent Office is in no way liable for these particulars which are merely given for the purpose of information.

08-03-2023

| Patent document cited in search report | Publication date | Patent family member(s) | Publication date |
|---|---|---|---|
| WO 2020104602 A1 | 28-05-2020 | AU 2019382881 A1 | 20-05-2021 |
| | | BR 112021007707 A2 | 27-07-2021 |
| | | CA 3120665 A1 | 28-05-2020 |
| | | CL 2021001331 A1 | 22-10-2021 |
| | | CN 113164628 A | 23-07-2021 |
| | | CO 2021006034 A2 | 20-05-2021 |
| | | EP 3883613 A1 | 29-09-2021 |
| | | IL 283281 A | 29-07-2021 |
| | | JP 2022508185 A | 19-01-2022 |
| | | KR 20210095168 A | 30-07-2021 |
| | | PE 20211471 A1 | 05-08-2021 |
| | | SG 11202104657R A | 29-06-2021 |
| | | US 2022008562 A1 | 13-01-2022 |
| | | WO 2020104602 A1 | 28-05-2020 |
| WO 2016193190 A1 | 08-12-2016 | AR 104897 A1 | 23-08-2017 |
| | | AU 2016272602 A1 | 14-12-2017 |
| | | BR 112017026135 A2 | 28-08-2018 |
| | | CA 2987993 A1 | 08-12-2016 |
| | | CL 2017003083 A1 | 25-05-2018 |
| | | CN 107667096 A | 06-02-2018 |
| | | CO 2017012490 A2 | 09-03-2018 |
| | | CU 20170155 A7 | 03-04-2018 |
| | | CY 1122323 T1 | 27-01-2021 |
| | | DK 3303307 T3 | 25-11-2019 |
| | | DK 3611169 T3 | 11-10-2021 |
| | | DO P2017000282 A | 31-12-2017 |
| | | EA 201792675 A1 | 31-05-2018 |
| | | EC SP17080394 A | 31-01-2018 |
| | | EP 3101012 A1 | 07-12-2016 |
| | | EP 3303307 A1 | 11-04-2018 |
| | | EP 3611169 A1 | 19-02-2020 |
| | | ES 2756703 T3 | 27-04-2020 |
| | | ES 2893244 T3 | 08-02-2022 |
| | | GE P20207146 B | 10-09-2020 |
| | | HK 1246281 A1 | 07-09-2018 |
| | | HR P20191631 T1 | 13-12-2019 |
| | | HR P20211467 T1 | 24-12-2021 |
| | | HU E045967 T2 | 28-01-2020 |
| | | HU E056328 T2 | 28-02-2022 |
| | | IL 255945 A | 31-01-2018 |
| | | JO 3702 B1 | 31-01-2021 |
| | | JP 6703012 B2 | 03-06-2020 |
| | | JP 2018521017 A | 02-08-2018 |
| | | KR 20180011264 A | 31-01-2018 |
| | | LT 3303307 T | 11-11-2019 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO.

EP 22 20 3296

This annex lists the patent family members relating to the patent documents cited in the above-mentioned European search report. The members are as contained in the European Patent Office EDP file on The European Patent Office is in no way liable for these particulars which are merely given for the purpose of information.

08-03-2023

| Patent document cited in search report | Publication date | Patent family member(s) | Publication date |
|---|---|---|---|
| | | LT 3611169 T | 11-10-2021 |
| | | MA 43146 B1 | 31-01-2020 |
| | | MA 50918 A | 17-03-2021 |
| | | NI 201700149 A | 11-04-2018 |
| | | NZ 737707 A | 25-11-2022 |
| | | PE 20180261 A1 | 05-02-2018 |
| | | PH 12017502205 A1 | 11-06-2018 |
| | | PL 3303307 T3 | 30-04-2020 |
| | | PL 3611169 T3 | 27-12-2021 |
| | | PT 3303307 T | 25-11-2019 |
| | | PT 3611169 T | 06-10-2021 |
| | | RS 59565 B1 | 31-12-2019 |
| | | RS 62353 B1 | 29-10-2021 |
| | | SI 3303307 T1 | 29-11-2019 |
| | | SI 3611169 T1 | 30-11-2021 |
| | | SV 2017005578 A | 29-05-2018 |
| | | TN 2017000505 A1 | 12-04-2019 |
| | | TW 201708198 A | 01-03-2017 |
| | | UA 123313 C2 | 17-03-2021 |
| | | US 2018169274 A1 | 21-06-2018 |
| | | US 2019083659 A1 | 21-03-2019 |
| | | US 2020353104 A1 | 12-11-2020 |
| | | UY 36711 A | 30-12-2016 |
| | | WO 2016193190 A1 | 08-12-2016 |
| | | ZA 201800024 B | 25-05-2022 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2016193190 A
- WO 2001051095 A2
- US 5560903 A
- DE 19652386, Schering AG
- EP 3303307 B1


### Non-patent literature cited in the description


- **K. SATTLER.** Thermische Trennverfahren - Grundlagen, Auslegung, Apparate. Wiley-VCH, 1995, 415
- IUPAC Rules Section E. *Pure Appl Chem,* 1976, vol. 45, 11-30
- **S. M. BERGE et al.** Pharmaceutical Salts. *J. Pharm. Sci.,* 1977, vol. 66, 1-19
- **W. HAYES et al.** *Tetrahedron,* 2003, vol. 59, 7983-7996
- *CHEMICAL ABSTRACTS,* 100-02-7
- *CHEMICAL ABSTRACTS,* 208252-78-2
- *CHEMICAL ABSTRACTS,* 693-13-0
- **W. HAYES et al.** *Tetrahedron,* 2003, vol. 59, 7983